# EUROPÄISCHE PATENTSCHRIFT

(11) **EP 1 485 021 B1**
(45) Veröffentlichungstag und Bekanntmachung des Hinweises auf die Patenterteilung: **22.06.2005**
(21) Anmeldenummer: 03706537.2
(22) Anmeldetag: 20.02.2003
(51) Int. Cl.: A61B 5/15

(54) **BLUTANALYSEGERÄT UND STECHVORRICHTUNG ZUR VERWENDUNG BEI DER BLUTANALYSE**
BLOOD ANALYZER AND PRICKING DEVICE FOR USE IN BLOOD ANALYSIS
ANALYSEUR DE SANG ET DISPOSITIF DE PIQURE UTILISABLES EN ANALYSE DU SANG

(30) Priorität: 21.02.2002 DE 10208575; 17.09.2002 US 411834 P; 24.09.2002 DE 10245721
(43) Veröffentlichungstag der Anmeldung: 15.12.2004
(73) Patentinhaber: Facet Technologies, LLC, Marietta, GA 30067 (US)
(72) Erfinder: KENNEDY, Gwenn , Elaine, Ellenwood, GA 30294 (US); RUF, Christopher, John, Atlanta, GA 30345 (US); ROBBINS, Avi, Melech, Longwood, FL 32779 (US); CAMPBELL, Stephanie, Jean, Kennesaw, GA 30152 (US); LEVAUGHN, Richard, W., Talking Rock, GA 30175 (US); HEATH, Jason, R., Marietta, GA 30062 (US); SOLIS, Mitchell, Cumming, GA 30004 (US); OSTERTAG, Wolfgang, 89547 Gerstetten (DE); LOHRENGEL, Armin, 89555 Steinheim (DE); STÖHR, Herbert, 89522 Grosskuchen (DE)
(74) Vertreter: Schoppe, Fritz, Dipl.-Ing.
(86) Internationale Anmeldenummer: PCT/EP2003/001700
(87) Internationale Veröffentlichungsnummer: WO 2003/070099

(56) Entgegenhaltungen:
- EP-A- 0 877 250
- US-B1- 6 228 100

## Beschreibung

Die Erfindung betrifft ein Blutanalysegerät zur Bestimmung eines Analyten, wie z.B. Fructosamin, Lactat, Cholesterol oder insbesondere Glucose, an unmittelbar zuvor entnommenen Minimalmengen von Blut eines Benutzers, mit einem Gehäusekörper, mit einer ein Stechelement aufweisenden Blutentnahmevorrichtung, mit einem Testmittel für die Aufnahme einer Minimalmenge von Blut, mit einer eine Auswerteelektronik umfassenden Auswerteeinrichtung und mit einer Anzeigeeinrichtung, die ein als ein einziges Gerät handhabbares Komplettsystem bilden, wobei der Gehäusekörper eine der Arbeitsposition des Stechelements zugeordnete Stechposition zum Anlegen einer Hautoberfläche eines Benutzers aufweist und eine an anderer Stelle des Gehäusekörpers ausgebildete Aufgabeposition zum Aufgeben einer aus der zuvor gestochenen Hautoberfläche austretenden Minimalmenge von Blut auf ein Testmittel aufweist, wobei eine Mehrzahl von Testmitteln und Stechelementen in das Gerät einsetzbar ist, die zur Durchführung mehrerer Messungen nacheinander in eine Arbeitsposition bringbar sind, wobei bei Positionierung eines Stechelements in seiner Arbeitsposition das Stechelement in die an der Stechposition angelegte Hautoberfläche eines Benutzers einstechbar ist und aus der Hautoberfläche austretendes Blut durch Anlegen der Hautoberfläche an die Aufgabeposition auf ein Testmittel aufgegeben werden kann, welches sich in einer Arbeitsposition der Testmittel befindet.

Die Erfindung befasst sich also mit sogenannten "all-in-one"-Komplettgeräten. Die Testmittel können beipielsweise in Form einer ein Messfeld definierenden Membran ausgebildet sein, die mit der entnommenen Minimalmenge von Blut benetzt wird und Testreagenzien.umfasst, mit Hilfe derer die Analyse ausgeführt wird. Die Auswerteeinrichtung kann beispielsweise optisch, vorzugsweise reflektometrisch, oder elektrochemisch arbeiten.

US-A-5,971,941 zeigt nach einer Ausführungsform ein Komplettsystem im vorstehend geschilderten Sinne, wobei eine Kassette mit ungebrauchten streifenförmigen Testmitteln in ein Grundgerät eingesetzt und dann mittels eines Schiebers ein jeweiliges Testmittel in eine jeweilige Arbeitsposition gebracht werden kann. Über eine Auslöseeinrichtung, welche einen Teil der Blutentnahmevorrichtung bildet, wird mittels eines Stößels ein Stechelement zur Blutentnahme nach außen gestoßen, um die Hautoberfläche eines Benutzers zu durchstechen, damit kapillares Blut zur Analyse gewonnen werden kann. Die Stechelemente sind jeweils auf einem Teststreifen integriert und werden so zusammen mit dem Teststreifen in Position gebracht. Nähere Ausführungen, wie die Analyse ausgeführt wird, lassen sich dieser Druckschrift nicht entnehmen. Nach einer weiteren in dieser Druckschrift beschriebenen Ausführungsform wird ein wegwerfbarer zylinderförmiger Aufsatz oder Einsatz beschrieben, welcher ein Stechelement und eine tablettenförmige Testmembran mit einer Druchtrittsöffnung für das Stechelement aufweist. Dieser Aufsatz oder Einsatz wird dann in eine Halteausnehmung einer Stößelanordung eingesetzt, welche das Stechelement zur Blutentnahme nach außen drückt. Vor und nach jedem Testvorgang muss die wegwerfbare Einheit montiert bzw. demontiert werden.

Auch nach DE 198 19 407 A1 soll nach einer Ausführungsform eine Mehrzahl von streifenförmigen Testmitteln mit in nicht beschriebener Weise darauf integrierten Stechelementen in ein Analysegerät einsetzbar und nacheinander in eine Arbeitsposition bringbar sein. Eindeutige Hinweise auf die technische Realisierbarkeit lassen sich der Druckschrift nicht entnehmen.

Aus US-A-4,787,398 ist ein Blutzuckermessgerät bekannt mit einem Grundgerät mit einer Stößelanordnung zum Auslenken eines Stechelements und mit einer Auswerteeinrichtung und einer Anzeigeeinrichtung. Für jede Messung muss an dem Grundgerät eine auswechselbare Einheit angeordnet werden, welche das Stechelement und ein mit Blut zu benetzendes Testmittel in Form eines Teststreifens umfasst. Diese auswechselbare Einheit wird nach jedem Gebrauch verworfen.

Ein aus EP 0 449 525 A1 bekanntes Blutanalysegerät umfasst zwar ebenfalls eine integrierte Blutentnahmevorrichtung mit einem Stechelement. Vor jeder Inbetriebnahme muss aber ein neues Stechelement manuell in die Auslösevorrichtung als Teil der Blutentnahmevorrichtung eingesetzt werden, und danach muss ein Teststreifen in das Gerät eingesetzt werden.

Auch US-A-4,627,445 zeigt ein Blutzuckermessgerät mit integrierter Blutentnahmevorrichtung. Auch hier muss aber vor jeder Messung eine neue auswechselbare Einheit aus Stechelement und Testmittel aufwendig an einem Grundgerät montiert und danach demontiert werden.

Entsprechendes zeigt US-A-5,951,492. Gemäß dieser Druckschrift umfasst eine wegwerfbare Einheit eine kapillare Röhre, an deren körperabgewandtem Ende ein Teststreifen vorgesehen ist, der mit der entnommenen Minimalmenge von Blut beaufschlagt wird. Die kapillare Röhre ist an ihrem Vorderende mit einem Stechelement ausgebildet. Wiederum muss vor und nach jedem Messvorgang eine neue wegwerfbare Einheit der soeben beschriebenen Art montiert bzw. demontiert werden.

Des weiteren sind aus EP 0 877 250 A2, EP 0 949 506 A2 und EP 0 811 843 A2 nicht gattungsgemäße Blutanalysegeräte bekannt, bei denen eine Mehrzahl von Testmitteln auf einer drehbaren Scheibe als Träger für die Testmittel angeordnet sind, wobei die Testmittel aufeinanderfolgend in eine Arbeitsposition bringbar und zum Benetzen mit einer Minimalmenge von Blut aus dem Gehäusekörper herausschiebbar sind.

Aus US 6,228,100 B1 und US 4,794,926 sind Blutentnahme- bzw. -stechvorrichtungen bekannt, bei denen eine Anzahl von Stechelementen auf einem gegenüber einem Gehäusekörper drehbaren Träger angeordnet sind. Gemäß US 6,228,100 B1 werden die Stechelemente radial mittels einer Stoßvorrichtung ausgestoßen und gemäß US 4,794,926 sind die Stechelemente in axialer Richtung orientiert und aktivierbar.

Ausgehend von dem eingangs genannten, ein "all-in-one"-Komplettgerät beschreibenden Stand der Technik, liegt der vorliegenden Erfindung die Aufgabe zugrunde, ein Gerät zu schaffen, welches kompakt, also raumsparend, ausgebildet werden kann und als benutzer- und bedienungsfreundlich angesehen werden kann. Gebrauchte und ungebrauchte Testmittel bzw. Stechelemente sollen auf einfache Weise in das Gerät eingesetzt bzw. aus dem Gerät entnommen werden können.

Diese Aufgabe wird bei einem Blutanalysegerät der eingangs genannten Art erfindungsgemäß dadurch gelöst, dass die Testmittel und die Stechelemente auf einem gegenüber dem Gehäusekörper drehbaren Träger angeordnet und mit diesem in das Gerät einsetzbar sind und dass durch Drehen des Trägers die Testmittel und die Stechelemente in voneinander verschiedene Arbeitspositionen bezüglich des Gehäusekörpers bringbar sind.

Nach der Erfindung werden also sowohl die Testmittel als auch die Stechelemente auf einem drehbaren Träger angeordnet und können so durch eine Drehbewegung in ihre jeweilige Arbeitsposition gebracht werden. Wenn vorstehend von Arbeitspositionen der Testmittel und der Stechelemente die Rede ist, so wird hierunter diejenige Position oder auch diejenige Drehstellung eines Testmittels bzw. eines Stechelements gegenüber dem Gehäusekörper verstanden, in der es bestimmungsgemäß zum Einsatz kommt. Ein Stechelement befindet sich in der Arbeitsposition der Stechelemente, wenn es aus dieser Arbeitsposition zur Ausführung eines Stechvorgangs über die Stechposition hinausbewegbar ist. Ein Testmittel befindet sich in der Arbeitsposition der Testmittel, wenn es in dieser Arbeitsposition mit einer Minimalmenge von Blut benetzbar ist. Wenn vorstehend von verschiedenen Arbeitspositionen der Testmittel und der Stechelemente die Rede ist, so wird hierunter verstanden, dass ein Benutzer nach dem Stechen der Hautoberfläche in der Stechposition des Geräts die Hautoberfläche, also üblicherweise den Finger, von der Stechposition löst und an die Aufgabeposition anlegt, um eine Minimalmenge von Blut an ein Testmittel zu übertragen. Beispielsweise kann sich die Arbeitsposition der Stechelemente in einer 3-Uhr-Position und die Arbeitsposition der Testmittel in einer 6-Uhr-Position befinden. Verschiedene Arbeitspositionen von Testmitteln und Stechelementen lassen sich aber auch in einer einzigen Drehstellung des Trägers erreichen, indem etwa die Stechelemente radial angeordnet sind und die Testmittel axial orientiert sind. Die zugehörige Stechposition wäre dann radial am Gehäusekörper und die Aufgabeposition wäre axial am Gehäusekörper vorgesehen.

Die Verwendung eines drehbaren Trägers gestattet es, eine kompakte Bauform des Gehäusekörpers zu verwirklichen. Dadurch, dass die jeweils gebrauchten Testmittel und Stechelemente durch Drehen ihres Trägers aus der Arbeitsposition gebracht werden, gelangen sie auf diese Weise automatisch in eine Entsorgungsposition, ohne dass ein weiterer separater Translationsvorgang vorgesehen werden müsste.

Es wäre denkbar und vorteilhaft, wenn die Testmittel auf demselben manuell handhabbaren Träger angeordnet sind, so dass Stechelemente und Testmittel als eine einzige manuell handhabbare Einheit beispielsweise einer Umverpackung entnommen werden können und mit einem einzigen Vorgang in das Analysegerät eingesetzt werden können.

Im Hinblick auf eine vereinfachte Herstellbarkeit der Testmittel und Stechelemente erweist es sich aber auch als vorteilhaft, wenn der Träger ein erstes Trägerteil für die Testmittel und ein zweites Trägerteil für die Stechelemente umfasst, die zu einer manuell handhabbaren Einheit, vorzugsweise bereits werksseitig, montierbar sind, so dass diese handhabbare Einheit als ganze in das Gerät einsetzbar und nach Gebrauch wieder entnehmbar ist. Es soll aber auch die separate Herstellung und Bereitstellung von Testmitteln auf einem ersten Trägerteil und Stechelementen auf einem zweiten Trägerteil erfasst werden, die einzeln in das Blutanalysegerät in ihre dortige drehbare Anordnung einsetzbar sind, obschon dies zwei getrennte Bestückungsvorgänge durch den Benutzer erfordert.

Wenn verschiedene Trägerteile für die Testmittel und die Stechelemente vorgesehen sind, so erweist es sich als vorteilhaft, wenn diese beiden Trägerteile drehfest miteinander koppelbar sind, so dass es ausreichend ist, wenn ein einziger Antrieb des einen oder anderen Trägerteils im Inneren des Analysegeräts vorgesehen wird. Auch wenn die Trägerteile zu einer einzigen manuell handhabbaren Einheit montiert sind, wäre es denkbar, zwei getrennte Antriebsvorrichtungen im Analysegerät vorzusehen, was sich für einige Anwendungen als vorteilhaft erweisen mag.

Nach einer bevorzugten Ausführungsform der Erfindung weist der Träger oder die Trägerteile oder eines der Trägerteile eine mittige Ausnehmung auf, innerhalb der eine Antriebseinrichtung für die Blutentnahmevorrichtung vorgesehen ist.

Vorzugweise ist der Träger oder die Trägerteile oder eines der Trägerteile ringartig ausgebildet und um das Ringzentrum drehbar im Sinne von rotierbar angeordnet.

Der Antrieb des Trägers oder der Trägerteile kann in an sich beliebiger, vorzugsweise kompaktbauender Weise verwirklicht sein. Als zweckmäßig erweisen sich elektromotorische Antriebsvorrichtungen oder aber manuell betätigbare, also mechanische Antriebsvorrichtungen, die beispielsweise hebel- oder schiebergesteuert ausgebildet sein können. Vorzugsweise sind diskrete Drehstellungen des Trägers oder der Trägerteile vorgesehen, die sich entweder durch Rast-, Schritt- oder Anschlagmittel oder durch geeignete Ausbildung der Antriebsvorrichtung verwirklichen lassen.

Als besonders vorteilhaft und kompaktbauend hat es sich erwiesen, wenn der Träger oder die Trägerteile eine mittige Ausnehmung aufweisen, die zugleich ein Antriebsmittel oder einen Teil eines Antriebsmittels zum Drehen des Trägers oder der Trägerteile umfasst. In vorteilhafter Weise ist dieses Antriebsmittel durch eine Innenverzahnung ausgebildet, mit der ein weiteres Antriebsrad kämmt.

Von der Erfindung sind sowohl Ausführungsformen des Blutanalysegeräts erfasst, bei denen die Stechelemente so auf dem Träger angeordnet sind, dass sie in der Arbeitsposition eine Stechbewegung in radialer Richtung in Bezug auf die Drehbarkeit des Trägers ausführen, als auch solche Ausführungsformen, bei denen die Stechelemente eine Stechbewegung in axialer Richtung ausführen.

Die Anordnung der Stechelemente auf dem Träger kann in verschiedener Weise verwirklicht werden. Nach einer vorteilhaften Ausführungsform ist ein jeweiliges Stechelement vor Ausführung eines Stechvorgangs in einem einen Zylinderraum bildenden Hülsenmittel angeordnet und von einem darin bewegbaren Kolbenmittel gehalten. Es erweist sich als vorteilhaft, wenn in diesem Fall das Stechelement ein Einspritzteil des als Kunststoffspritzteil ausgebildeten Kolbenmittels bildet.

Da es an sich zwingend erforderlich ist, dass die Stechelemente auf dem Träger vor Ausführung eines Stechvorgangs von einer Sterilitätsbarriere umgeben sind, erweist sich die vorstehend beschriebene Ausführungsform als vorteilhaft, da in diesem Fall die Sterilitätsbarriere von dem dann einseitig geschlossenen Hülsenmittel und dem Kolbenmittel gebildet sein kann. Um das Hülsenmittel auf der von dem Kolbenmittel abgewandten Seite keimdicht, also mithin steril, zu verschließen, erweist es sich als vorteilhaft, wenn dieses Ende von einer insbesondere aufgesiegelten Folie abgedeckt ist. Um auch eine den Anforderungen genügende Abdichtung zwischen dem Kolbenmittel und einer Wandung des Zylinderraums zu erreichen, ist dort ein weiteres Dichtungsmittel vorgesehen. Es kann sich hierbei um eine Verbindung zwischen Wandung und Kolbenmittel handeln, die bei der Ausführung des Stechvorgangs überwunden wird; es könnte ferner eine Dichtungsmasse dort zum Einsatz kommen, oder es könnten beispielsweise ringförmige Wulste und damit zusammenwirkende Stufen, Absätze oder Ausnehmungen in dem jeweils anderen Teil vorgesehen sein.

Im Hinblick auf die Fertigung der so beschriebenen Anordnung der Stechelemente auf dem Träger erweist es sich als vorteilhaft, wenn mehrere Hülsenmittel bandförmig aneinandergefügt und die Bandenden zur Bildung einer Kreisform miteinander verbunden sind. Auf diese Weise könnten Hülsenmittel in Form endloser Bänder hergestellt, in Abschnitte geteilt und deren Enden zur Bildung einer Kreisform und damit zur Bildung eines Trägers oder eines Trägerteils gefertigt werden. Die bandförmig angeordneten Hülsenmittel könnten aber auch nur entlang eines Teilkreises angeordnet sein.

Nach einer anderen Ausführungsform kann der Träger mehrere Vertiefungen aufweisen, in denen jeweils ein Stechelement angeordnet ist. Bei dieser Ausführungsform erweist es sich als vorteilhaft, wenn die Stechelemente in axialer Richtung bezogen auf die Drehbarkeit des Trägers angeordnet sind.

Es erweist sich des weiteren als vorteilhaft, wenn eine die Vertiefung begrenzende Wandung deformierbar ausgebildet ist, so dass sie zur Ausführung des Stechvorgangs durch eine Antriebseinrichtung der Blutentnahmevorrichtung samt Stechelement auslenkbar ist. Um die Deformierbarkeit zu erhöhen, können in der die Vertiefung begrenzenden Wandung auch Schwächungszonen ausgebildet sein. Es hat sich auch als vorteilhaft erwiesen, wenn die Vertiefung im wesentlichen mulden- oder halbschalenförmig ausgebildet ist.

Auch bei dieser Anordnung der Stechelemente auf dem Träger erweist es sich als vorteilhaft, wenn die Stechelemente vor Ausführung eines Stechvorgangs in steriler Umgebung aufgenommen sind. Eine Sterilitätsbarriere kann in diesem Fall in vorteilhafter Weise von einem die Vertiefung überfangenden folienartigen Abdeckmittel gebildet sein. Dieses ist unmittelbar vor Ausführung des Stechvorgangs entweder entfernbar, oder es ist entsprechend dünn und damit von dem Stechelement durchstoßbar ausgebildet.

Nach einer weiteren Ausführungsform des Blutanalysegeräts tragen die Stechelemente vor Ausführung eines Stechvorgangs an ihrem freien Ende ein lösbares Schutzkappenmittel, welches vorzugsweise zugleich eine Sterilitätsbarriere bildet und für das freie Ende eines jeweiligen Stechelements sterile Bedingungen gewährleistet.

Bei der Ausführung des Stechvorgangs könnte das Stechelement durch das Schutzkappenmittel hindurch gestochen werden. Es wäre aber auch als vorteilhaft anzusehen, wenn das Schutzkappenmittel unmittelbar vor Ausführung des Stechvorgangs von dem betreffenden Stechelement losgelöst werden kann. Dies kann in vorteilhafter Weise durch geringfügiges Zurückziehen des jeweiligen Stechelements unmittelbar vor Ausführung des Stechvorgangs erreicht werden, indem das Schutzkappenmittel durch ein Anschlagmittel oder dergleichen an einer Mitbewegung mit dem Stechelement gehindert wird. Es erweist sich dann als vorteilhaft, wenn das jeweilige Schutzkappenmittel nach dem Ablösen von dem betreffenden Stechelement aus dem Bewegungspfad des Stechelements entfernbar und in einen Aufnahmeraum verbringbar ist. Hierfür könnte beispielsweise die Schwerkraft oder ein Federmittel verwendet werden.

Auch die Anordnung der Testmittel auf dem Träger kann derart sein, dass die Testmittel in Bezug auf die Drehbarkeit des Trägers axial orientiert sind oder dass sie radial orientiert sind.

Vorzugsweise sind die Testmittel axial orientiert, was bedeutet, dass auch die Aufgabeposition für eine Hautoberfläche zumeist eines Fingers eines Benutzers in axialer Richtung orientiert ist, wenn nicht, kapillare Flüssigkeitspfade zwischen der Aufgabeposition und dem in seiner Arbeitsposition befindlichen Testmittel zwischengeordnet sind. Bei einer axialen Ausrichtung oder Orientierung der Testmittel mit ihrem im allgemeinen ebenen oder flächenhaft erstreckten Messfeld erweist es sich als vorteilhaft, wenn die Testmittel an einen scheibenförmigen, insbesondere ringscheibenförmigen Trägerteil vorgesehen sind, dessen Ebene senkrecht zur Drehachse des Trägers orientiert ist und vorzugsweise mit einer Ebene der jeweiligen Testmittel übereinstimmt.

Nach einer weiteren Ausführungsform der Erfindung erweist es sich als vorteilhaft, wenn die Aufgabeposition am Gehäusekörper durch ein bewegbares Abdeckteil abgedeckt ist, wenn sie nicht benötigt wird, und freigebbar ist, wenn eine Analyse durchgeführt werden soll.

In weiterer Ausbildung dieses Erfindungsgedankens erweist sich als vorteilhaft, wenn durch Bewegen des Abdeckteils in Richtung einer Freigabe der Aufgabeposition eine Antriebseinrichtung der Blutentnahmevorrichtung aktivierbar ist, d.h. wenn hierdurch ein Auslösedruck oder eine Spannung erzeugt oder auch ein elektromotorisches Antriebsmittel angesteuert wird. Als besonders vorteilhaft hat es sich erwiesen, wenn die Antriebseinrichtung der Blutentnahmevorrichtung durch Spannen eines Federmittels aktivierbar ist.

Nach einer ganz besonders bevorzugten Weiterbildung der Erfindung wird vorgeschlagen, dass das Blutanalysegerät ein manuell bewegbares Steuerelement aufweist, das mit der Antriebsvorrichtung für das Stechelement und mit dem drehbaren Träger so gekoppelt ist, dass bei Bewegen des Steuerelements eine Aktivierung der Antriebsvorrichtung für das Stechelement und eine Drehbewegung des Trägers erfolgt. Dieses manuell bewegbare Steuerelement kann beispielsweise in Form eines Rades, eines Schiebers oder aber in bevorzugter Weise auch von dem vorstehend erwähnten Abdeckteil gebildet sein. Die Kopplung zwischen Steuerelement und Antriebsvorrichtung bzw. Träger kann derart ausgebildet sein, dass das Aktivieren der Antriebsvorrichtung und das Drehen des Trägers gleichzeitig erfolgt. Es wäre aber auch denkbar, dass dies zeitlich nacheinander erfolgt. Inbesondere könnte die Kopplung zwischen Steuerelement und Antriebsvorrichtung bzw. Träger derart ausgebildet sein, dass während einer ersten Phase der Bewegung in einer ersten Stellrichtung das Drehen des Trägers erfolgt und während einer zweiten Phase der Bewegung, die beispielsweise in Gegenrichtung zur ersten Stellrichtung erfolgen kann, die Aktivierung der Antriebsvorrichtung erfolgt.

Um ein schrittweises Weiterdrehen des Trägers zu erreichen, erweist es sich als vorteilhaft, wenn das Steuerelement während einer ersten Phase der Bewegung in einer ersten Stellrichtung mit dem Träger in Antriebsverbindung bringbar ist und während einer zweiten Phase der Bewegung entgegen der Stellrichtung, also bei einer Rückbewegung des Steuerelements in seine Ausgangslage, außer Antriebsverbindung bringbar ist. Bei dieser Rückstellbewegung des Steuerelements kann es sich des weiteren als vorteilhaft erweisen, wenn hierdurch die Antriebsvorrichtung für das Stechelement in eine Ausgangs- oder Ruheposition zurücksetzbar ist.

Die Kopplung zwischen Steuerelement und Träger kann in vorteilhafter Weise über einen Zahntrieb verwirklicht werden. Beispielsweise kann eine translatorische oder auch eine Schwenkbewegung über zahnartige Mitnahmemittel in einfacher Weise in eine rotatorische Bewegung umgesetzt werden, die sich dann leicht zum Antrieb des Trägers nutzen lässt. Es wäre aber auch denkbar, dass die Kopplung zwischen Steuerelement und Träger auf andere Weise, beispielsweise durch Mitnahmemittel in der Form von Rastenanordnungen oder dergleichen verwirklicht ist.

Es hat sich des weiteren als vorteilhaft erwiesen, wenn die Antriebsvorrichtung für das Stechelement eine spannbare und schlagartig entspannbare Feder, vorzugsweise in der Form einer Biegefeder, umfasst. Zum Spannen dieser Biegefeder kann das Steuerelement, insbesondere über einen Zahntrieb, auf eine Aufnahme für die Biegefeder einwirken und diese Aufnahme in der Biegeebene der Biegefeder verschwenken und so die Antriebsvorrichtung für das Stechelement spannen, d. h. aktivieren.

Als ganz besonders vorteilhaft erweist es sich, wenn die Biegefeder über einen Totpunkt hinweg in eine stabile Spannlage bringbar ist, so dass die Antriebsvorrichtung selbsttätig in dem aktivierten Zustand verbleibt. Es kann dann auf einen lösbaren Rastmechanismus verzichtet werden.

Nach einem weiteren, an sich selbständigen Erfindungsgedanken ist eine Auslöseeinrichtung für die Antriebsvorrichtung für das Stechelement durch Anlegen einer Hautoberfläche an die Stechposition betätigbar. Die Auslöseeinrichtung kann in vorteilhafter Weise als Berührungssensor oder als eindrückbarer Taster ausgebildet sein.

Es kann sich als vorteilhaft erweisen, wenn die Auslöseeinrichtung an ergonomisch gut betätigbarer Stelle des Gehäusekörpers vorgesehen ist, insbesondere auf der der Stechposition im wesentlichen gegenüberliegenden Seite. In Weiterbildung der Erfindung wird auch eine Ausführungsform vorgeschlagen, bei der die Auslöseeinrichtung in der Stechposition vorgesehen ist und durch Anlegen der zu stechenden Hautoberfläche betätigbar ist. Diesbezüglich wird eine Ausführungsform bevorzugt, bei der die Auslöseeinrichtung eine Aussparung für den Durchtritt des Stechelements zur Ausführung des Stechvorgangs aufweist.

In weiterer Ausbildung der Erfindung wird vorgeschlagen, dass ein Retraktionsmechanismus vorgesehen ist, welcher ein Zurückziehen eines jeweiligen Stechelements unmittelbar im Anschluss an den Stechvorgang bewirkt, so dass die Hautoberfläche eines Benutzers nur ganz kurzzeitig gestochen wird. Ein solcher Retraktionsmechanismus könnte im einfachsten Fall durch ein Federmittel, welches eine Rückstellkraft ausübt, verwirklicht sein. Ein solches Federmittel könnte in vielfacher Weise ausgebildet sein. Beispielsweise könnte sich ein Stechelement durch ein spiral- oder bandförmiges Federmittel hindurch erstrecken, so dass bei der Ausführung des Stechvorgangs dieses Federmittel gespannt wird. Es wäre aber auch denkbar, dass ein solcher Retraktionsmechanismus bei der Antriebseinrichtung der Blutentnahmevorrichtung verwirklicht ist, beispielsweise durch eine Zwangsführung oder durch eine motorische Vor- und Zurückbewegung eines mit dem Stechelement gekoppelten Antriebsmittels. Wenn beispielsweise das Stechelement an einem Kolbenmittel gehalten ist, so könnte ein Retraktionsmechanismus auch durch Ausbildung eines Federmittels an dem Kolbenmittel gebildet werden. Der Retraktionsmechanismus könnte aber auch durch die elastische Formstabilität eines verformbaren Wandbereichs, welcher das Stechelement unmittelbar hält, gegeben sein.

Das erfindungsgemäße Blutanalysegerät lässt sich mit einem im wesentlichen kreisscheibenförmigen Gehäusekörper bzw. einer kreisscheibenförmigen Außenkontur herstellen. Es könnte also insbesondere nach Art eines Uhrengehäuses, insbesondere nach Art eines Armbanduhrengehäuses ausgebildet sein und zusätzlich eine Zeitanzeigeeinrichtung umfassen. Solchenfalls erweist es sich als vorteilhaft, wenn der Gehäusekörper mittels eines daran befestigbaren Bandes am Handgelenk eines Benutzers getragen werden kann.

Des weiteren betrifft die Erfindung eine Stechvorrichtung für die Entnahme einer Minimalmenge von Blut am menschlichen oder tierischen Körper zu Analysezwecken. Diese Stechvorrichtung kann in Form eines selbständigen Geräts oder integriert in das vorausgehend beschriebene Blutanalysegerät zur Bildung eines "all-in-one"-Komplettgeräts ausgebildet sein. Sämtliche nachfolgenden Ausführungen und Beschreibungen der Stechvorrichtung und deren bevorzugter Merkmale werden also für sich genommen oder in Kombination mit dem vorausgehend beschriebenen Blutanalysegerät bzw. mit dessen Merkmalen in beliebiger Kombination als erfindungswesentlich angesehen.

Somit ist von der Erfindung erfasst eine Stechvorrichtung zur Verwendung bei der Entnahme einer Minimalmenge von Blut am menschlichen oder tierischen Körper zu Analysezwecken, mit einem Gehäusekörper und einer Mehrzahl von Stechelementen, wobei die Mehrzahl von Stechelementen auf oder in einem Träger angeordnet und mit diesem in den Gehäusekörper einsetzbar und nach Gebrauch wieder aus dem Gehäusekörper entnehmbar ist, wobei ein jeweiliges Stechelement in einer Arbeitsposition mit seinem spitzen Ende in eine an eine Stechposition am Gehäusekörper angelegte Hautoberfläche eines Benutzers einstechbar ist, und mit einer auf ein jeweiliges Stechelement in seiner Arbeitsposition einwirkenden Stoßvorrichtung.

Eine derartige Stechvorrichtung ist aus DE 100 57 832 C1 bekannt.

Weitere Stechvorrichtungen mit einer Mehrzahl von Stechelementen sind beispielsweise aus US 2002/0087056 A1 oder WO 02/36010 A1 bekannt.

Aus EP 0 589 186 B1 ist es beispielsweise bekannt, die angeschliffene Spitze eines Stechelements mit einem Schutzkappenmittel zu versehen, welches vor Ausführung des Stechvorgangs manuell abgedreht wird.

Gemäß WO 01/66010 A1 ist eine Vielzahl von Stechelementen in voneinander unabhängigen Kammern eines Magazins untergebracht, wobei eine jeweilige Öffnung der Kammer von einem elastischen Material verschlossen ist, welches beim Stechvorgang durchstoßen werden kann.

Ausgehend von einer Stechvorrichtung nach der vorstehend genannten DE 100 57 832 C1 liegt der vorliegenden Erfindung die Aufgabe zugrunde, die Handhabbarkeit der Stechelemente innerhalb des Gehäusekörpers zu verbessern und einen wirksamen Schutz der freien Enden der Stechelemente zu gewährleisten, ohne dass die Ausführung des Stoßvorgangs hierdurch kompliziert würde oder mit erheblichem Platzbedarf verbunden wäre.

Diese Aufgabe wird ausgehend von einer Stechvorrichtung der genannten Art erfindungsgemäß dadurch gelöst, dass ein jeweiliges Stechelement zumindest bereichsweise in einem Haltekörper aufgenommen ist und ein das spitze einstechbare Ende bildender Endabschnitt des Stechelements von einem lösbaren Schutzkappenmittel umgeben ist, dass die Längsabmessung des jeweiligen Stechelements mit Haltekörper und Schutzkappenmittel in Stechrichtung ≤ 15 mm beträgt und dass das Schutzkappenmittel vor der Ausführung des Stechvorgangs vorzugsweise quer zur Stechrichtung aus dem Bewegungspfad des Stechelements mittels eines vorrichtungsinternen Verdrängungsorgans verbringbar ist.

Wie vorausgehend angedeutet kann die Stechvorrichtung in ein Blutanalysegerät integriert sein, so dass als Erfindungsgegenstand auch angesehen wird: ein Blutanalysegerät zur Bestimmung eines Analyten, wie z.B. Fructosamin, Lactat, Cholesterol oder insbesondere Glucose, an unmittelbar zuvor entnommenen Minimalmengen von Blut eines Benutzers, mit einem Gehäusekörper, mit einer ein Stechelement und eine Stoßvorrichtung für das Stechelement aufweisenden Blutentnahme- oder Stechvorrichtung, mit einem Testmittel für die Aufnahme einer Minimalmenge von Blut, mit einer eine Auswerteelektronik umfassenden Auswerteeinrichtung und mit einer Anzeigeeinrichtung, die ein als ein einziges Gerät handhabbares Komplettsystem bilden, wobei der Gehäusekörper eine der Arbeitsposition des Stechelements zugeordnete Stechposition zum Anlegen einer Hautoberfläche eines Benutzers aufweist und eine, insbesondere an anderer Stelle des Gehäusekörpers ausgebildete Aufgabeposition zum Aufgeben einer aus der zuvor gestochenen Hautoberfläche austretenden Minimalmenge von Blut auf ein Testmittel aufweist, wobei eine Mehrzahl von Testmitteln und Stechelementen in das Gerät einsetzbar ist, die zur Durchführung mehrerer Messungen nacheinander in eine Arbeitsposition bringbar sind, wobei bei Positionierung eines Stechelements in seiner Arbeitsposition das Stechelement in die an der Stechposition angelegte Hautoberfläche eines Benutzers einstechbar ist und aus der Hautoberfläche austretendes Blut durch Anlegen der Hautoberfläche an die Aufgabeposition auf ein Testmittel aufgegeben werden kann, welches sich in einer Arbeitsposition der Testmittel befindet, wobei das Blutanalysegerät weiter so ausgebildet ist, dass ein jeweiliges Stechelement zumindest bereichsweise in einem Haltekörper aufgenommen ist und ein das spitze einstechbare Ende bildender Endabschnitt des Stechelements von einem lösbaren Schutzkappenmittel umgeben ist, dass die Längsabmessung des jeweiligen Stechelements mit Haltekörper und Schutzkappenmittel in Stechrichtung ≤ 15 mm beträgt und dass das Schutzkappenmittel vor der Ausführung des Stechvorgangs vorzugsweise quer zur Stechrichtung aus dem Bewegungspfad des Stechelements mittels eines vorrichtungsinternen Verdrängungsorgans verbringbar ist.

Dadurch dass ein jeweiliges Stechelement in einem Haltekörper aufgenommen ist, bei dem es sich insbesondere um ein Kunststoffspritzteil handeln kann, das an das Stechelement angespritzt ist, kann eine weitgehende Miniaturisierung des Stechelements auf eine Abmessung von höchstens 15 mm, in einer bevorzugten Ausführungsform von höchstens 14 und insbesondere von höchstens 13 mm erreicht werden, wobei die Längsabmessung auch den Haltekörper und das Schutzkappenmittel mit einschließt. Das Schutzkappenmittel ist innerhalb der Stechvorrichtung unmittelbar vor der Ausführung des Stechvorgangs aus dem Bewegungspfad des Stechelements entfernbar. Beispielsweise könnte das betreffende Schutzkappenmittel zunächst in der Stechrichtung von dem Stechelement abgezogen werden, so dass es vom freien Ende des Stechelements freikommt, damit es dann vorzugsweise quer zur Stechrichtung und automatisiert mittels eines Verdrängungsorgans beiseite gebracht werden kann, um daraufhin den Stechvorgang ausführen zu können. Ebenso wäre es denkbar, dass das jeweilige Schutzkappenmittel zunächst in seiner Position verbleibt und das Stechelement geringfügig entgegen der Stechrichtung zurückgezogen wird, damit das freie Ende des Stechelements aus dem Schutzkappenmittel freikommt.

Zwar könnte das Schutzkappenmittel unabhängig von der Fertigung des Haltekörpers auf das freie spitze Ende des Stechelements aufgebracht werden; indessen erweist es sich herstellungstechnisch, insbesondere im Hinblick auf die angestrebte Miniaturisierung, als vorteilhaft, wenn das Schutzkappenmittel ebenfalls, vorzugsweise im selben Vorgang zusammen mit dem Haltekörper, an das Stechelement angespritzt wird. Solchenfalls kann das Schutzkappenmittel über einen, einen Schwächungsbereich oder Sollbruchbereich bildenden Abschnitt einstückig in den Haltekörper übergehen. Dies vereinfacht die Handhabbarkeit der Stechelemente unmittelbar im Anschluss an ihre Fertigung.

Zwar könnte der den Schwächungs- oder Sollbruchbereich bildende Abschnitt durch Abdrehen des Schutzkappenmittels lösbar sein. Im Anschluss an die vorstehenden Ausführungen erweist es sich aber als vorteilhaft, wenn der den Schwächungs- oder Sollbruchbereich bildende Abschnitt auf Zugbelastung in Längsrichtung des jeweiligen Stechelements, also in der Stechrichtung, brechbar ist.
Zum Lösen des Schutzkappenmittels könnte dieses durch ein an sich beliebiges, beispielsweise stössel- oder hülsenförmiges, Verdrängermittel zunächst in Stechrichtung von dem Stechelement abgezogen werden oder - wie bereits erwähnt - könnte das Stechelement entgegen der Stechrichtung zurückbewegt werden und so von dem Schutzkappenmittel freikommen. Nach einer besonders bevorzugten Ausführungsform der Erfindung ist das Schutzkappenmittel beim Spannen der Stoßvorrichtung von dem Stechelement lösbar. Es wird also eine mit dem Spannen der Stoßvorrichtung einhergehende Bewegung zum Lösen des Schutzkappenmittels verwandt. Insbesondere wird dabei das Stechelement entgegen der Stechrichtung zurückgezogen.

Nach einem weiteren Erfindungsgedanken kommt dem Haltekörper für das Stechelement nicht nur eine Haltefunktion für das Stechelement zu, sondern auch eine Führungsfunktion bei der Ausführung des Stoßvorgangs. Die Außenform des Haltekörpers ist dabei komplementär zu Führungsmitteln, beispielsweise in Form von Leitwänden zur gleitverschieblichen Anordnung des Stechelements, ausgebildet.

In weiterer Ausbildung des Haltekörpers weist dieser wenigstens ein Lagesicherungsmittel, insbesondere in Form eines abstehenden Stegs auf. Ein solcher abstehender Steg kann beispielsweise den Haltekörper und damit das Stechelement in einer Position in der Ebene halten, also verhindern, dass eine Drehung um die Längsachse des Stechelements, insbesondere während des Stoßvorgangs, stattfindet oder dass das Stechelement beim Austausch eines Stechelemente-Trägers aus dem Träger herausgleitet.

Ein jeweiliger Haltekörper kann aber auch einen beim Stechvorgang elastisch nachgiebig abspreizbaren Steg aufweisen, der eine Rückzugskraft auf das Stechelement ausüben kann, so dass der Haltekörper samt Stechelement wieder hinter eine Anlagefläche am Gehäusekörper zurückgezogen wird.

Für die Ausführung des Stechvorgangs wäre es denkbar, dass die Stechvorrichtung ein federvorspannbares Stoßorgan aufweist, welches in Stechrichtung auf ein Ende des Haltekörpers bzw. des jeweiligen Stechelements auftrifft und dieses dann in Stechrichtung schlagartig bewegt. Demgegenüber erweist es sich als vorteilhaft, wenn der jeweilige Haltekörper mit der Stechvorrichtung zusammenwirkende Hintergriffsmittel aufweist. Über diese Hintergriffsmittel kann ein jeweiliger Haltekörper mit der Stechvorrichtung gekoppelt werden und insbesondere mit dem Spannen der Stechvorrichtung in eine aktivierte Position bewegt werden. Auf diese Weise kann - wie eingangs erwähnt - das Schutzkappenmittel von dem Stechelement gelöst werden. Zum Lösen des Schutzkappenmittels auf diese Weise ist es erforderlich, dass das Schutzkappenmittel gegen ein Anschlagmittel läuft oder in sonstiger Weise zurückgehalten wird. In vorteilhafter Weise ist das jeweilige Schutzkappenmittel in Längsrichtung des Stechelements formschlüssig gegenüber dem Träger gehalten, so dass es diesbezüglich an einer Bewegung des Haltekörpers samt Stechelement nicht teilnimmt, sondern vom Haltekörper und Stechelement gelöst wird.

Des weiteren erweist es sich als vorteilhaft, wenn das Schutzkappenmittel quer zum Bewegungspfad des Stechelements zwangsgeführt bewegbar ist. Dies kann zweckmäßigerweise durch komplementäre Ausbildung des Trägers oder Bestandteilen am Träger und des Schutzkappenmittels erreicht werden. Es wird ausdrücklich darauf hingewiesen, dass es hierfür nicht auf eine konkrete Gestalt des Schutzkappenmittels, sondern stets auf eine komplementäre Ausbildung der Außenkontur des Schutzkappenmittels und geeigneter Aufnahmen vorzugsweise in oder am Träger ankommt, so dass das Schutzkappenmittel nach dem Lösen vom Stechelement bzw. vom Haltekörper des Stechelements sofort entlang dieser Zwangsführung aus dem Bewegungspfad des Stechelements mittels des vorrichtungsinternen Verdrängungsorgans verbracht werden kann.

Um das Schutzkappenmittel, insbesondere quer aus dem Bewegungspfad sicher und rasch zu verbringen, ist dieses vorzugsweise vorgespannt, und zwar insbesondere quer zum Bewegungspfad des Stechelements. Vorteilhafterweise liegt das Verdrängungsorgan unter Vorspannung belastend mittelbar oder unmittelbar gegen das Schutzkappenmittel an. Um eine Relativbewegung zwischen Schutzkappenmittel und Verdrängungsorgan zu vermeiden, erweist es sich als vorteilhaft, dass jedem Schutzkappenmittel ein Verdrängungsorgan dauerhaft, also auch außerhalb der jeweiligen Arbeitsposition zugeordnet ist.

Es sei wiederum darauf hingewiesen, dass für die Ausbildung des Verdrängungsorgans keine zwingenden Anforderungen mit Ausnahme derjenigen gestellt werden, dass nach dem Verdrängen des gelösten Schutzkappenmittels der Bewegungspfad für das Stechelement wieder freigegeben sein muss. Insbesondere im Hinblick hierauf erweist es sich als vorteilhaft, wenn das Verdrängungsorgan einen insbesondere U-förmigen Bügel aufweist, der das Schutzkappenmittel aus dem Bewegungspfad des Stechelements verbringt. Durch die bügelförmige Ausbildung kann dann auch nach dem Verdrängungsvorgang das Verdrängungsorgan weiterhin belastend gegen das Schutzkappenmittel anliegen, wobei sich das Stechelement zwischen Schenkeln des Bügels hindurchbewegen kann.

Als besonders vorteilhaft erweist es sich, dass der Träger Entsorgungspositionen für ein jeweiliges Schutzkappenmittel aufweist, in denen das jeweilige Schutzkappenmittel nach dem Abtrennen vom Stechelement unverlierbar aufnehmbar ist. Als ganz besonders vorteilhaft erweist es sich, dass solchenfalls definierte Aufnahmepositionen, insbesondere Aufnahmekavitäten, am Träger vorgesehen sind, und dass die Schutzkappenmittel zusammen mit dem Träger aus dem Gehäusekörper entnehmbar sind.

Nach einem weiteren Erfindungsgedanken ist das jeweilige Schutzkappenmittel in seiner Entsorgungsposition in Klemmlage untergebracht, so dass es ohne störende Geräusche zu entwickeln, aufgenommen ist. Beispielsweise kann es unter Vorspannung oder Belastung gegen eine Wandung des Trägers anliegen. Es erweist sich als besonders vorteilhaft, wenn hierfür das Verdrängungsorgan verwendbar ist. Zu diesem Zweck ist es in vorteilhafter Weise derart ausgebildet, dass es zumindest in der Entsorgungsposition flächenhaft gegen das Schutzkappenmittel anliegt.

Es erweist sich im Hinblick auf eine kompakte Ausbildung der Vorrichtung als vorteilhaft, wenn das Schutzkappenmittel in seiner Ausgangsposition am freien Ende des Stechelements und in seiner Entsorgungsposition durch dasselbe Mittel, insbesondere und vorzugsweise durch das Verdrängungsorgan, vorgespannt ist.

Das Verdrängungsorgan ist vorzugsweise an dem Träger montiert, so dass es mit diesem in den Gehäusekörper einsetzbar ist.

In bevorzugter Ausbildung dieses Erfindungsgedankens ist das Verdrängungsorgan derart am Träger vorgesehen, dass es die Stechelemente mit ihren Haltekörpern und die Schutzkappenmittel verliersicher, aber gleichwohl gleitverschieblich am Träger hält. Solchenfalls erweist es sich als vorteilhaft, wenn das Verdrängungsorgan als Federelement ausgebildet ist, welches belastend gegen die Schutzkappenmittel anliegt. Bei drehbarer Ausbildung des Trägers für die Stechelemente kann das Verdrängungsorgan als Federring mit radial vorstehenden Federzungen ausgebildet sein.

Nachfolgend seien bevorzugte Ausführungen der Stoßvorrichtung erläutert: Die Stoßvorrichtung kann ein spannbares Kolbenmittel oder Stößelmittel als Stoßorgan für die Ausführung des Stechvorgangs umfassen. Während es denkbar ist, dass das Stoßorgan auf das freie Ende des Haltekörpers oder Stechelements auftrifft, um dieses schlagartig zu beschleunigen, erweist es sich als vorteilhaft, wenn ein Stoßorgan der Stoßvorrichtung schon vor Ausführung des Stoßvorgangs mit dem Haltekörper verbunden ist. Hierfür weist das Stoßorgan einen Kopplungsbereich auf, der mit dem Haltekörper für das Stechelement koppelbar ist, so dass Stoßorgan und Stechelement schon vor der Ausführung des Stoßvorgangs in Mitnahmeverbindung stehen, also insbesondere das Stoßorgan zusammen mit dem Haltekörper eine Spannbewegung ausführen kann.

Die formschlüssige Kopplung zwischen Stoßorgan und Haltekörper bzw. Stechelement kann durch an sich beliebige Klemmmittel, Rastbügel oder dergleichen lösbare Verbindungen erreicht werden. In Weiterbildung der Erfindung ist der Kopplungsbereich des Stoßorgans und der Haltekörper aber' dadurch koppelbar, dass beide relativ zueinander quer zur Stechrichtung in eine formschlüssige Mitnahmeverbindung bewegbar sind. Solchenfalls brauchen keine biegsamen Klauen, Rast- oder Klemmmittel eingesetzt zu werden. Insbesondere bei konzentrischer drehbarer Anordnung mit radial ausgerichteten Stechelementen sind der Haltekörper und der Kopplungsbereich des Stoßorgans in Umfangsrichtung der drehbaren Anordnung in die Mitnahmeverbindung drehbar.

Um das Stoßorgan der Stoßvorrichtung entgegen einer Spannkraft zu spannen, weist das Stoßorgan einen quer zur Stechrichtung vorstehenden Spannnocken auf. Zwar wäre wiederum eine an sich beliebige Ausbildung der Stoßvorrichtung zum Spannen des Stoßorgans denkbar; der beschriebene Spannnocken erweist sich aber insoweit als vorteilhaft, als er eine Verlagerung des Spannmechanismus für das Stoßorgan in eine parallele Ebene zulässt. Der Spannnocken kann dann in vorteilhafter Weise entlang einer Kurvenbahn eines verstellbaren oder betätigbaren Spannmittels geführt sein. Während dieser Bewegung des Spannnockens entlang der Kurvenbahn wird das linear zwangsgeführte Stoßorgan in einen gespannten aktivierten Zustand gebracht. Bei der erwähnten Kurvenbahn kann es sich vorteilhafterweise um eine Kulissenbahn oder eine Nockenführungsbahn an sich beliebiger Realisierung handeln.

Es erweist sich des weiteren als vorteilhaft, dass das Spannmittel nach Ausführung der Bewegung in Spannrichtung federkraftgesteuert zurückbewegbar ist. Es handelt sich hierbei beispielsweise um einen insbesondere scheibenförmigen schwenkbar oder rotatorisch gelagerten Hebel, bei dessen Bewegung in Spannrichtung eine Rückzugsfeder gespannt wird. Beispielsweise kann das Spannmittel einen über das Gehäuse der Stechvorrichtung vorstehenden Hebel umfassen, der dann manuell in Spannrichtung ausgelenkt werden kann und der sich beim Loslassen wieder selbsttätig in die Ausgangslage zurückbewegt.

In Weiterbildung der Erfindung von ganz besonderer Bedeutung bildet das Spannmittel zum Spannen oder Aktivieren der Stoßvorrichtung zugleich ein Stellmittel, um ein jeweiliges Stechelement in eine Arbeitsposition und ein benutztes Stechelement in eine Entsorgungsposition zu bringen. Es kann also insbesondere der Träger mit den Stechelementen um einen Schritt weitergestellt, insbesondere weitergedreht werden. Anstelle eines Weiterstellens des Trägers wäre es auch denkbar, dass ein Weiterstellen der Stoßvorrichtung relativ zum Träger ausgeführt wird. Nach dem vorstehend erwähnten Erfindungsgedanken wird also durch ein und dieselbe Stellbewegung sowohl die Stoßvorrichtung aktiviert als auch ein neues, noch ungebrauchtes Stechelement bzw. die Stoßvorrichtung in eine Arbeitsposition gebracht.

Das Spannmittel bzw. der Spannmechanismus kann dabei so ausgebildet und angeordnet sein, dass es in einer ersten Phase der Bewegung in Antriebsverbindung mit dem Träger für die Stechelemente und in einer zweiten Phase der Bewegung in Antriebsverbindung mit dem Stoßorgan steht. Solchenfalls kann die Antriebsverbindung zwischen Spannmittel und Träger am Ende der ersten Bewegungsphase durch Aufgleiten des Spannmittels oder eines Arms des Spannmittels gegen ein Rampenmittel gelöst werden. Auf diese Weise kann erreicht werden, dass trotz Betätigung durch dasselbe Spannmittel zunächst in der ersten Phase durch Weiterstellen des Trägers ein neues Stechelement in die Arbeitsposition gebracht und insbesondere mit dem Stoßorgan gekoppelt wird und dann in der zweiten Phase der Bewegung das Stoßorgan, insbesondere zusammen mit dem daran gekoppelten Stechelement, in Spannrichtung bewegt wird.

Es wurde bereits darauf hingewiesen, dass die Stechvorrichtung zusätzliche Komponenten enthalten kann, und insbesondere zusammen mit einer Mehrzahl von Testmitteln, einer Auswerteeinrichtung und einer Anzeigeeinrichtung ein als ein einziges Gerät handhabbares Blutanalysegerät bilden kann, ein sogenanntes "All-in-one"-Gerät.

Solchenfalls erweist es sich als vorteilhaft, dass auch die Testmittel nacheinander in eine Arbeitsposition bringbar sind, in der aus einer zuvor gestochenen Hautoberfläche eines Benutzers die erforderliche Minimalmenge von Blut auf das jeweilige Testmittel aufgebbar ist. Bei den Testmitteln kann es sich beispielsweise um Membranen mit darin enthaltenen Testreagenzien handeln, mit deren Hilfe die Analyse optisch oder elektrochemisch oder elektrophysikalisch in an sich bekannter und daher nicht näher zu beschreibender Weise ausgeführt wird. Beispielsweise kann hierdurch ein Analyt wie Fructosamin, Lactat, Cholesterol oder insbesondere Glucose, an der zuvor entnommenen Minimalmenge von Blut qualitativ und vorzugsweise auch quantitativ bestimmt werden.

Nach einer bevorzugten Ausführungsform der Erfindung sind die Stechelemente und/oder die Testmittel konzentrisch zu einem Drehpunkt angeordnet, so dass sie in ihre jeweiligen Arbeitspositionen drehbar sind. Hierfür ist vorteilhafterweise ein erster Träger für die Stechelemente und ein davon separater zweiter Träger für die Testmittel vorgesehen. Diese Träger können aber zu einer Einheit kombiniert sein, so dass sie als eine handhabbare Einheit in das Gerät einsetzbar und aus dem Gerät herausnehmbar sind.

Im Hinblick auf eine weitgehende Miniaturisierung der Vorrichtung erweist es sich als besonders vorteilhaft, wenn die Stechelemente bei radialer Anordnung auf dem Träger ein Kreissegment aussparen, damit der Träger so in den Gehäusekörper einsetzbar ist, dass die Stoßvorrichtung sich in dieses Kreissegment erstreckt. Dadurch dass also in einem kuchenstückartigen Kreissegment, welches insbesondere 10 bis 20° in Umfangsrichtung umfassen kann, kein Stechelement vorgesehen ist, kann sich die Stoßvorrichtung in diesem Bereich in das Kreissegment in radialer Richtung hineinerstrecken; das Einsetzen des Trägers mit den Stechelementen (und gegebenenfalls auch mit den Testmitteln) in den Gehäusekörper ist somit nicht durch die Stoßvorrichtung behindert. Dies erweist sich ferner weiter als vorteilhaft, wenn - wie eingangs beschrieben - ein jeweiliges Stechelement bzw. ein Haltekörper für ein jeweiliges Stechelement und ein Stoßorgan der Stoßvorrichtung relativ zueinander in eine Kopplungsverbindung gedreht werden.

Weitere Merkmale, Einzelheiten und Vorteile der Erfindung ergeben sich aus den Patentansprüchen und den beigefügten zeichnerischen Darstellungen und der nachfolgenden Beschreibung einer bevorzugten Ausführungsform der Erfindung. Es wird darauf hingewiesen, dass die in den Patentansprüchen beanspruchten Merkmale ungeachtet ihrer Kombination und Rückbeziehung der Ansprüche jeweils separat für sich gesehen als erfindungswesentlich betrachtet werden, so dass Schutz jederzeit für spezielle Ausgestaltungen des Blutanalysegeräts oder der Stechelemente, des Trägers oder der Träger, der Stoßvorrichtung, des Antriebs- und Stellmechanismus für die Stechelemente in Anspruch genommen wird, und zwar auch unabhängig von der Ausbildung weiterer Komponenten, insbesondere unabhängig von der derzeit beanspruchten Ausbildung des Blutanalysegeräts nach Anspruch 1.

In der Zeichnung zeigt:
- Figur 1: eine perspektivische Ansicht eines erfindungsgemäßen Blutanalysegeräts;
- Figur 2: eine Draufsicht auf das Gerät nach Figur 1;
- Figur 3: eine explosionsartige Darstellung eines geöffneten Gehäusekörpers des Blutanalysegeräts nach Figur 1;
- Figur 4: eine explosionsartige Darstellung eines Trägers mit Stechelementen und Testmitteln des Geräts nach Figur 3;
- Figur 5: eine Schnittansicht mit einer durch die Pfeile A-A bezeichneten Schnittebene;
- Figur 6: eine Schnittansicht mit durch die Pfeile B-B bezeichneten Schnittebene.
- Figur 7: eine Draufsicht auf das Gerät nach Figur 1 mit anders gestaltetem Display;
- Figuren 8,9: eine weitere Ausführungsform der Anordnung von Stechelementen;
- Figuren 10-13: eine weitere Ausführungsform der Anordnung von Stechelementen;
- Figur 14: einen beispielhaften Antriebsmechanismus für die Anordnung Figuren 10 - 13;
- Figuren 15, 16: eine weitere Ausführungsform der Anordnung von Stechelementen mit Schutzkappenmittel;
- Figur 17: das Entfernen der Schutzkappenmittel von den Stechelementen;
- Figur 18: einen Aktivierungs- und Auslösevorgang der Blutentnahmevorrichtung;
- Figuren 19-21: eine explosionsartige Darstellung sowie eine Ansicht von oben und unten einer weiteren Ausführungsform einer Anordnung von Stechelementen;
- Figuren 22-28: einen Betätigungszyklus der Blutentnahmevorrichtung unter Verwendung der Anordnung von Stechelementen nach Figuren 19-21; und
- Figur 29: eine weitere Ausführungsform des Blutanalysegeräts;
- Figuren 30-32: einen Betätigungszyklus der Blutentnahmevorrichtung bei der Ausführungsform nach Figur 29;
- Figur 33: eine perspektivische Ansicht einer bevorzugten Ausführungsform einer erfindungsgemäßen Stechvorrichtung;
- Figur 34: eine perspektivische Ansicht in das Innere der Stechvorrichtung nach Figur 33 mit weggelassenem Deckelteil;
- Figuren 35 und 36: in explosionsartiger Darstellung in einem Gehäusekörper der Stechvorrichtung nach Figuren 33 und 34 dargestellte Komponenten;
- Figuren 37 und 38: ein Stechelement der erfindungsgemäßen Stechvorrichtung;
- Figuren 39a bis c: den Herstellungsvorgang eines Stechelements;
- Figur 40: in perspektivischer Ansicht einen Träger für die Stechelemente;
- Figur 41: in perspektivischer Ansicht ein Haltemittel für die Stechelemente am Träger, welches zugleich ein Verdrängungsmittel für die Schutzkappenmittel bildet;
- Figuren 42, 43: eine perspektivische Ansicht des Trägers mit Stechelementen vor bzw. nach der Benutzung;
- Figuren 44 und 45: eine perspektivische Ansicht des Gehäusekörpers der Stechvorrichtung;
- Figur 46: eine perspektivische Ansicht des Stoßorgans der Stechvorrichtung;

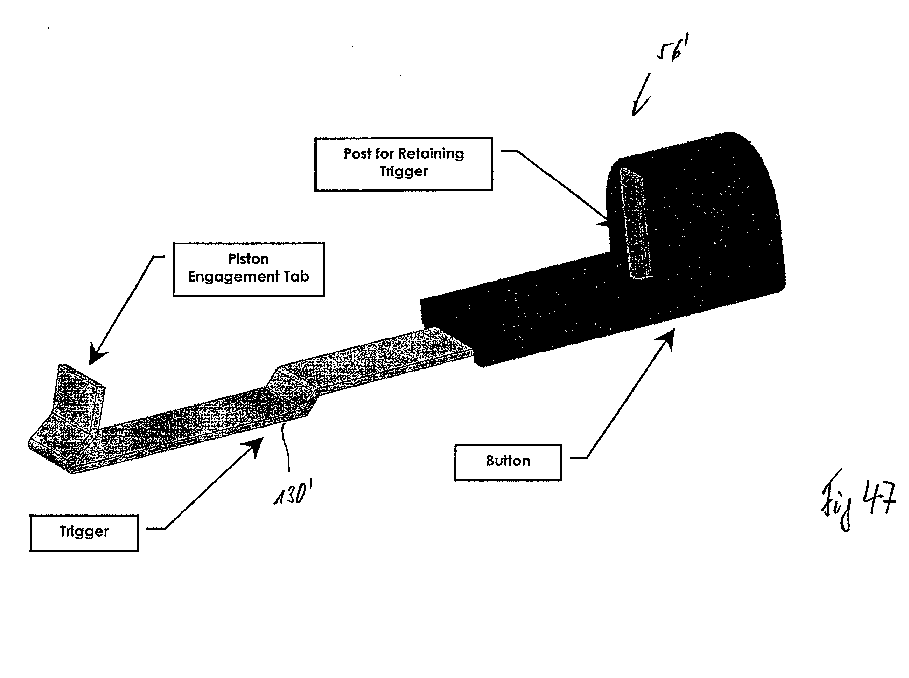

- Figur 47: eine perspektivische Ansicht des Auslösemittels der Stechvorrichtung;
- Figur 48: eine perspektivische Ansicht des Spannmittels der Stechvorrichtung und
- Figur 49: eine Ansicht auf die Stechvorrichtung von unten mit weggelassenem unterem Deckelteil.

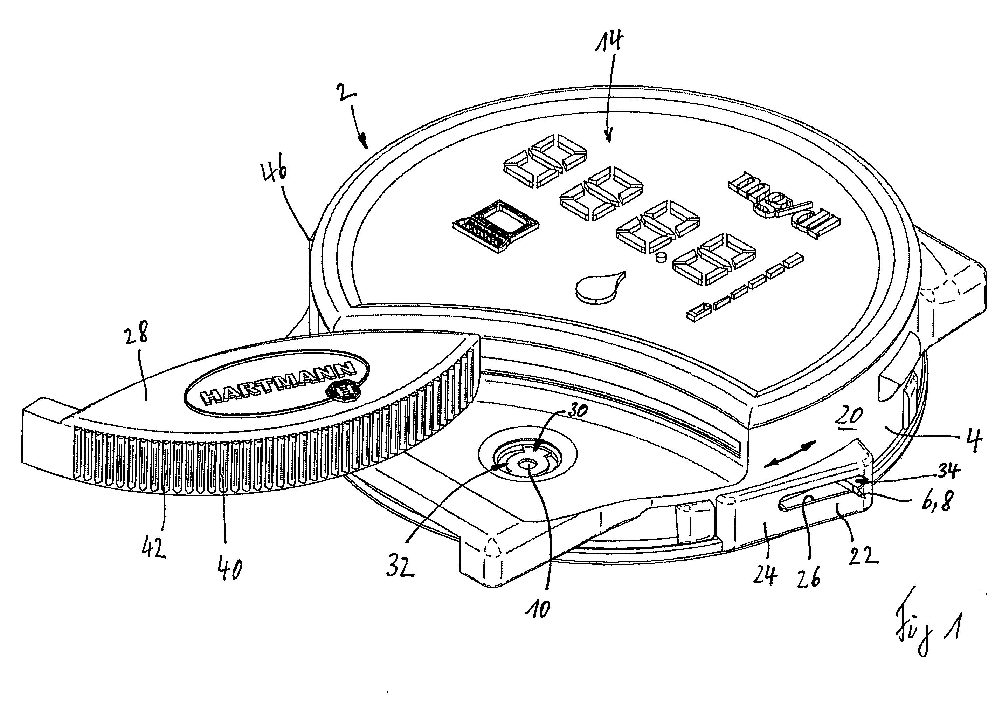

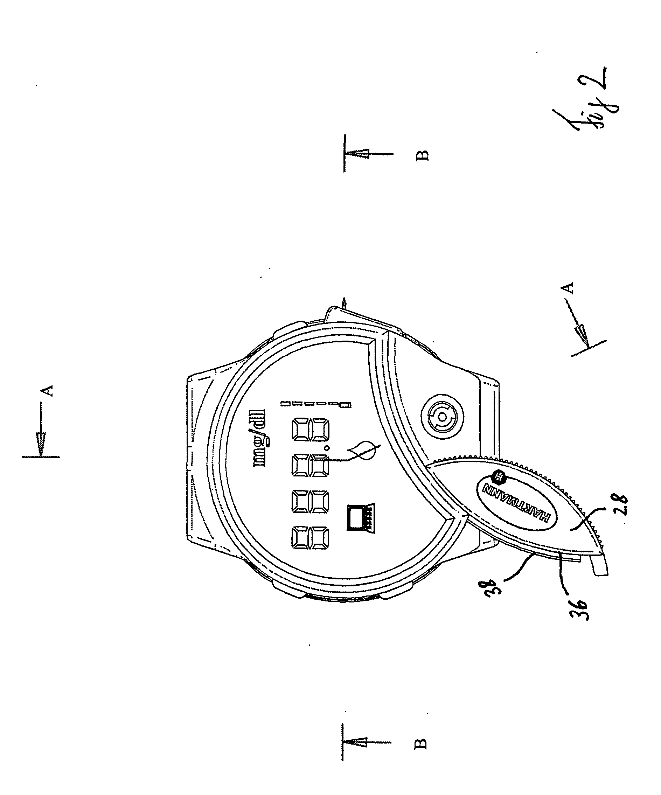

Die Figuren 1 und 2 zeigen als perspektivische Ansicht und als Draufsicht ein erfindungsgemäßes Blutanalysegerät, welches insgesamt mit dem Bezugszeichen 2 bezeichnet ist. Das Blutanalysegerät ist als sogenanntes Komplettgerät ausgebildet und umfasst einen noch näher bezeichneten Gehäusekörper 4, in dem eine Blutentnahmevorrichtung 6 mit einer Vielzahl von Stechelementen 8 sowie einer Vielzahl von Testmitteln 10 aufgenommen ist. Ungebrauchte Stechelemente 8 und Testmittel 10 werden magaziniert in den Gehäusekörper 4 eingesetzt und nach Gebrauch wieder entnommen, um verworfen bzw. entsorgt zu werden. Das Blutanalysegerät 2 umfasst ferner eine nur angedeutete Auswerteeinrichtung 12 mit einer nicht darstellbaren Auswerteelektronik sowie eine Anzeigeeinrichtung 14 in Form eines visuell auslesbaren Displays zur Anzeige des Ergebnisses einer Auswertung, also insbesondere der Anzeige der Menge eines Analyten, insbesondere des Blutzuckergehalts.

Der Gehäusekörper 4 des erfindungsgemäßen Blutanalysegeräts 2 ist kreisscheibenförmig nach Art des Gehäuses einer Armbanduhr ausgebildet. Er umfasst auch einander gegenüberliegend Befestigungsmittel 16 in Form von jeweils zwei miteinander fluchtenden Öffnungen 18 zur Aufnahme eines Steges eines an sich üblichen Uhrenarmbands.

An einer zylinderabschnittförmigen Wandung 20 des Gehäusekörpers 4 ist eine Stechposition 22 zum Anlegen einer Hautoberfläche, insbesondere eines Fingers eines Benutzers, ausgebildet. Die Stechposition 22 ist durch einen gegenüber der Wandung 20 in Umfangsrichtung bewegbaren Schieber 24 gebildet, der eine in Umfangsrichtung erstreckte langlochförmige Durchgriffsöffnung 26 aufweist, durch die ein Stechelement 8 hindurchstoßbar ist, so dass die Hautoberfläche des Benutzers zur Gewinnung einer Minimalmenge von Blut gestochen werden kann. Durch Verstellen des Schiebers 22 in Umfangsrichtung kann die Einstechtiefe des Stechelements 8 eingestellt werden. Das Stechelement 8 in Figur 1 ist in seiner maximal ausgestoßenen Position dargestellt, welche das Stechelement nur extremst kurzzeitig während der Ausführung des Stechvorgangs einnimmt. Im nicht betätigten Zustand der Blutentnahmevorrichtung 6 befindet sich das Stechelement 8 innerhalb des Gehäusekörpers 4.

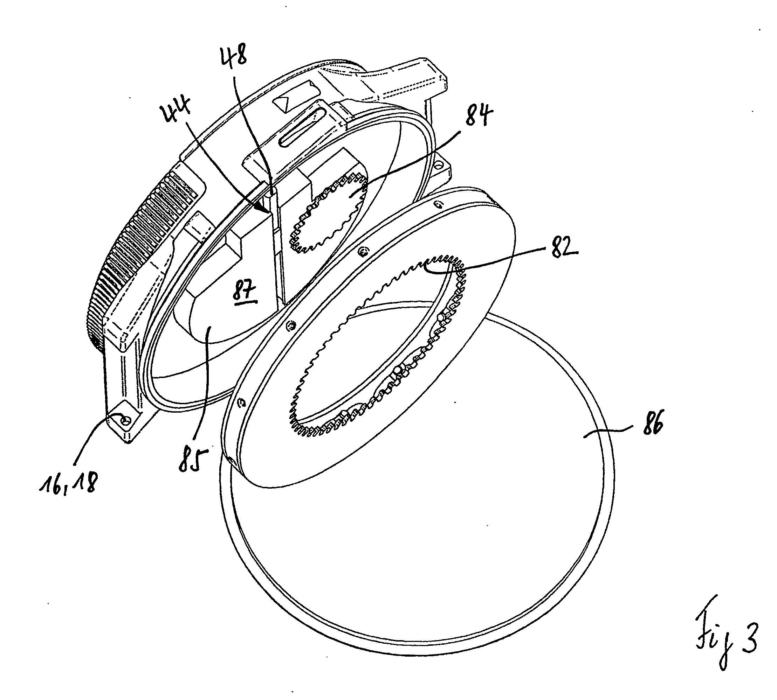

Wie aus den Figuren 1 und 2 ersichtlich ist, lässt sich ein in der Draufsicht konvexes Segment 28 des Gehäusekörpers 4 aus dem Gehäusekörper 4 teilweise herausbewegen, so dass das Segment 28 eine Aufgabeposition 30, die axial zur Ebene des scheibenförmigen Gehäusekörpers 4 orientiert ist, freigibt. In dieser Aufgabeposition 30 wird eine Mininmalmenge von Blut an ein unmittelbar unterhalb dieser Aufgabeposition angeordnetes Testmittel 10 aufgegeben. Wenn sich das Testmittel 10 in der aus Figuren 1 und 3 ersichtlichen, der Aufgabeposition 30 am Gehäusekörper 4 zugeordneten Position befindet, in der es mit einer Minimalmenge von Blut benetzbar ist, so befindet es sich definitionsgemäß in seiner Arbeitsposition. In entsprechender Weise befindet sich das aus Figuren 1 und 3 ersichtliche Stechelement 8 in seiner der Stechposition 22 zugeordneten Arbeitsposition, in der es auf noch näher zu beschreibende Weise in die Hautoberfläche eines Benutzers einstechbar ist.

Das Segment 28 ist entlang einer nach innen gewandten konvex gekrümmten Seite 36 über eine Gleitführungsschiene 38 mit Hintergriff am übrigen Gehäusekörper 4 verschieblich gehalten. Es kann die Aufgabeposition 30 im nichtbetätigten Zustand des Blutanalysegeräts abdecken, so dass seine mit einer geriffelten Angriffsfläche 40 versehene andere konvex gekrümmte Seite 42 einen Teil der zylindrischen Außenseite 20 des Gehäusekörpers 4 bildet.

Wenn eine Blutanalyse durchgeführt werden soll, so schiebt der Benutzer das Segment 28 in die in Figur 1 dargestellte Stellung, so dass die Aufgabeposition 30 freigegeben wird. Durch diese Bewegung wird gleichzeitig die im Inneren des Gehäusekörpers 4 vorgesehene Blutentnahmevorrichtung 6 aktiviert, indem ein aus Figur 3 ersichtlicher Stoßmechanismus 44 gespannt wird, indem mechanisch eine Feder gespannt wird. Der Benutzer kann nun einen Finger an die Stechposition 22 anlegen und die Blutentnahmevorrichtung 6 durch Betätigen eines Tasters 46 auslösen. Dabei wird ein Stößel 48 des Stoßmechanismus 44 durch die sich entspannende Federkraft in radialer Richtung nach außen gestoßen. Der Stößel 48 stößt dann das in seiner Arbeitsposition 34 befindliche Stechelement 6 in radialer Richtung nach außen, so dass dieses durch die Durchgriffsöffnung 26 in die Hautoberfläche eines Benutzers eingestochen wird. Der Benutzer löst nun seinen Finger von der Stechposition 22, lässt eine Minimalmenge von Blut aus seinem Finger austreten und legt dann den Finger an die Aufgabeposition 30 und überträgt dabei eine Minimalmenge von Blut an das in seiner Arbeitsposition 32 befindliche Testmittel 10. Anschließend beginnt die Auswertung der Analyse, und das Ergebnis wird mittels der Anzeigeeinrichtung angezeigt.

Eine beispielhafte Darstellung der Anordnung von Stechelementen 8 und Testmitteln 10 sowie der Blutentnahmevorrichtung 6 mit ihrem Stoßmechanismus 44 im Inneren des Gehäusekörpers 4 läßt sich den Figuren 4 bis 6 entnehmen. Figur 4 zeigt eine perspektivische Ansicht eines insgesamt mit dem Bezugszeichen 50 bezeichneten Trägers für Stechelemente 8 und Testmittel 10, wobei der Träger 50 eine Art magazinartige Aufnahme für Stechelemente 8 und Testmittel 10 bildet und als manuell handhabbare Einheit in das Gerät einsetzbar und nach Gebrauch entnehmbar und als Ganzes zusammen mit den gebrauchten Stechelementen und Testmitteln entsorgbar ist.

Der Träger 50 weist einen ersten Trägerteil 52 in Form einer im wesentlichen ebenen Ringscheibe 54 für die Testmittel 10 und einen zweiten Trägerteil 56 in Form eines auf Kreisform gebogenen Bandes 58 mit in radialer Richtung angeordneten und daran gehaltenen Stechelementen 8 auf.

Der zweiter Trägeteil 56 ist auf einen weiteren ringscheibenförmigen Trägerteil 60 aufgesetzt, dessen Ebene parallel zu dem ersten Trägerteil 52 erstreckt ist. Der zweite Trägerteil 56 und dieser weitere Trägerteil 60 sind über Formausnehmungen 62 am einen Teil und darin eingreifende Vorsprünge 64 am anderen Teil drehfest miteinander gekoppelt. Die Formausnehmungen 62 und die Vorsprünge 64 können auch reibschlüssig miteinander zusammenwirken, so dass der zweite Trägerteil 56 mit dem weiteren Trägerteil 60 dauerhaft gefügt ist. Der erste Trägerteil 52 ist in ähnlicher Weise auf den zweiten Trägerteil 56 aufklipsbar, indem auch zwischen diesen beiden Teilen form- und/oder vorzugsweise auch klemmschlüssig Ausnehmungen 62 und Vorsprünge 66 zusammenwirken.

Die Testmittel 10 sind in konzentrisch auf dem ersten Trägerteil angeordneten Ausnehmungen 68 angeordnet, bei denen es sich um Ausstanzungen oder Freistanzungen handelt. Sie sind derart angeordnet, dass sie in axialer Richtung, also in Richtung einer Drehachse 70 des Trägers 50 zugänglich sind. An der Oberseite des ersten Trägerteils 52 sind bei jedem Testmittel 10 Kontakte 69 angedeutet, an denen eine nicht dargestellte Elektrodenanordnung der Testmittel kontaktierbar und mit der Auswerteelektronik verbindbar ist. Im vorliegenden Fall wird also mit elektrochemischen Testmitteln gearbeitet, wie sie hinlänglich bekannt sind und daher hier nicht eingehend beschrieben zu werden brauchen.

Die radial angeordneten Stechelemente 8 am zweiten Trägerteil 56 durchdringen jeweils ein Federelement 74, welches in radialer Richtung spannbar ist. Die Stechelemente 8 weisen radial innen einen verdickten Kopf 76 auf, welcher nicht durch das Federelement 74 hindurchbewegbar ist. Beim Ausstoßen des Federelements 74 in radialer Richtung wird daher das Federelement 74 gespannt, und es bewirkt daher ein radiales Zurückziehen des Federelements 74 im wesentlichen in die in Figur 4 dargestellte Position innerhalb des Gehäusekörpers 4. Die Federelemente 74 bilden daher Retraktionsmittel 78 für die Stechelemente 8.

Die drei Trägerteile 52, 56 und 60 sind ringartig ausgebildet und weisen eine zentrale Ausnehmung 80 auf, in der der Stoßmechanismus 44 wie aus Figur 3 ersichtlich untergebracht ist. Der weitere Trägerteil 60 umfasst eine Innenverzahnung 82, welche mit einem Antriebsrad 84 (s. Figur 3) zusammenwirkt. Das Antriebsrad 84 ist elektromotorisch oder in sonstiger Weise, etwa durch Betätigen eines Schiebers oder Weiterdrehen eines manuell betätigbaren Rades am Gehäuse des Blutanalysegeräts antreibbar. Der Träger 50 ist wie aus Figur 3 ersichtlich auf einem zentralen in die Ausnehmung 80 eingreifenden Gehäusevorsprung 85 drehbar gelagert. Dabei bildet eine ebene axiale Oberfläche 87 eine Axiallagerfläche, gegen welche die unmittelbar an die Innenverzahnung 82 anschließenden Oberflächenbereiche des weiteren Trägerteils 60 gleitend anliegen.

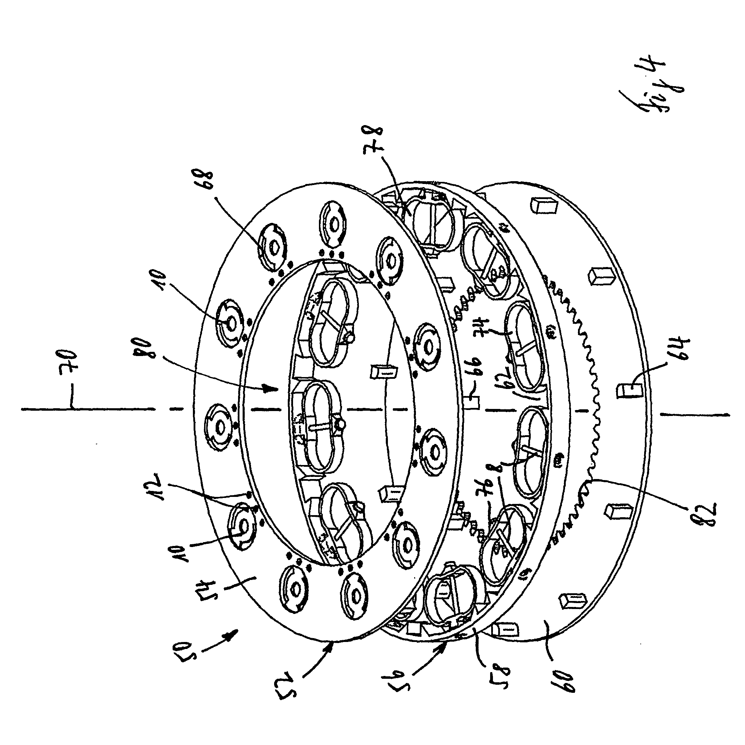

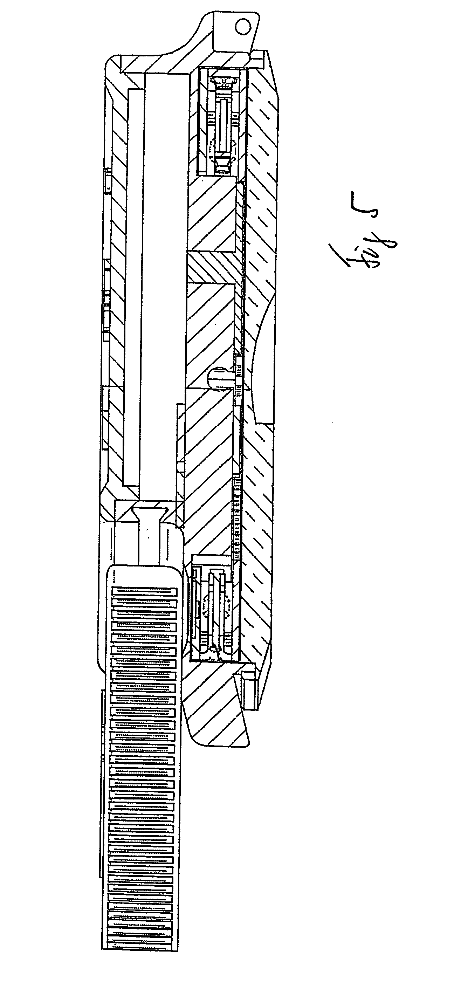

In der Schnittansicht nach Figur 5 verläuft die Schnittebene durch die Arbeitsposition 32 der Testmittel 10. In der Schnittansicht ist zwar auch ein Stechelement 8 erkennbar; es befindet sich jedoch nicht in der Arbeitsposition für Stechelemente. Die Arbeitsposition für Stechelemente ist von der Schnittebene nach Figur 6 erfasst, wobei das Stechelement 8 in maximal ausgestoßener Position dargestellt ist. Der in Figur 3 dargestellte Stoßmechanismus 44 ist in der Schnittansicht nach Figur 6 nicht gezeichnet. Man erkennt aus den Figuren 3, 5 und 6, wie der Träger 50 mit den Stechelementen 8 und Testmitteln 10 kassettenartig in das Innere des Gehäusekörpers 4 des Blutanalysegeräts einsetzbar ist. Der Gehäusekörper 4 wird von unten durch ein Deckelteil 86 und nach oben durch eine quer zur Drehachse 70 verlaufende Wandung 88 begrenzt. Oberhalb dieser Wandung 88 ist ein weiterer Raum 90 für die Auswerteeinrichtung 12 und deren elektronische Komponenten vorgesehen.

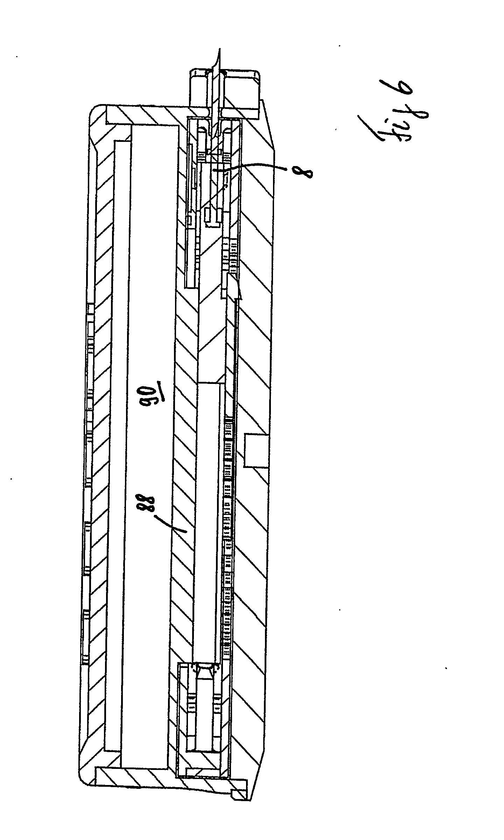

Figur 7 zeigt eine der Figur 2 entsprechende Draufsicht auf ein Blutanalysegerät, welches sich von dem vorstehend beschriebenen dadurch unterscheidet, dass auf der Sichtseite des Gehäusekörpers 4 eine Zeitanzeigeeinrichtung 92 in Form eines an sich üblichen Uhrenziffernblatts vorgesehen ist. Diese Zeitanzeige könnte aber auch in Form eines LCD-Displays entsprechend Figur 2 ausgebildet sein. Vorzugsweise kann solchenfalls zwischen einer Zeitanzeige und einem das Ergebnis der Blutanalyse anzeigenden Betriebsmodus gewählt werden.

Nachfolgend werden weitere Ausführungsformen zur Ausbildung der Blutentnahmevorrichtung erörtert. Figur 8 zeigt in perspektivischer Ansicht eine Anordnung von Stechelementen innerhalb von radial angeordneten Hülsenmitteln 100, die an einem auf geschlossene Kreisform gebogenen Band 102 als Trägerteil 104 angeordnet sind. Von radial innen erstrecken sich Kolbenmittel 106 in die Hülsenmittel 100 hinein, wobei die Hülsenmittel 100 eine zylinderförmige Aufnahme bilden. Die Kolbenmittel 106 tragen die aus Figur 8 nicht ersichtlichen Stechelemente, die dann in radialer Ausrichtung innerhalb der Hülsenmittel 100 aufgenommen sind. Am radial inneren Ende der Kolbenmittel 106 sind jeweils zwei Federzungen 108 in Form von zur radialen Richtung geneigten Stegen vorgesehen. Wenn ein jeweiliges Kolbenmittel 106 in Richtung des Pfeils 110 nach radial außen gestoßen wird, so werden die Federzungen 108 verformt und üben eine Retraktionskraft auf das jeweilige Kolbenmittel 106 aus, so dass dieses wieder zumindest geringfügig zurückgezogen wird.

Die Hülsenmittel 100 weisen auf ihrer radial äußeren Seite eine Abdeckung 112 in Form eines Folienabschnitts auf, der von dem Stechelement entweder durchstoßen werden kann oder unmittelbar vor der Ausführung des Stechvorgangs ganz oder teilweise entfernt wird.

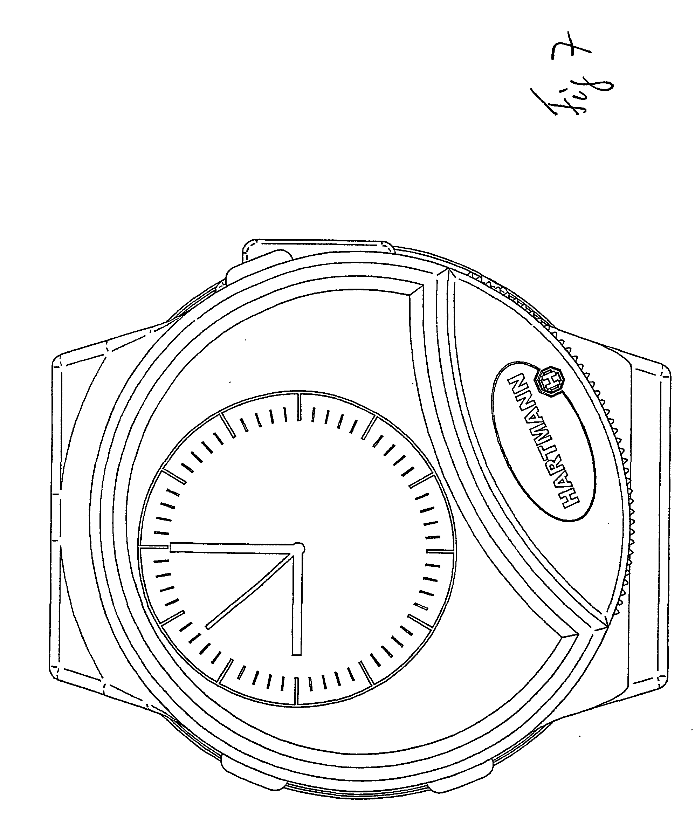

Figur 9 zeigt einen in Figur 8 dargestellten Ausschnitt in Vergrößerung. Man erkennt das Kolbenmittel 106 mit eingespritztem Stechelement 114. Zwischen der inneren Zylinderwandung 116 des Hülsenmittels 100 und dem Kolbenmittel 106 ist ein Abdichtmittel in Form einer Ringwulst 118 an der Wandung 116 und einer Ringwulst 120 am Kolbenmittel 106 vorgesehen. Wenn das Kolbenmittel 106 in Richtung des Pfeils 100 in radialer Richtung ausgestoßen wird, so gleitet die Ringwulst 120 unter Wirkung der Zylinderwandung 116 über die Ringwulst 118. Vor der Ausführung des Stoßvorgangs bilden die beiden Ringwülste 118, 120 eine Abdichtung des Zylinderraums, so dass das Stechelement 114 unter sterilen und abgeschlossenen Bedingungen aufgenommen ist. Auf der anderen Seite ist der Zylinderraum durch die Folie 112 abgedichtet.

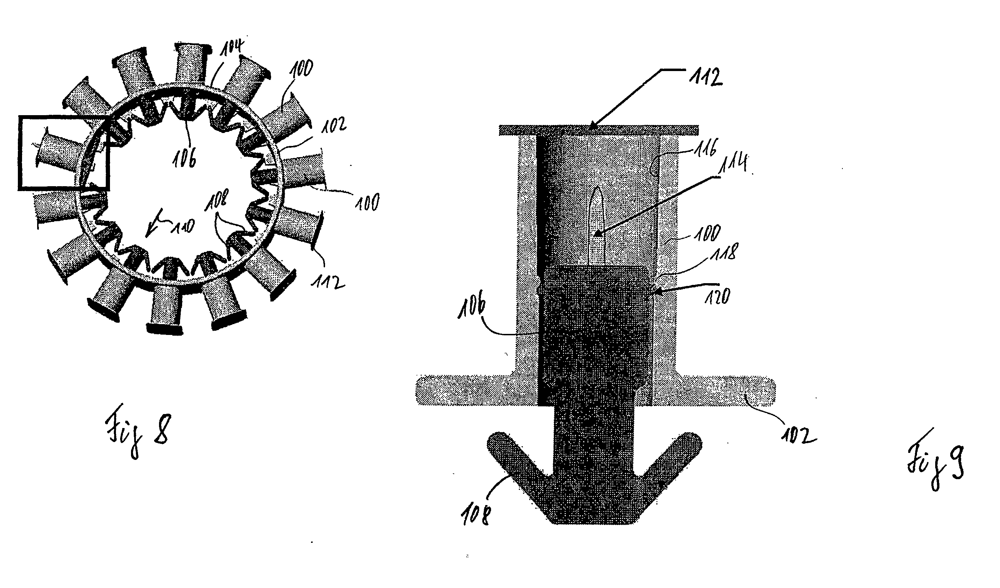

Figur 10 zeigt eine weitere Ausführungsform, bei der die Stechelemente 122 in halbkugelförmigen Vertiefungen 124 eines scheibenförmigen Trägerteils 126 aufgenommen und in axialer Richtung, also parallel zur Drehachse 70 des Trägerteils 126 erstreckt sind.

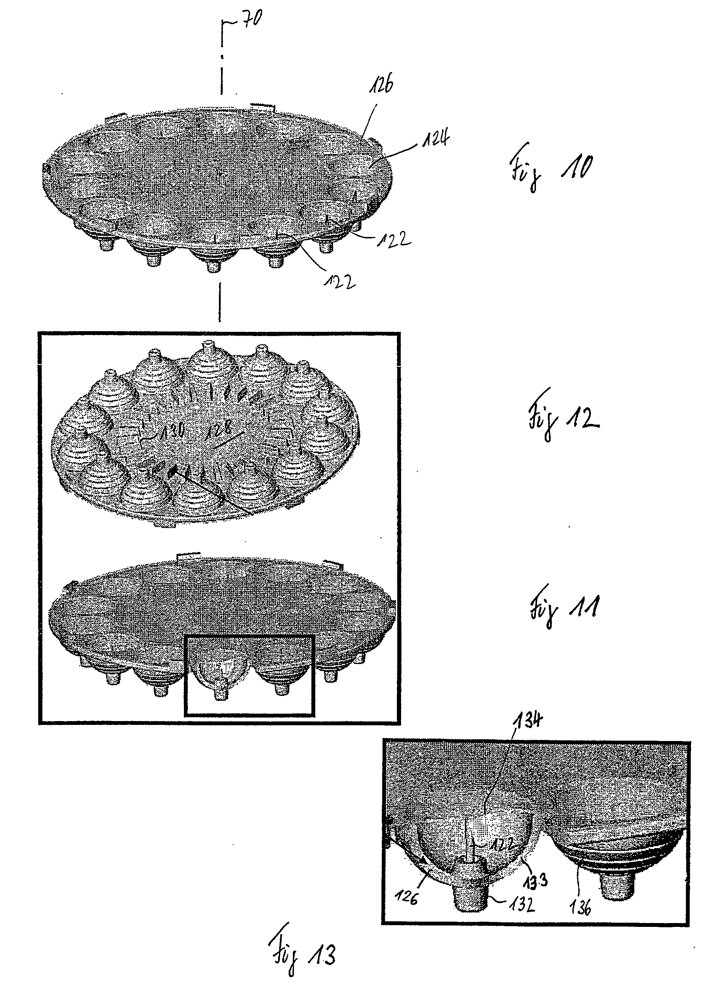

Die Figuren 11 und 12 zeigen zwei perspektivische Ansichten des Trägerteils 126 nach Figur 10. Man erkennt auf der Unterseite 128 eine Stirnverzahnung 130 zum rotierenden Antrieb des Trägerteils 126. Anstelle der Stirnverzahnung könnte aber auch eine Ausnehmung mit einer Innenverzahnung vorgesehen sein.

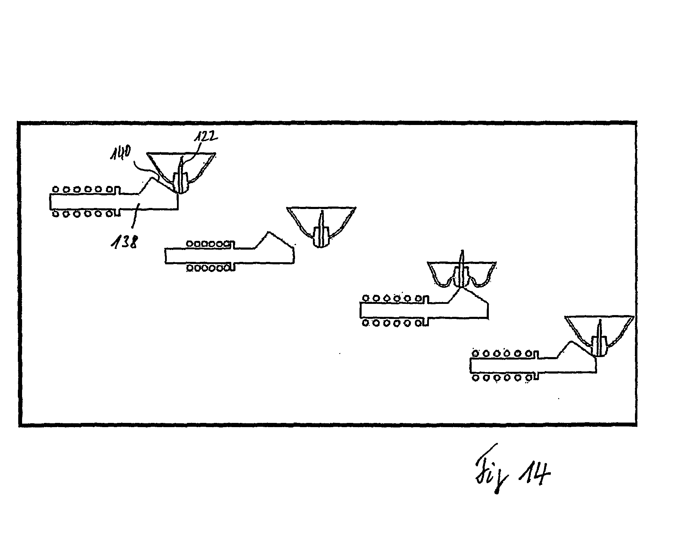

Wie sich aus der Detaildarstellung nach Figur 13 erkennen lässt, sind die Stechelemente 122 in stammförmige Trägerabschnitte 132 eingespritzt, die einstückig mit dem Material der halbkugelförmigen Wandungen 133 ausgebildet sind, welche die Vertiefungen 124 begrenzen. Die Vertiefungen 124 sind oben von einer Folie 134 überfangen, die dann einen keimdicht abgeschlossenen Raum zur Aufnahme des jeweiligen Stechelements 122 bildet. Des weiteren erkennt man ringförmige Schwächungsnuten 136 in der die Vertiefungen 124 begrenzenden Randung des Trägerteils 126.

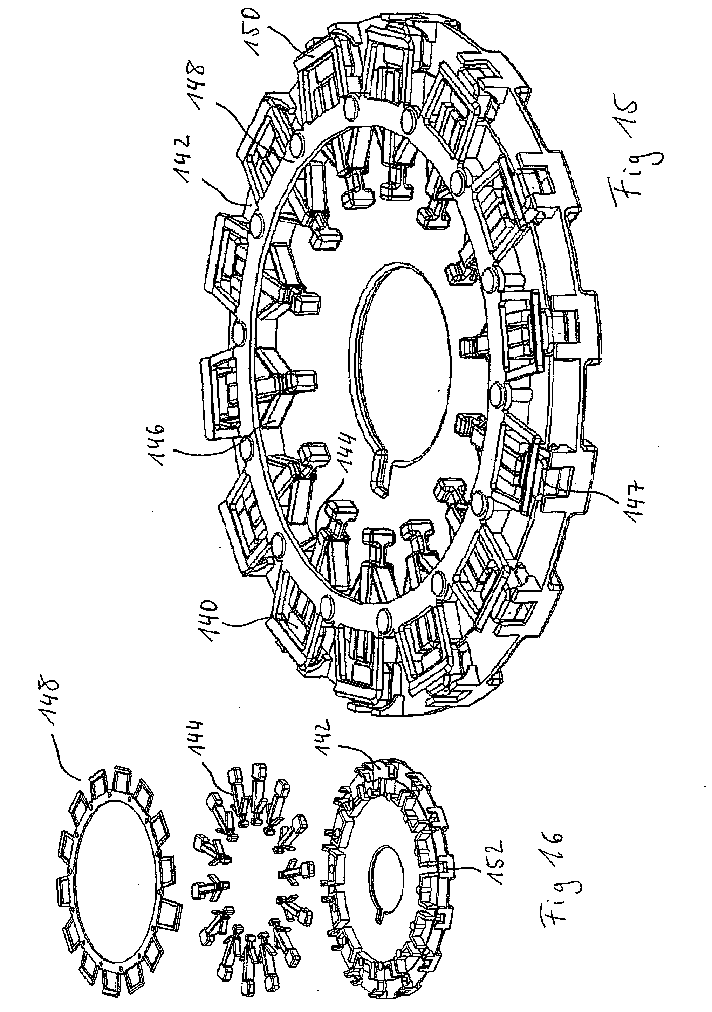

Figur 14 verdeutlicht den Antriebsmechanismus für die Blutentnahmevorrichtung bei der axialen Anordnung der Stechelemente 122. Beispielsweise ist es möglich, einen radial wirkenden Stoßmechanismus mit einem radial bewegbaren Stoßorgan 138 zu verwenden, wobei die radiale Bewegung über eine keilförmige Anlaufschräge 140 zur axialen Auslenkung der Stechelemente 122 verwendet werden kann. Die Anlaufschräge 140 gleitet gegenüber dem stammförmigen Trägerabschnitt 132, welcher das Stechelement 122 hält und lenkt dieses unter Verformung der Muldenform in axialer Richtung aus, so wie dies in Figur 14 in vier verschiedenen Zuständen dargestellt ist.

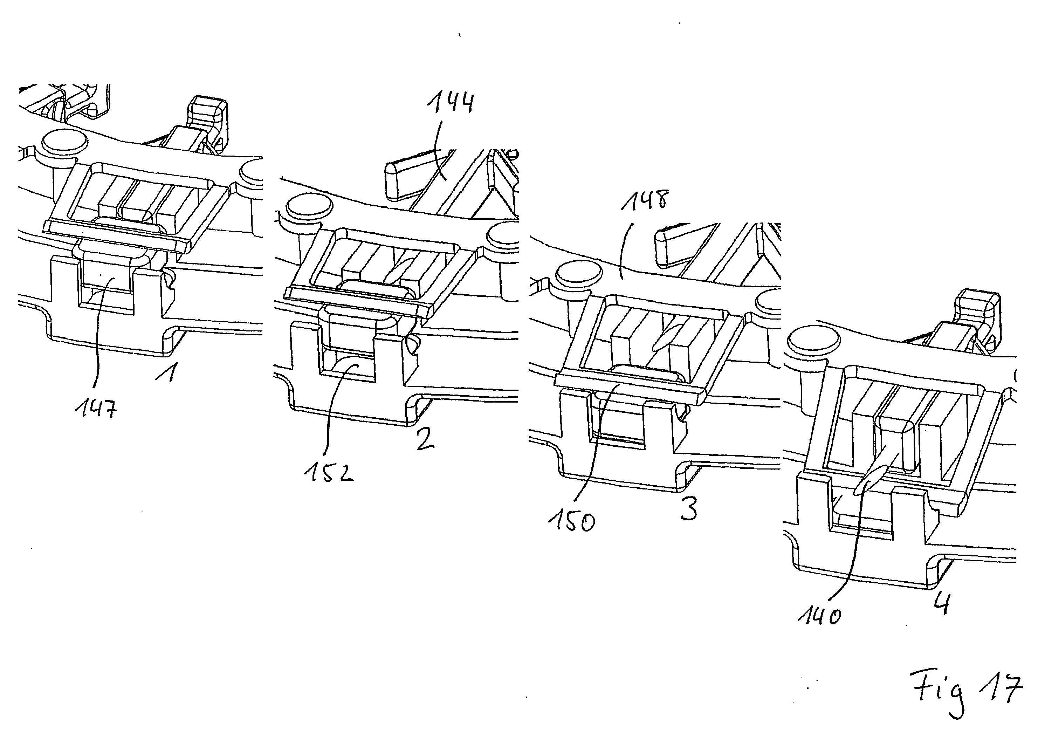

Figuren 15 und 16 zeigen in perspektivischer bzw. explosionsartiger Darstellung eine weitere Anordnung von Stechelementen 140 als Teil der Blutentnahmevorrichtung auf einem drehbaren Trägerteil 142. Die Stechelemente 140 sind wiederum in radialer Ausrichtung angeordnet und mit einem Ende in einen Haltestößel 144 aus Kunststoff eingespritzt. An dem Haltestößel 144 sind wie bei den Kolbenmitteln nach Figur 8 Federzungen 146 angeformt, die sich bei radialer Bewegung der Haltestößel 144 und damit der Stechelemente 140 spannen und ein dann Zurückziehen der Stechelemente bewirken. Der Trägerteil 142 weist einstückig angeformte Lagervorsprünge zur Aufnahme und radial verschieblichen Lagerung der Haltestößel 144 mit Stechelementen 140 auf. Die in die Haltestößel 144 eingespritzten Stechelemente 140 sind vorzugsweise vollständig umgeben von einem Schutzkappenmittel 147, welches eine sterile Umgebung und damit eine Sterilitätsbarriere für die Stechelemente 140 bildet.

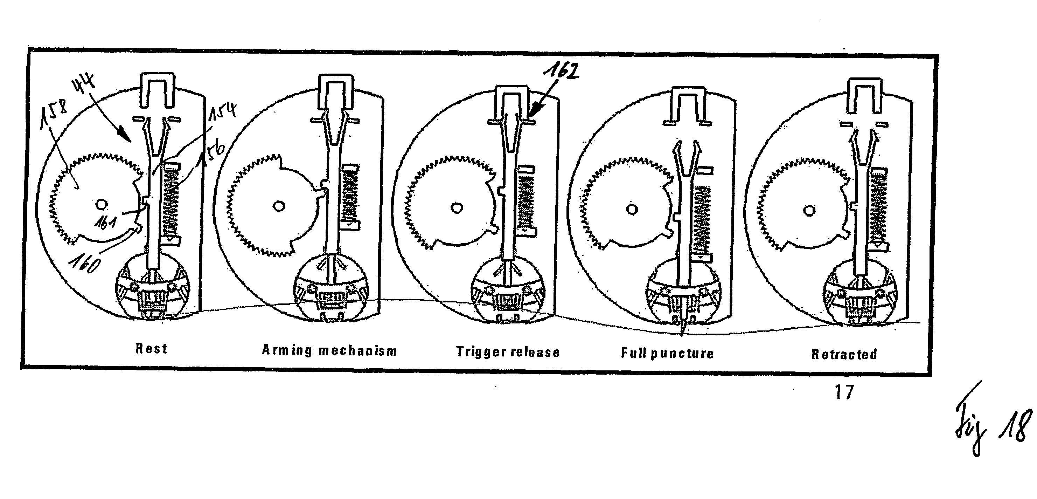

Ein Federring 148 wird von oben durch an sich beliebige Fügemittel mit dem Träger 142 vorzugsweise unlösbar und derart verbunden, dass die Haltestößel 144 in radialer Richtung beweglich, jedoch unverlierbar gehalten sind. Dabei übt ein oberhalb eines jeweiligen Schutzkappenmittels 147 angeordneter Steg 150 des Federrings 148 einen geringfügigen Druck in axialer Richtung auf das Schutzkappenmittel 147 aus. Der Stoßmechanismus der Blutentnahmevorrichtung ist im vorliegenden Fall derart ausgebildet, dass unmittelbar vor Ausführung eines Stoß- bzw. Stechvorgangs der in der Arbeitsposition befindliche Haltestößel 144 geringfügig nach radial innen gezogen wird. Dabei wird das Schutzkappenmittel 147 durch Anschlagsmittel zunächst in seiner Position gehalten, so dass das betreffende Stechelement 140 aus dem Schutzkappenmittel 147 freikommt. Das Schutzkappenmittel wird dann unter der Wirkung des federnden Stegs 150 in eine Formausnehmung 152 in dem Trägerteil 142 bewegt, so dass es aus dem Bewegungspfad des Stechelements 140 verbracht wird. Dieser Vorgang des Zurückziehens des Haltestößels 144, des Lösens des Schutzkappenmittels 147 und des Verbringens des Schutzkappenmittels aus dem Bewegungspfad des Stechelements ist in Figur 17 dargestellt.

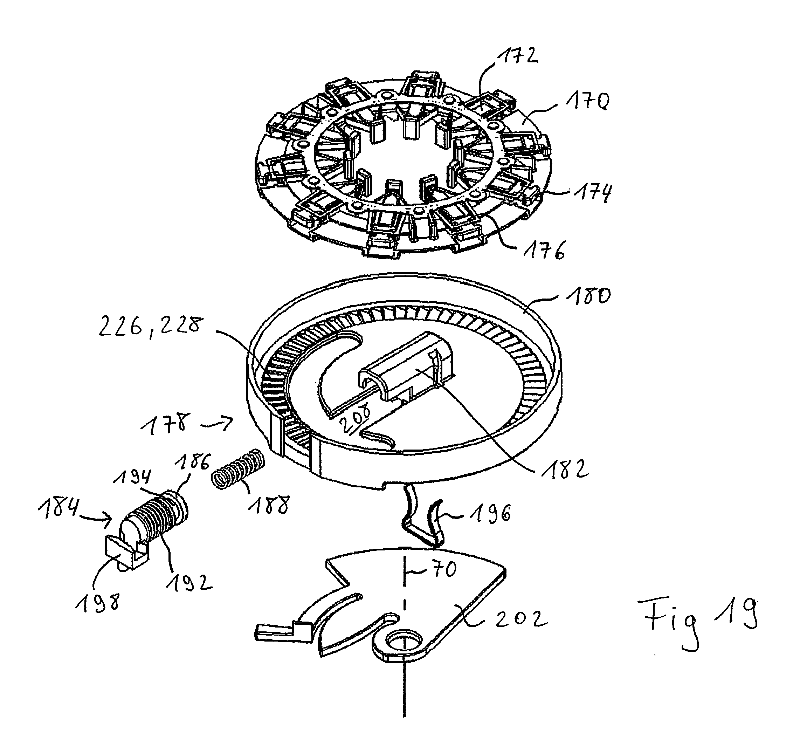

Figur 18 zeigt schematisch die Aktivierbarkeit der Blutentnahmevorrichtung. Mit dem Bezugszeichen 44 ist insgesamt ein Stoßmechanismus bezeichnet, der ein stößelartiges Stoßorgan 154 umfasst, welches mittels einer Zug- und Druckfeder 156 in radialer Richtung ausstoßbar und wieder zurückziehbar ist. Mittels eines drehbaren Stellmittels 158 mit einer radial vorstehenden Nocke 160 kann der Stoßmechanismus 44 gespannt werden, indem durch Zusammenwirken der Nocke 160 mit einem Vorsprung 161 an dem Stoßorgan 154 dieses entgegen der Druckkraft der Feder 156 bewegt werden kann, so dass es über einen Rastmechanismus 162 unter Beibehaltung der Federspannung in einem aktivierten Zustand gehalten wird. Das Stellmittel 158 mit der Nocke 160 ist insbesondere federbelastet in seine Ausgangsposition zurückbewegbar. Wird nun die Verrastung manuell gelöst, so schnellt das Stoßorgan 154 schlagartig unter der Wirkung der Federkraft in radialer Richtung, wobei das freie Ende des Stoßorgans 154 auf das innere Ende des Stechelements oder eines das Stechelement tragenden Halters auftrifft und dieses ebenfalls nach außen stößt. Über die Feder 156 wird dann das Stoßorgan 154 wieder in seine in der Figur 18 dargestellte Ausgangsposition versetzt. Das Stechelement wird dabei unter der Wirkung der vorstehend beschriebenen Federmittel wieder in das Gehäuse zurückgezogen.

Die Figuren 19 bis 29 veranschaulichen eine weitere bevorzugte Ausführungsform der Anordnung von Stechelementen auf einem drehbaren Träger.

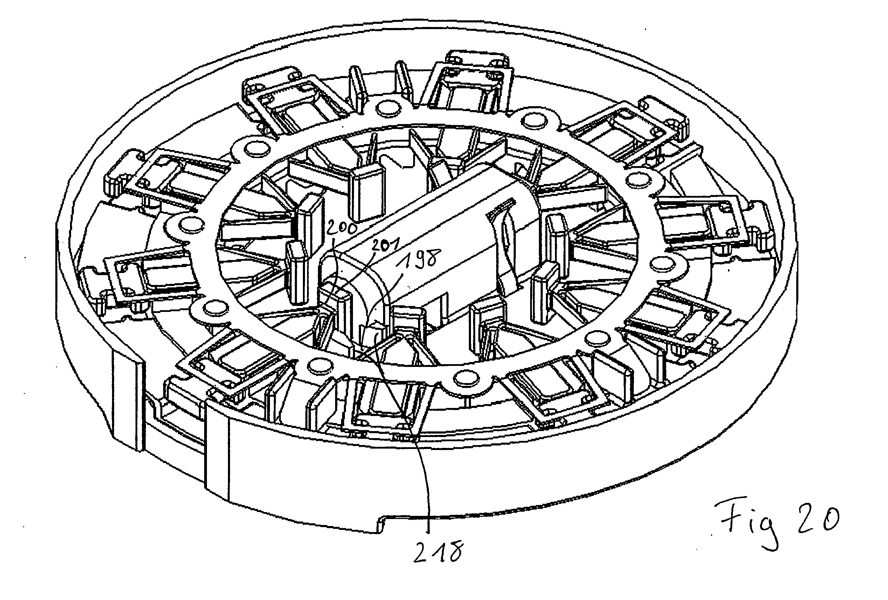

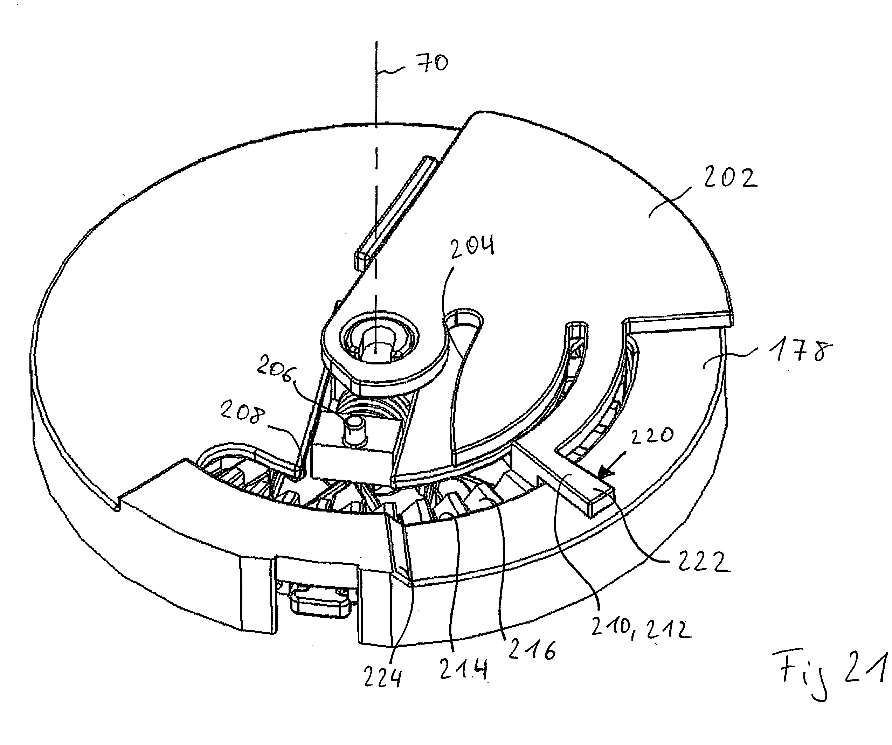

Figur 19 zeigt eine explosionsartige Darstellung einer Stechelemente-Kassette, die in Figur 20 im zusammengebauten Zustand von oben und in Figur 21 von unten dargestellt ist.

Sie umfasst ein Trägerteil mit radial bewegbar angeordneten Haltestößeln 172 mit Stechelementen, welche durch ein Schutzkappenmittel 174 abgedeckt sind, wobei die Anordnung weitestgehend derjenigen nach Figur 15 entspricht. Sie weist auch ein ringförmiges Federmittel 176 auf, welches mit jedem Schutzkappenmittel 174 so wie im Zusammenhang mit Figur 17 beschrieben zusammenwirkt. Die vorstehend beschriebenen Komponenten sind in ein weiteres Trägerteil 178 mit einem zylindrischen, nach oben gezogenen Wandbereich 180 einsetzbar. Dieses weitere Trägerteil 178 weist im Zentrum eine in das Innere vorspringende gehäusebildende Aufnahme 182 für ein stößel- oder kolbenartiges Stoßorgan 184 auf, welches darin radial bewegbar ist. Zwischen einem radial inneren Ende 186 des Stoßorgans 184 und einer Wandung der gehäusebildenden Aufnahme 182 ist eine erste Antriebsfeder 188 vorgesehen. Zwischen einem Ringbund 190 des Stoßorgans 184 und der Aufnahme 182 ist eine Rückstellfeder 192 vorgesehen.

Das Stoßorgan 184 weist des weiteren an seinem Umfang ein Haltemittel 194, beispielsweise in Form einer Ringnut, auf, mittels dessen das Stoßorgan 184 bei gespannter Feder 188 in der Aufnahme 182 geahlten werden kann. Dies kann durch einen Rastmechanismus erreicht werden, der vorliegend beispielhaft durch eine Klammerfeder 196 gebildet ist und mit dem Haltemittel zusammenwirkt. Das Stoßorgan 184 weist an seinem radial äußeren Ende ein Hintergriffsmittel 198 auf, welches zum Zurückziehen der Haltestößel 172 dient, um die Schutzkappenmittel 174 zu lösen. Dieses Hintergriffsmittel hintergreift einen hintergreifbaren Abschnitt 200 der Haltestößel 172. Dieser hintergreifbare Abschnitt kann von einem verdickten und daher auch zur Aufnahme einer Stoßkraft ausgebildeten radial inneren Ende 201 der Haltestößel 172 gebildet sein.

Auf der Außenseite des Trägerteils 178 ist ein flächenhaft ausgebildeter Steuerhebel 202 um die Drehachse 70 des Trägerteils 170 drehbar vorgesehen. Der Steuerhebel 202 umfasst eine Nockensteuerfläche 204, welche mit einem Nockenmittel 206 des Stoßorgans 184 zusammenwirkt, welches sich in axialer Richtung durch eine Ausnehmung 208 im Boden des Trägerteils 178 erstreckt. Wenn der Steuerhebel 202 in Figur 21 in Uhrzeigersinnrichtung verschwenkt wird, so gelangt die Nockensteuerfläche 204 in Wirkkontakt mit der Nocke 206 und zwingt die Nocke 206 und mit ihr das Stoßorgan 184 beim Weiterdrehen des Steuerhebels 202 in radialer Richtung nach innen, so dass die Antriebsfeder 188 gespannt wird. Mittels des vorstehend beispielhaft beschriebenen Halte- oder Rastmechanismus lässt sich das Stoßorgan 184 im gespannten Zustand der Feder 188 arretieren, auch wenn der Steuerhebel 202 wieder in die in Figur 21 gezeigte Ausgangsposition zurückgedreht wird.

Der Steuerhebel 202 weist des weiteren ein Mitnahmemittel 210 in Form eines in axialer Richtung federnden Rasthebels 212 auf, mittels dessen der Trägerteil 170 und damit die Anordnung der Stechelemente in Umfangsrichtung drehbar ist. Hierfür greift der Rasthebel 212 durch eine in Umfangsrichtung erstreckte Ausnehmung 214 im Boden des Trägerteils 178 hindurch und ergreift den Trägerteil 170. Hierfür weist der Trägerteil in vorteilhafter Weise eine nur aus Figur 21 ersichtliche Stirnrastenanordnung 216 auf.

Beim Drehen des Steuerhebels 202 ausgehend von der in Figur 21 dargestellten Position bewirkt der Rasthebel 212 ein Weiterdrehen des Trägerteils 170, so dass ein neues, noch ungebrauchtes Stechelement in seine Arbeitsposition gebracht wird. Dabei gleitet der betreffende Haltestößel 172 mit seinem hintergreifbaren Abschnitt 200 in die aus Figur 20 ersichtliche Hintergriffsituation mit dem Hintergriffsmittel 198 des Stoßorgans 184. Um diese Positionierbewegung zu vereinfachen, erweist es sich als besonders vorteilhaft, wenn das Hintergriffsmittel 198 mit einer aus Figur 20, aber auch aus Figur 19 ersichtlichen Anlaufschräge oder Einführschräge 218 versehen ist. Über diese Anlaufschräge 218, die vorzugsweise radial innen und radial außen ausgebildet ist, lassen sich Positionierungenauigkeiten im Ruhezustand des Stoßorgans 184 ausgleichen.

Um sicherzustellen, dass ein jeweiliges Stechelement exakt in der Arbeitsposition der Stechelemente positioniert und nicht versehentlich weitertransportiert wird, ist exakt in dieser Drehstellung eine Transportunterbrechungseinrichtung 220 vorgesehen. Diese Transportunterbrechungseinrichtung 220 ist beispielhaft und in vorteilhafter Weise derart ausgebildet, dass sie eine axiale Auslenkung des Rasthebels 212 bewirkt, so dass dessen Mitnahmemittel 210 nicht mehr mit der Stirnrastenanordnung 216 zusammenwirken kann. Hierfür umfasst der Rasthebel 212 einen radialen Fortsatz 222, der gegen eine Außenseite des Trägerteils 178 gleitet. Diese Außenseite des Trägerteils 178 weist an entsprechender Stelle eine Auflaufschräge 224 auf, welche den Rasthebel dann im vorstehend beschriebenen Sinne in axialer Richtung anhebt, wenn der Steuerhebel 202 bei der Drehung diese Auflaufschräge 224 erreicht. Beim Zurückdrehen des Steuerhebels 202 gleitet das Mitnahmemittel 210 des Rasthebels 212 über schräg verlaufende Flanken der Stirnrastenanordnung, ohne den Trägerteil 170 jedoch zurückzudrehen. Um eine Rückdrehung des Trägerteils 170 sicher zu verhindern, ist eine Rückdrehungsverhinderungseinrichtung 226 vorgesehen, die in vorteilhafter Weise durch eine entsprechend orientierte Stirnrastenverzahnung 228 an der Innenseite des Trägerteils 178 verwirklicht sein kann, so wie dies beispielhaft aus Figur 19 ersichtlich ist. Ausbildung und Orientierung dieser Stirnrastenanordnung 228 ist derart, dass sie mit der Stirnrastenanordnung 216 des ersten Trägerteils 170 derart zusammenwirkt, dass sich das Trägerteil 170 in der Transportrichtung, nicht jedoch in Gegenrichtung drehen lässt. Hierfür ist eine geringfügige axiale Auslenkbarkeit des Trägerteils 170 gegenüber dem weiteren Trägerteil 178 erforderlich. Die Rückdrehungsverhinderungseinrichtung 226 könnte aber auch in anderer Weise verwirklicht sein.

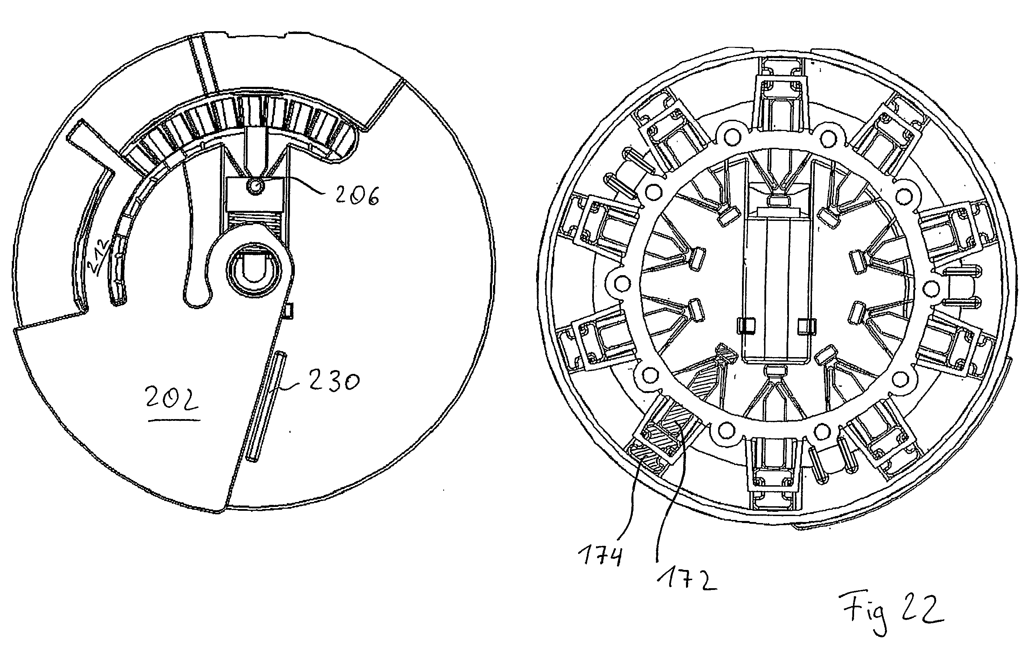

Die Figuren 22 bis 29 zeigen einen Betätigungszyklus der vorstehend beschriebenen Anordnung von Stechelementen. In der jeweiligen Figur links ist eine Ansicht der Stechelemente-Kassette von unten und auf der rechten Seite von oben dargestellt:
Figur 22 zeigt den Steuerhebel 202 im Ausgangszustand in Anlage gegen einen Endanschlag 230. In Figur 22 rechts ist ein Haltestößel 172 mit Stechelement und Schutzkappenmittel 174 hervorgehoben in einer Position vor der Arbeitsposition der Stechelemente dargestellt.

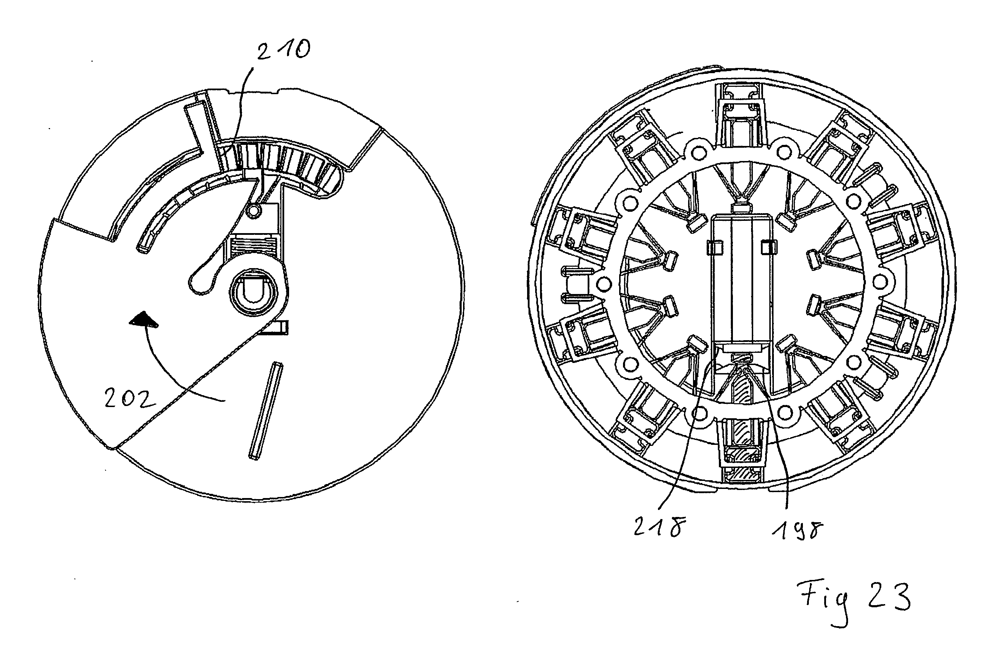

Figur 23 verdeutlicht eine Drehbewegung des Steuerhebels 202 in Uhrzeigersinnrichtung. Dabei greift das Mitnahmemittel 210 in die Stirnrastenanordnung 216 am Boden des Trägerteils 170 ein und transportiert bzw. dreht den Trägerteil 170 und die darauf angeordneten Stechelemente ebenfalls in Uhrzeigersinnrichtung, und zwar bis der vorstehend erwähnte Haltestößel 172 die Arbeitsposition, in Figur 23 rechts dargestellt, erreicht. Man erkennt in Figur 23 die vorstehend erörterten Anlauf- oder Einführschrägen 218 bei dem Hintergriffsmittel 198 des Stoßorgans.

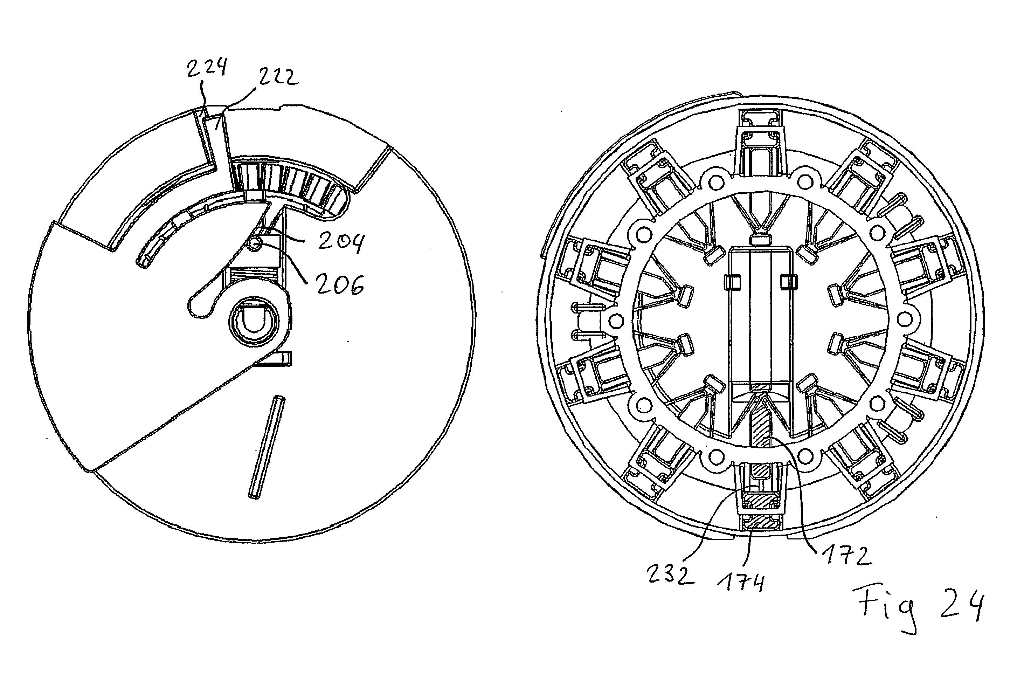

Beim Weiterdrehen des Steuerhebels 202 gemäß Figur 24 wird der radiale Fortsatz 222 und damit der Rasthebel 212 durch Aufgleiten gegenüber der Auflaufschräge 224 in axialer Richtung ausgelenkt, so dass das Trägerteil 170 nicht weitergedreht werden kann. Gleichzeitig drückt die Nockenführungsfläche 204 des Steuerhebels 202 die Nocke 206 und damit das Stoßorgan 184 in radialer Richtung nach innen, wobei die Stoßfeder 188 gespannt wird. Dabei wird durch Zusammenwirken des Hintergriffsmittels 198 und des hintergreifbaren Abschnitts 200 des Haltestößels 172 dieser nach radial innen mitgenommen, wodurch das erstmals in Figur 24 rechts ersichtliche Stechelement 232 aus dem Schutzkappenmittel 174 freikommt.

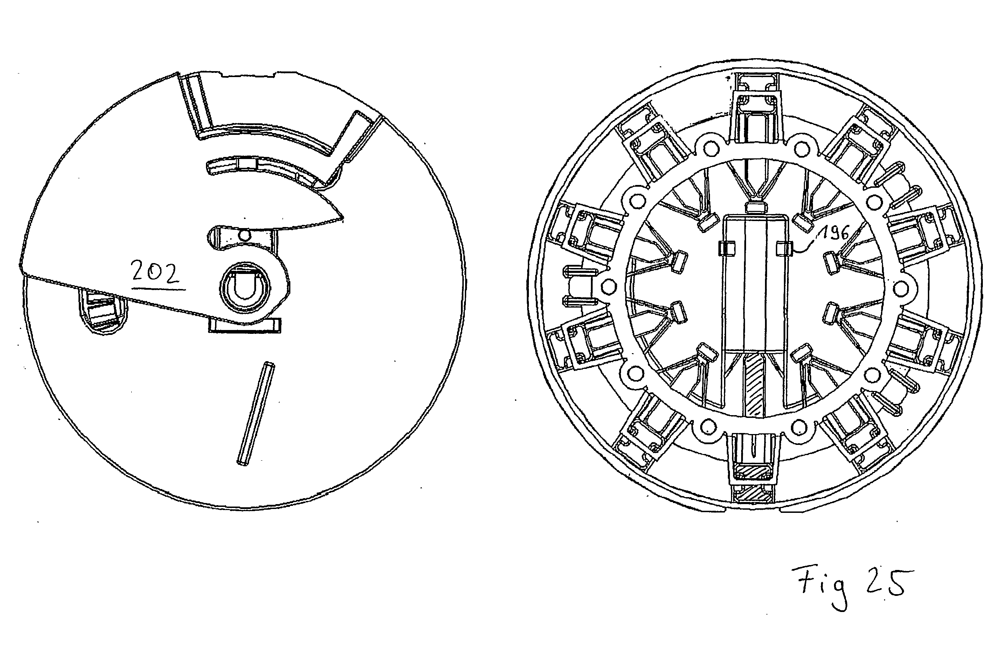

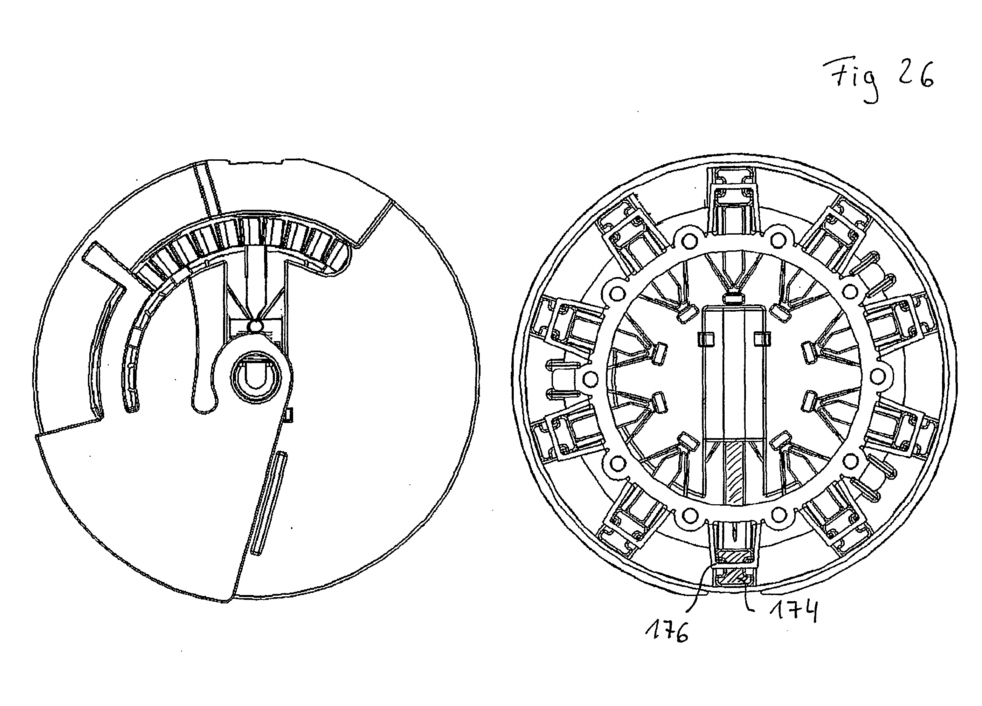

Figur 25 zeigt den maximal gedrehten Zustand des Steuerhebels 202 bzw. des Stoßorgans 184, in dem das Stoßorgan 184 in dieser gespannten Position über einen Haltemechanismus, der vorliegend durch die Klammerfeder 196 und die Ringnut am Stoßorgan 184 ausgebildet ist, arretiert wird. Beim Zurückdrehen des Steuerhebels 202 gemäß Figur 26 verbleibt das Stoßorgan 184 daher in seiner gespannten Position. Das Schutzkappenmittel 174 ist jetzt durch das axial wirkende Federmittel 176 aus dem Bewegungspfad des Stechelements 232 verbracht worden.

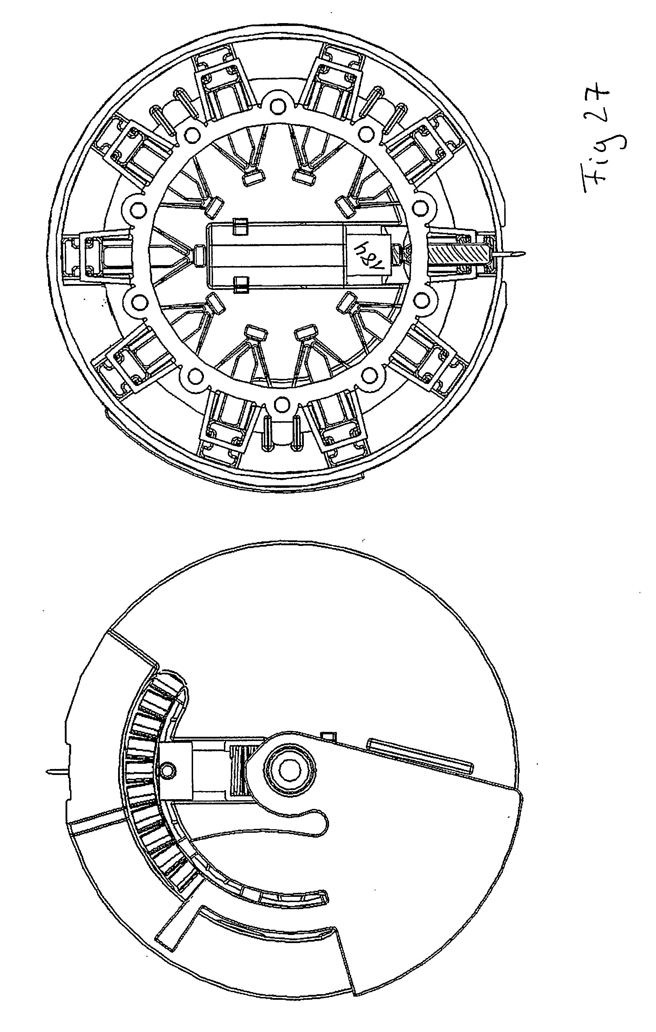

Schließlich zeigt Figur 27 den ausgeführten Stechvorgang, wobei durch Betätigen des Haltemittels in Form einer Spreizung der Klammerfeder 196 das Stoßorgan 184 in radialer Richtung nach außen schnellt und dabei auf das Ende des Haltestößels 172 trifft und diesem zusammen mit dem Stechelement 232 nach außen stößt.

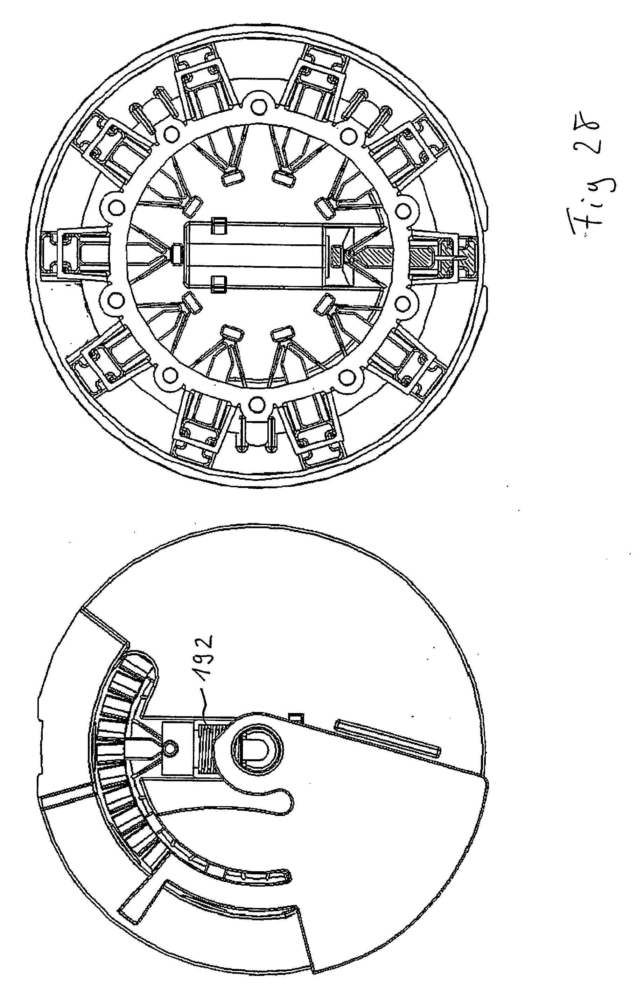

Im nächsten Moment bewirkt die Rückstellfeder 192 eine Rückzugsbewegung des Stoßorgans 184 in die in Figur 28 dargestellte Ausgangsposition, in der das Stechelement in der Arbeitsposition in das Gehäuse zurückgezogen ist.

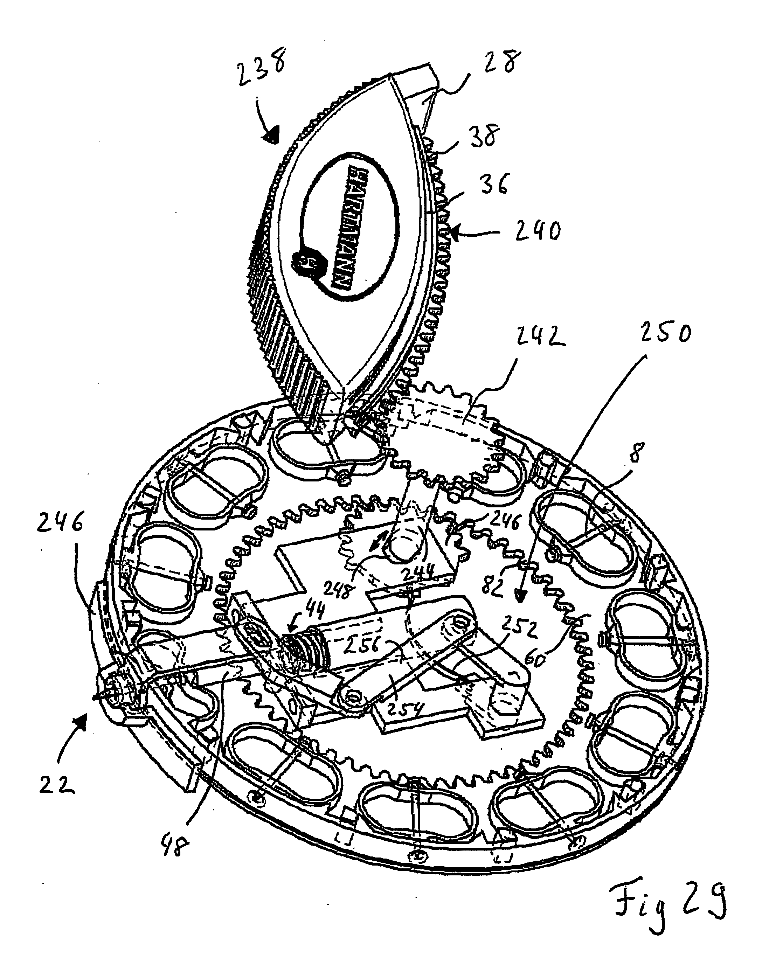

Die Figuren 29 bis 32 verdeutlichen eine bevorzugte Ausführungsform der Antriebsvorrichtung, d. h. des Stoßmechanismus, für ein in seiner Arbeitsposition befindliches Stechelement. Des weiteren veranschaulichen diese Figuren (auch unabhängig von der Anordnung und Ausbildung der Stechelemente auf einem drehbaren Träger) das Aktivieren, also Spannen, der Antriebseinrichtung für die Stechelemente und das Weiterdrehen des Trägers mittels eines einzigen Steuerelements 238, welches in beispielhafter Weise von dem bereits früher erörterten Abdeckteil gebildet ist. Hierzu im Einzelnen:
Figur 29 zeigt eine perspektivische Ansicht der Trägerkomponenten dieser Ausführungsform des Analysegeräts, wobei gehäusebildende Komponenten weggelassen sind. Man erkennt jedoch das Abdeckteil 28, welches an seiner Innenseite 36 neben der in Verbindung mit Figur 2 erläuterten Gleitführungsschiene 38 eine der Innenseite 36 folgende zahnstangenartige Anordnung von Zähnen 240 aufweist. Diese Zähne 240 sind in Eingriff bringbar mit der Zahnung eines in der Bewegungsebene des Abdeckteils 28 drehbaren ersten Zahnrads 242. Dieses erste Zahnrad 242 ist drehfest auf einer senkrecht zur genannten Ebene erstreckten Welle 244 angeordnet, die an ihrem anderen Ende ein abtriebsseitiges zweites Zahnrad 246 aufweist. Dieses zweite Zahnrad 246 ist in Eingriff und außer Eingriff bringbar mit einer Innenverzahnung 82 eines Trägerteils 60 (es wurden hier entsprechende Bezugszeichen wie bei Figuren 1 bis 6 verwendet). Die Welle 244 ist in einem in der genannten Bewegungsebene ersteckten Langloch 248 bewegbar. Bei Verschwenken des Abdeckelements 28 in Richtung einer Freigabe der im Zusammenhang mit Figur 1 erörterten Aufgabeposition wird die Welle 244 in die in Figur 29 dargestellte Position an einem Ende das Langlochs 248 gezwungen, in der das zweite Zahnrad 246 mit der Innenverzahnung 82 des Trägerteils 60 kämmt, so dass bei Umdrehung der Zahnrad- und Wellenanordnung das Trägerteil 60 und damit die Anordnung von Stechelementen in Uhrzeigersinnrichtung weitergedreht wird. Wenn das Abdeckteil 28 in seine Ausgangsposition zurückgeschoben oder zurückgeschwenkt wird, so wird die Zahnrad- und Wellenanordnung gegen das gegenüberliegende Ende des Langlochs 48 gezwungen, so dass die Zähne des zweiten Zahnrads 246 außer Eingriff mit der Innenverzahnung 82 des Trägerteils 60 gelangen. Auf diese Weise wird eine Rückdrehung der Anordnung von Stechelementen 8 verhindert.

Die Antriebseinrichtung für ein in der Arbeitsposition befindliches Stechelement sei insgesamt mit dem Bezugszeichen 250 bezeichnet und umfasst einen Stoßmechanismus 44 mit einem auf ein jeweiliges Stechelement einwirkenden Stößel 48. Die Antriebseinrichtung 250 umfasst aber auch einen Spannmechanismus, welcher vorliegend von dem zweiten Zahnrad 246 und einer Biegefeder 252 gebildet ist, und eine Auslöseeinrichtung 254, die eine Hebelanordnung 256 umfasst.

Die Biegefeder 252 ist mit ihren Enden einerseits an einer Biegefederaufnahme 258 am zweiten Zahnrad 246 und anderenends an einer Biegefederaufnahme 260 an einem gegenüber dem Gehäusekörper 4 schwenkbaren Bauteil 262 befestigt. Dieses schwenkbare Bauteil 262 ist Teil der Hebelanordnung 256, welche dieses Bauteil 262 mit einem Taster 264 in der Stechposition 22 am Gehäusekörper 4 verbindet.

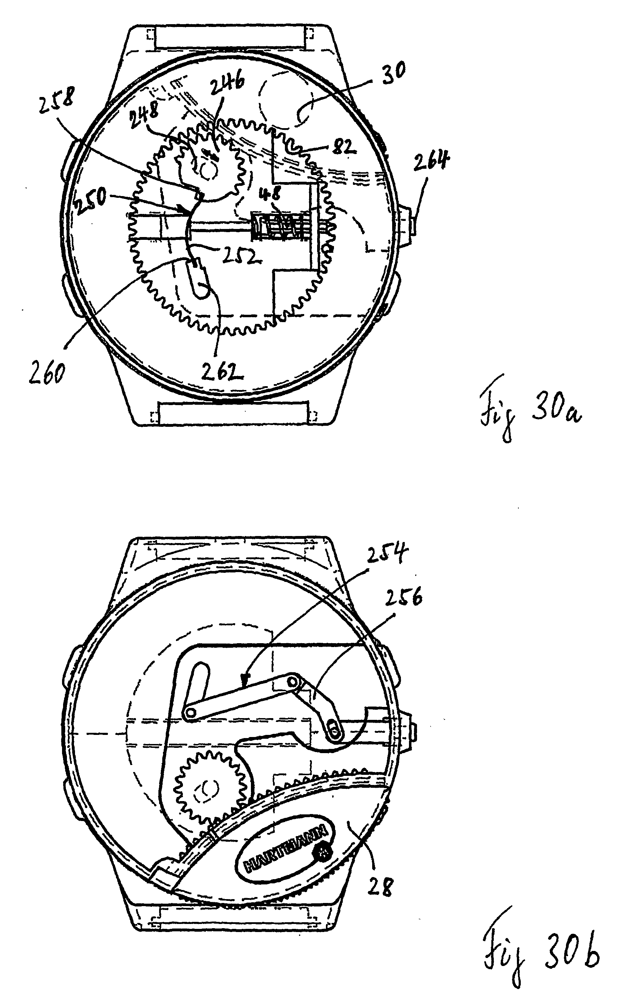

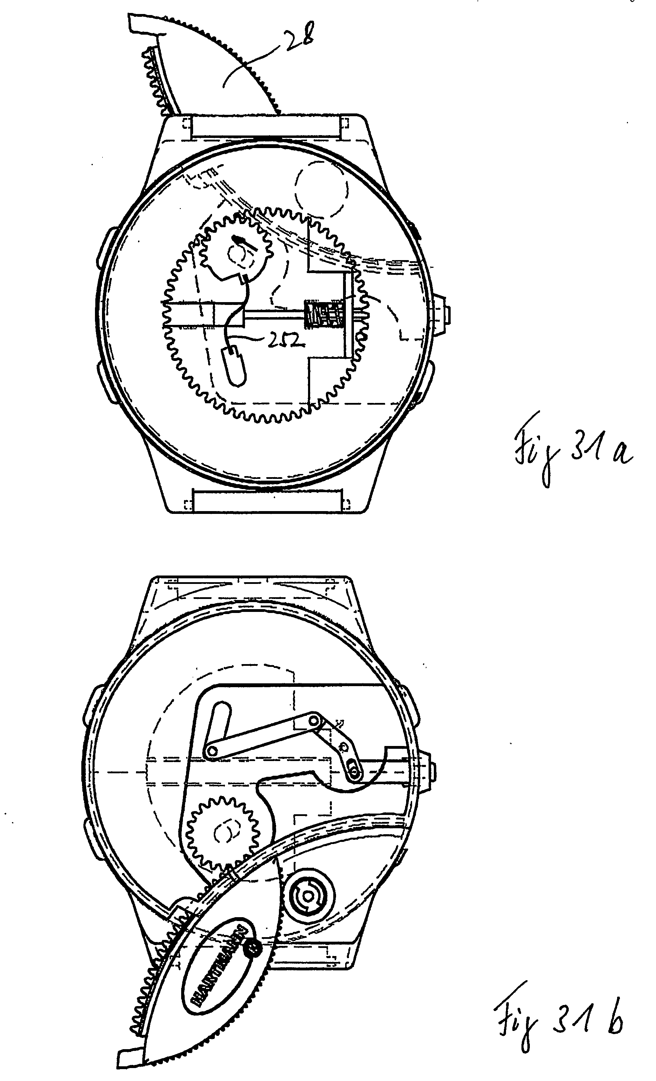

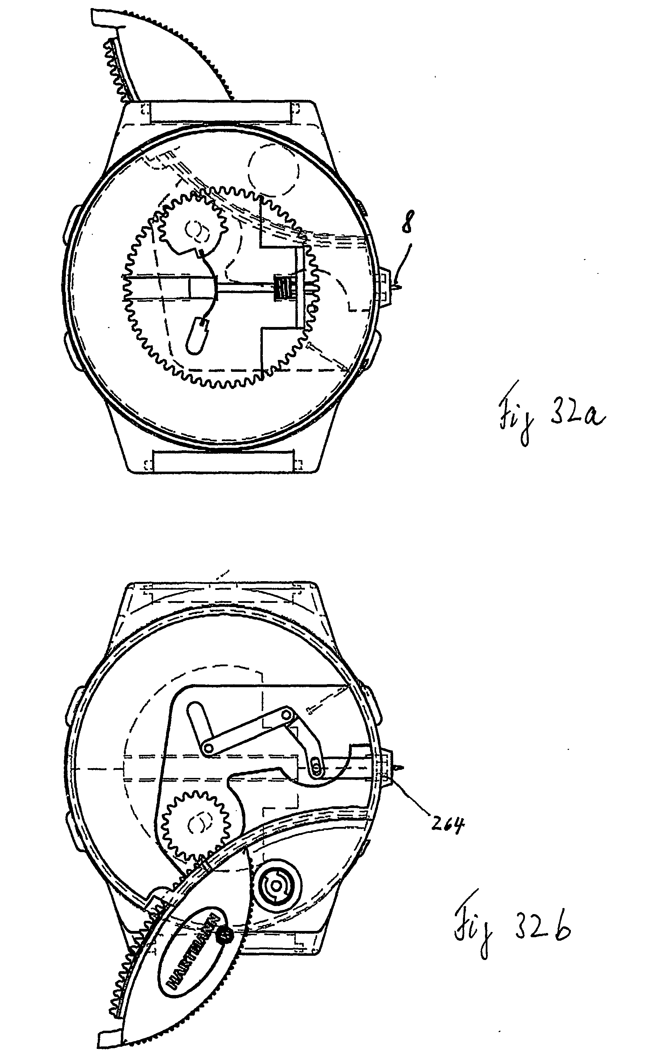

Bei Nachauswärtsbewegen des Abdeckteils 28 dreht das zweite Zahnrad 246 nicht nur den Trägerteil 60 weiter, sondern es verschwenkt die Biegefederaufnahme 258 und versetzt dadurch die Biegefeder 252 in einen gespannten Zustand. Durch Betätigen des Tasters 264 wird die andere Biegefederaufnahme 260 durch die Hebelanordnung 56 ebenfalls verschwenkt, so dass die Biegefeder 252 von ihrem stabilen gespannten Zustand über einen Totpunkt sich schlagartig entspannt und dabei den Stößel 48 nach radial außen stößt, welcher seinerseits das betreffende Stechelement zur Ausführung des Stechvorgangs ebenfalls nach radial außen schnellen lässt. Dieser Bewegungsablauf ist in den Figuren 30, 31 und 32 dargestellt. In diesen Figuren ist jeweils eine Ansicht von oben und unten der hier interessierenden Komponenten innerhalb des Gehäusekörpers dargestellt. Figur 30 zeigt die Antriebseinrichtung 250 der Blutentnahmeeinrichtung in einem nicht aktivierten Ausgangszustand. Die Biegefeder 252 nimmt zwischen den Biegefederaufnahmen 258 und 260 eine bogenförmige Gestalt ein. Wenn nun wie in Figur 31 ersichtlich das Abdeckteil 28 in Richtung einer Freigabe der Aufgabeposition 30 verschwenkt wird, so wird über das Zahnrad 242, die Welle 244 und das Zahnrad 248 und die Innenverzahnung 82 der Trägerteil 30 mitgedreht. Gleichzeitig bewegt sich in der Darstellung der Figur 31a die Biegefederaufnahme 258 am zweiten Zahnrad 246 entgegen der Uhrzeigersinnrichtung, und die Biegefeder 252 nimmt eine S-förmig gebogene Gestalt an. Währenddessen verbleibt die Biegefederaufnahme 260 und die Stellung des schwenkbaren Bauteils 262 unverändert. Die Antriebseinrichtung 250 und deren Biegefeder 252 sind nun im gespannten aktivierten Zustand. Wenn nun zur Auslösung des Stechvorgangs ein Benutzer die Auslöseeinrichtung 254 durch Drücken des Tasters 264 betätigt, so wird über die Hebelanordnung 256 das schwenkbare Bauteil 262 in die in Figur 32a dargestellte Position verschwenkt. Hierdurch wird die Biegefeder 252 über eine Totpunktlage bewegt und die in der S-förmig gespannten Feder gespeicherte Federenergie entlädt sich schlagartig, indem die Feder die aus Figur 32a ersichtliche, wiederum bogenförmige Gestalt, jedoch mit entgegengesetzter Krümmung als in Figur 29a dargestellt, einnimmt. Durch Kopplung der Biegefeder 252 mit dem Stößel 48 des Stoßmechanismus 244 wird dieser ebenfalls schlagartig nach außen gestoßen.

Wenn eingangs von Minimalmengen von Blut zum Aufbringen auf ein Testmittel des Blutanalysegeräts die Rede war, so werden hierbei Blutmengen von < 20 µl, insbesondere < 10 µl und vorzugsweise < 5 µl verstanden.

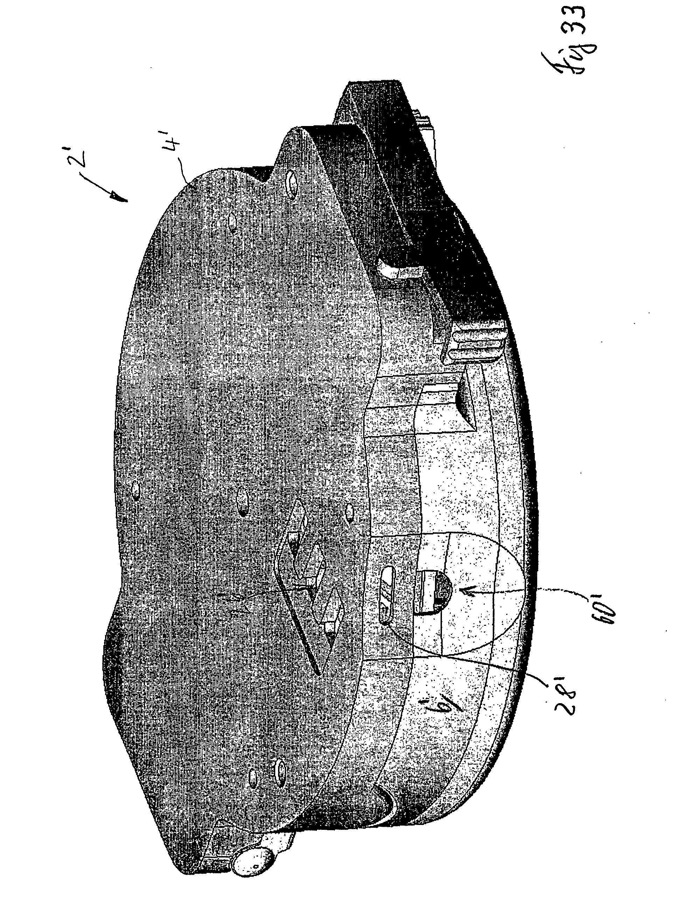

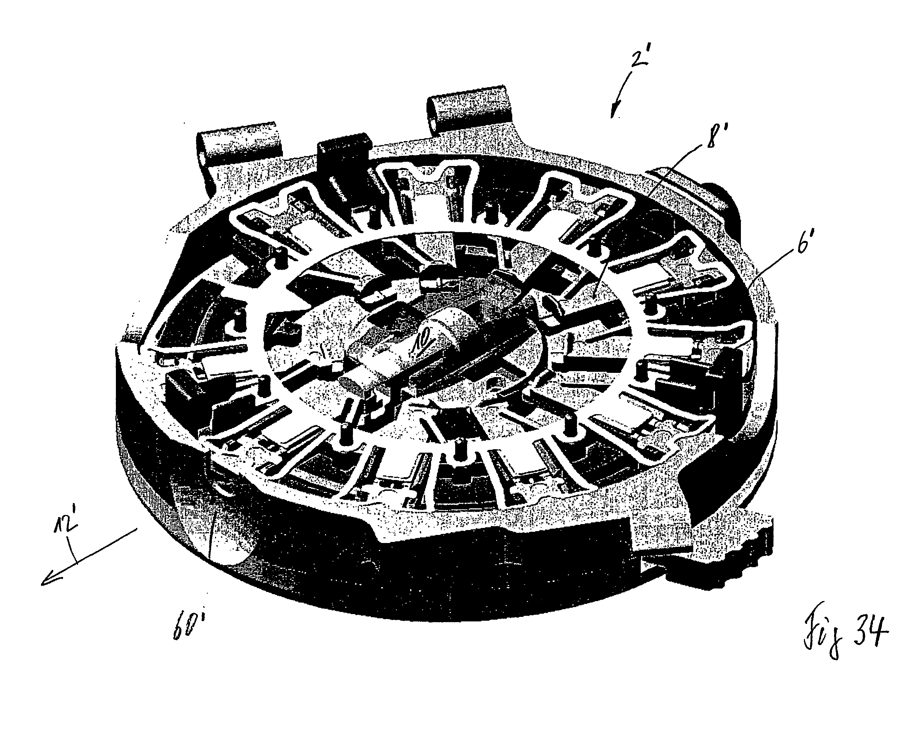

Die Figuren 33 und 34 zeigen perspektivische Ansichten einer insgesamt mit dem Bezugszeichen 2' bezeichneten Stechvorrichtung für die Entnahme einer Minimalmenge von Blut am menschlichen oder tierischen Körper zu Analysezwecken. Bei Figur 34 ist ein in Figur 33 dargestelltes schwenkbar angelenktes Deckelteil 4' weggelassen. Man erkennt im Inneren eines Gehäusekörpers 6' eine Mehrzahl von konzentrisch und radial angeordneten Stechelementen 8', die nachfolgend noch näher beschrieben werden. Im Zentrum der konzentrischen Anordnung ist eine Stoßvorrichtung 10' ersichtlich, die eine Stoß- oder Stechrichtung 12' definiert.

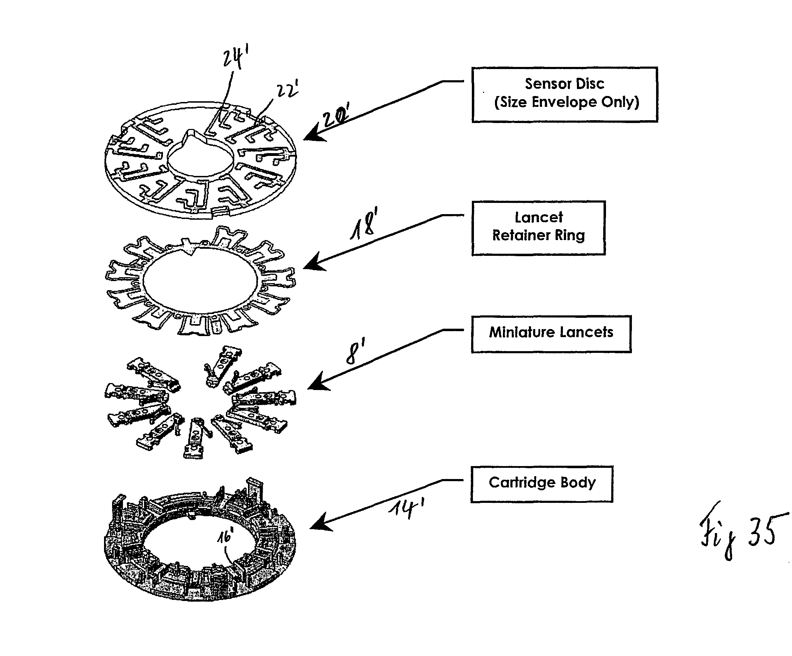

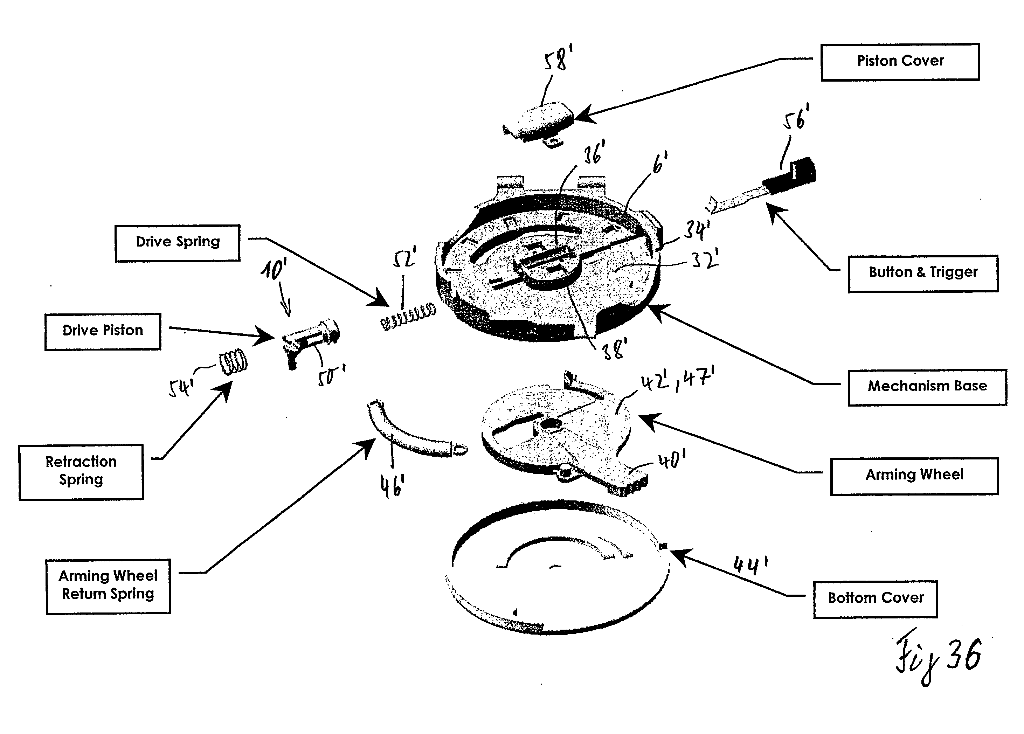

Die Figuren 35 und 36 zeigen in explosionsartiger Darstellung die in den Gehäusekörper 6' einsetzbaren Komponenten. Man erkennt, dass die nachfolgend noch näher zu beschreibenden und mit einer Schutzumhüllung versehenen Stechelemente 8', deren radiale Anordnung in Figur 35 angedeutet ist, auf einem ringscheibenförmigen Träger 14' in dafür vorgesehenen Führungsbahnen oder Führungsausnehmungen 16' des Trägers 14' in radialer Richtung gleitverschieblich sind.

Oberhalb der Stechelemente 8' ist in Figur 35 eine Ringscheibe 18' aus Federstahl dargestellt, welche auf noch näher zu beschreibende Weise die Stechelemente 8' unverlierbar in den Führungsausnehmungen 16' des Trägers 8', jedoch radial verschieblich hält.

Oberhalb der Ringscheibe 18' ist ein zweiter Träger 20' dargestellt mit schematisch angedeuteter Kontaktierung 22' für im Bereich der Kontaktierung 22' vorgesehene Testmittel 24' zur Durchführung der Blutanalyse, also zur Bestimmung der Anwesenheit und des Gehalts eines Analyten, beispielsweise Blutzucker, Lactat, Cholesterol oder Fruktosamin. Es wäre denkbar, dass diese im Einzelnen nicht dargestellten, insbesondere membranartigen Testmittel 24' durch eine Aufgabeöffnung im Deckelteil 4' der Stechvorrichtung mit der erforderlichen Minimalmenge von Blut beaufschlagbar sind. Es wäre aber auch denkbar, dass ein nicht dargestellter Analyseteststreifen durch eine schlitzförmige Öffnung 28' ausgegeben und mit der Minimalmenge von Blut benetzt wird. Über die Kontaktierung 22' und eine nicht dargestellte Auswerteeinrichtung kann dann eine Analyse amperometrisch oder potentiometrisch durchgeführt werden. Auch denkbar wäre die Mitführung separater Analyseteststreifen, die dann durch die schlitzförmige Öffnung 28' zu der Kontaktierung 22' auf dem Träger 20' eingesteckt werden, die also nicht im Inneren des Gehäusekörpers 6' auf dem Träger 20' mitgeführt werden. Bei einer bevorzugten Ausführungsform trägt jedoch der zweite Träger 20' eine den Stechelementen 8' entsprechende Anzahl von Testmitteln 24'.

Auf der in Figur 33 nach oben gewandten Sichtseite des Deckelteils 4' ist eine ein Display aufweisende Anzeigeeinrichtung beispielsweise in Kombination mit den üblichen Komponenten einer Armbanduhr befestigbar.

Figur 36 zeigt den Gehäusekörper 6', der eine Bodenplatte 32' mit einem zylindrisch nach oben kragenden Randabschnitt 34' sowie einer in der Mitte angeordneten domförmigen Erhebung 36' mit abschnittsweise kreisförmigem Umfang 38' aufweist. An der Unterseite der Bodenplatte 32' ist ein mit einem Betätigungshebel 40', der radial nach außen vorsteht, versehenes scheibenförmiges Bauteil 42' drehbar angeordnet. Es wird durch ein bodenseitiges Deckelteil 44' umdrehbar an der Unterseite der Bodenplatte 32' des Gehäusekörpers 6' gehalten. Ferner angedeutet ist ein Retraktionsmittel in Form einer Rückzugsfeder 46'. Das scheibenförmige Bauteil 42' bildet ein Spannmittel 48' für die Stoßvorrichtung 10'.

In der Mitte der domförmigen Erhebung 36' ist die insgesamt mit dem Bezugszeichen 10' bezeichnete Stoßvorrichtung untergebracht. Sie umfasst ein noch näher zu beschreibendes Stoßorgan 50', eine Stoßfeder 52' und eine Rückstellfeder 54', ein Auslösemittel 56' und eine Abdeckung 58'. Die Stoßvorrichtung 10' ist durch Verschwenken des Betätigungshebels 40' und damit des scheibenförmigen Bauteils 42' aktivierbar, indem das Stoßorgan 50' gegen den Druck der Stoßfeder 52' gespannt wird. Durch Betätigen des Auslösemittels 56' schnellt das Stoßorgan 50' in radialer Richtung und führt zusammen mit einem Stechelement 8' einen Stechvorgang aus, dabei schnellt das Stechelement 8' über die in den Figuren 33 und 34 dargestellte Anlageposition 60' für einen Finger eines Benutzers kurzzeitig hervor und durchsticht die Hautoberfläche, damit ein Benutzer unmittelbar im Anschluss daran eine Minimalmenge von Blut aus der Fingerkuppe hervordrücken kann.

Nachfolgend werden die vorstehend kursorisch erwähnten Komponenten anhand von Einzeldarstellungen detailliert in Aufbau und Funktion beschrieben:

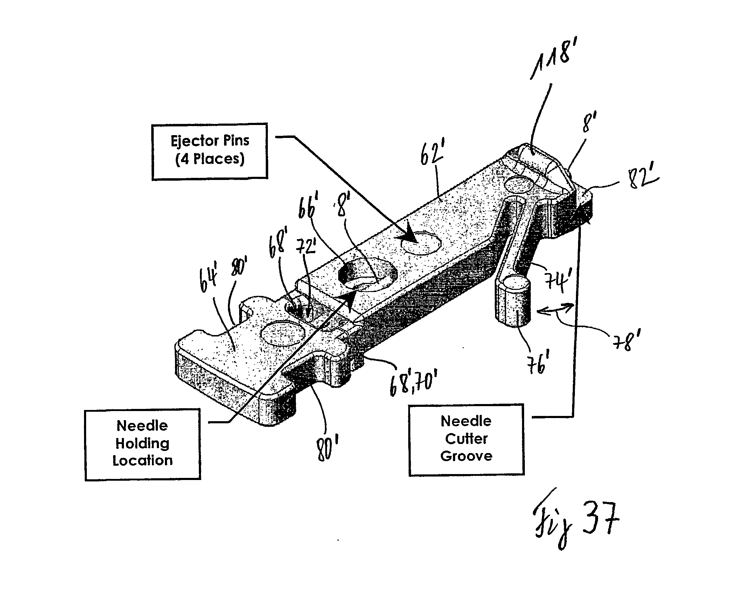

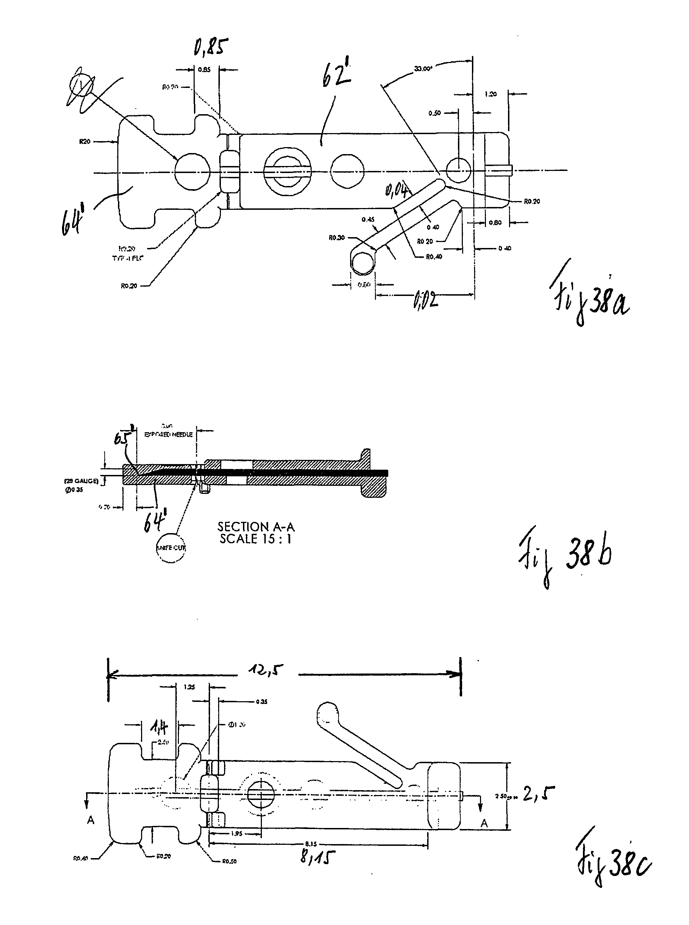

Figur 37 zeigt in perspektivischer Darstellung stark vergrößert ein Stechelement 8' mit der auch aus den Figuren 34 und 35 ersichtlichen Umhüllung. Bei dieser Umhüllung handelt es sich um einen Haltekörper 62' aus Kunststoff, welcher an das eigentliche nadelförmige Stechelement 8' (häufig auch als Lanzette bezeichnet) angespritzt ist, und um ein Schutzkappenmittel 64' im Bereich des angeschliffenen spitzen Endes 65' (s. Figur 38b). Beim Einspritzvorgang wird das Stechelement 8' durch die freibleibende Öffnung 66' in dem Haltekörper 62' hindurch in der Spritzgießform gehalten. Es wird in einem Vorgang der Haltekörper 62' und das Schutzkappenmittel 64' angespritzt. Haltekörper 62' und Schutzkappenmittel 64' gehen dabei über einen dünnwandigen Übergangsbereich 68', der einen Schwächungsbereich 70' bildet, ineinander über. Es wird aber ausdrücklich darauf hingewiesen, dass mittels Schiebern oder durch aufeinander folgende Fertigung von Haltekörper 62' und Schutzkappenmittel 64' auch eine nicht einstückige Ausbildung dieser Komponenten um das Stechelement 8' herum denkbar wäre. Man erkennt des weiteren eine Freischneidung 72' im Übergangsbereich 68', welcher die Ausbildung des dünnwandigen Schwächungsbereichs 70' begünstigt. Diese Freischneidung 72' kann beispielsweise durch ein weiteres Haltemittel für das Stechelement 8' beim Einspritzvorgang gebildet werden.

Man erkennt ferner einen schräg in einem Winkel von etwa 40° zur Längsrichtung des Stechelements 8' von dem Haltekörper 62' abstehenden und einstückig mit diesem ausgebildeten Steg 74', der ein verrundetes freies Ende 76' aufweist. Der Steg 74' ist bezüglich des quaderförmigen Haltekörpers 62' für das Stechelement 8' in Richtung des Doppelpfeils 78' abspreitzbar oder elastisch nachgiebig verformbar. Er sichert einerseits eine Stabilisierung in der Auflageebene für den Haltekörper 62' und verhindert beispielsweise ein Verkippen um die Längsrichtung. Nach Ausführung eines Stoßvorgangs vermag er aber auch ein Rückzugsmoment in den Haltekörper und damit in das Stechelement 8' einzuleiten und dieses wieder zurückzuziehen. Er sichert das Stechelement 8' auch gegen Herausgleiten aus den Führungsausnehmungen 16' des Trägers 14'.

Das Schutzkappenmittel 64' weist in der Draufsicht eine H-förmige Gestalt auf. Es weist beidseits Führungsausnehmungen 80' auf, mit denen es einerseits in Längsrichtung des Stechelements 8', also in radialer Richtung, unverschieblich am Träger 14' gehalten ist, andererseits aber quer, und zwar senkrecht zur Längsrichtung der Stechelemente 8' bezüglich des Trägers gleiten kann.

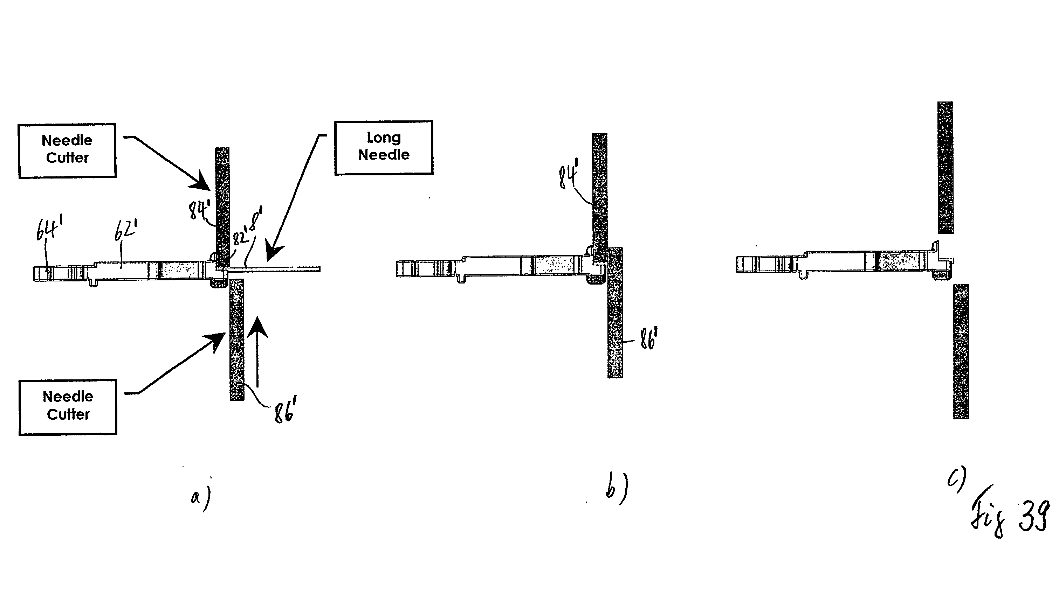

Die Figuren 38a, b und c verdeutlichen die Dimensionierung des weitgehend miniaturisierten Stechelements mit Haltekörper und Schutzkappenmittel. Es umfasst in seiner Längsrichtung einschließlich Haltekörper 62' und Schutzkappenmittel 64' eine Länge von nur 12,5 mm. Figuren 39a, b und c verdeutlichen den Herstellungs- bzw. Vereinzelungsvorgang bei der Herstellung der mit Haltekörper 62' und Schutzkappenmittel 64' umgebenen Stechelemente 8'. Man erkennt auch schon aus Figur 37, dass das vom Schutzkappenmittel 64' abgewandte Ende des Haltekörpers 62' abgesetzt ausgebildet ist und eine das Stechelement 8' freilegende Stufe 82' aufweist. Mittels angedeuteter Ober- und Untermesser 84', 86' wird von einem endlosen Draht oder einer endlosen Röhre das Stechelement 8' abgelängt, indem das Obermesser 84' nahezu in Anlage an die Stufe 82' gebracht wird und dadurch bezüglich des Haltekörpers 62' und des Stechelements 8' definiert positionierbar ist. Dies ist in den Figuren 39a bis 39c dargestellt.

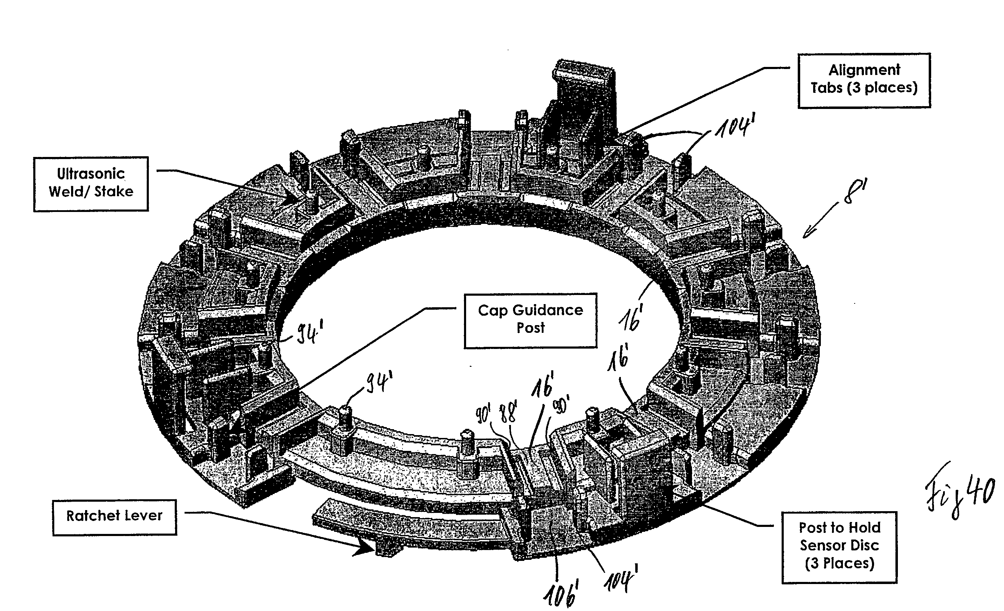

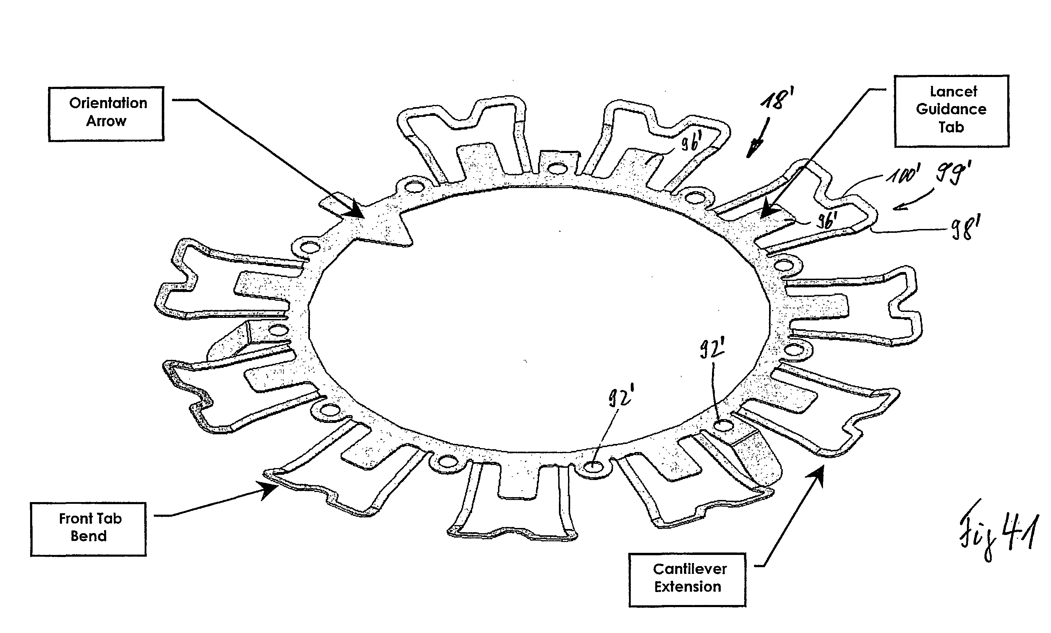

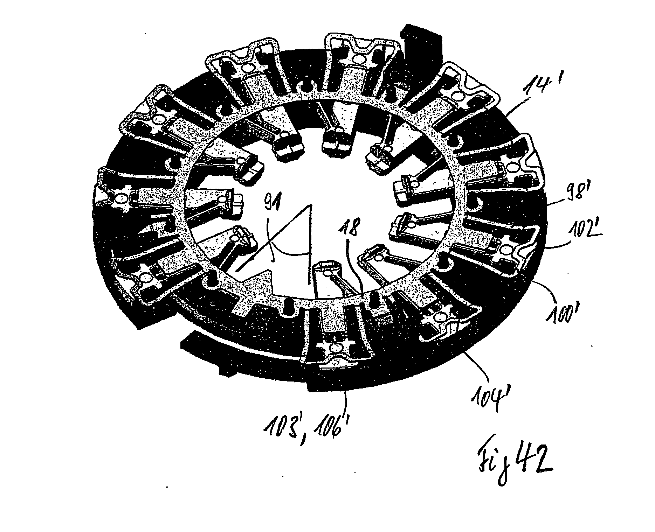

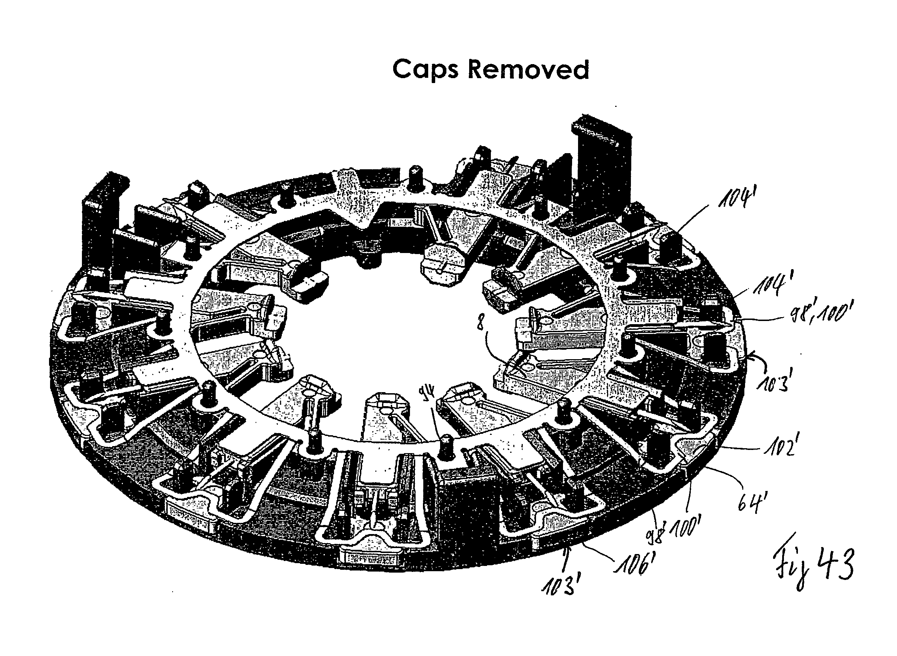

Die Figuren 40 bis 43 zeigen die Anordnung der Stechelemente 8' nebst Haltekörper 62' und Schutzkappenmittel 64' an dem Träger 14'. Der Träger 14' ist ringscheibenförmig ausgebildet und auf seiner dargestellten Sichtseite aufwendig konturiert. Er umfasst im dargestellten Fall zehn radiale Führungsausnehmungen 16', die von einer in der Scheibenebene liegenden Stützwandung 88' und zwei senkrecht hierzu und radial ausgerichteten seitlichen Führungswandungen 90' begrenzt sind. Darin ist jeweils ein Stechelement 8' mit Haltekörper 62' und Schutzkappenmittel 64' radial gleitverschieblich. Die jeweiligen Stechelemente 8' sind von oben, also in axialer Richtung in die Führungsausnehmungen 16' eingesetzt und nehmen dann die in Figur 34 und Figur 42 gezeigten Positionen ein. Man erkennt, dass in einem Kreissegment 91' des Trägers 14' kein Stechelement 8' angeordnet ist. Wenn der Träger von oben in den Gehäusekörper 6' eingesetzt wird, so ist der Träger 14' so zu positionieren, dass das Kreissegment 91' oberhalb der Stoßvorrichtung 10' orientiert ist, so dass sich die Stoßvorrichtung 10' mit ihrem radial äußeren Ende in dieses Kreissegment 91' hineinerstreckt. Das Stoßorgan 50' ist dann innerhalb des Kreissegments 91' quasi zwischen zwei benachbarten Stechelementen, nämlich dem ersten und dem letzten Stechelement, angeordnet. Man erkennt auch ein Positioniermittel oder eine Positionierhilfe in Form einer pfeilförmigen Konturierung der Ringscheibe 18'. Die Stechelemente 8' sind mittels der Ringscheibe 18' aus Federstahl in ihren Positionen innerhalb der Führungsausnehmungen 16' des Trägers 14' gehalten. Hierfür ist die Ringscheibe 18' über eine Anzahl von Öffnungen 92' auf korrespondierende Stifte 94' des Trägers 14' gesteckt und diese sind dann nietenartig, insbesondere durch Ultraschallschweißen, aufgeweitet. Die Ringscheibe 18' aus Federstahl weist radial vorstehende Zungen 96' auf, welche parallel zu der Stützwandung 88' orientiert sind und einen jeweiligen Gehäusekörper 62' eines jeweiligen Stechelements 8' halten und führen. Um jede Zunge 96' herum erstreckt sich eine weitere bügelförmige Zunge 98', die U-förmig ausgebildet ist und sich mit den beiden Schenkeln der U-form an die Ringscheibe 18' anschließt. Die bügelförmige Zunge bildet ein Verdrängungsmittel 99' für die Schutzkappenmittel 64', um diese aus dem Bewegungspfad der Stechelemente 8' zu verdrängen. Die in Umfangsrichtung verlaufenden Verbindungsstege 100' der Zunge 98' sind zur Ebene der Ringscheibe 18' leicht nach oben angewinkelt und mäandrierend ausgebildet. Sie definieren daher jeweils eine Anlageebene, die geringfügig schräg zur Ebene der Ringscheibe 18' verläuft. Dies hat folgenden Grund: Man erkennt aus Figur 10, dass die Verbindungsstege 100' und die jeweils durch sie gebildete Ebene ebenfalls schräg zur Oberseite 102' der jeweiligen Schutzkappenmittel 64' angeordnet ist. Gleichwohl ruht der jeweilige Verbindungssteg 100' unter leichter Vorspannung auf der Oberseite 102' des betreffenden Schutzkappenmittels 64'. Wenn auf noch näher zu beschreibende Weise ein Haltekörper 62' mit dem eingespritzten Stechelement 8' nach radial innen gezogen und der Schwächungsbereich 70' zwischen Haltekörper 62' und Schutzkappenmittel 64' gebrochen wird, so drückt die bügelförmige Zunge 98' mit ihrem Verbindungssteg 100' das betreffende Schutzkappenmittel 64' quer zur Stechrichtung oder Längsrichtung der Stechelemente 8' nach unten in die in Figur 11 dargestellte Position. In dieser Entsorgungsposition 103' ruht der Verbindungssteg 100' der federnden Zunge 98' flächenhaft auf der Oberseite 102' des jeweiligen Schutzkappenmittels 64' (Figur 43 zeigt die Stechelemente 8' nach Ausführung des Stechvorgangs).

Des weiteren erkennt man aus den Figuren 40, 42 und 43 leistenförmige in axialer Richtung orientierte Führungspfähle 104', welche in die Führungsausnehmungen 80' des jeweiligen Schutzkappenmittels 64' eingreifen. Das jeweilige Schutzkappenmittel 64' ist in axialer Richtung, also senkrecht zur radial orientierten Stechrichtung auf diesen Führungspfählen 104' verschieblich. Zugleich halten diese Führungspfähle 104' ein jeweiliges Schutzkappenmittel 64' in radialer Richtung unverschieblich, so dass bei Nachinnenziehen des Haltekörpers 62' der Schwächungsbereich 70' gebrochen werden kann. In der Folge wird dann - wie vorausgehend erläutert - ein jeweiliges Schutzkappenmittel 64' in eine Aufnahmekavität 106' im Träger 14' aufgenommen, welche die Entsorgungsposition 103' für das Schutzkappenmittel bildet. In dieser Aufnahmekavität 106' ist ein jeweiliges Schutzkappenmittel dann unter Belastung durch die bügelförmige federnde Zunge 98' spielfrei gehalten. Ein störendes Klappergeräusch kann dabei nicht auftreten.

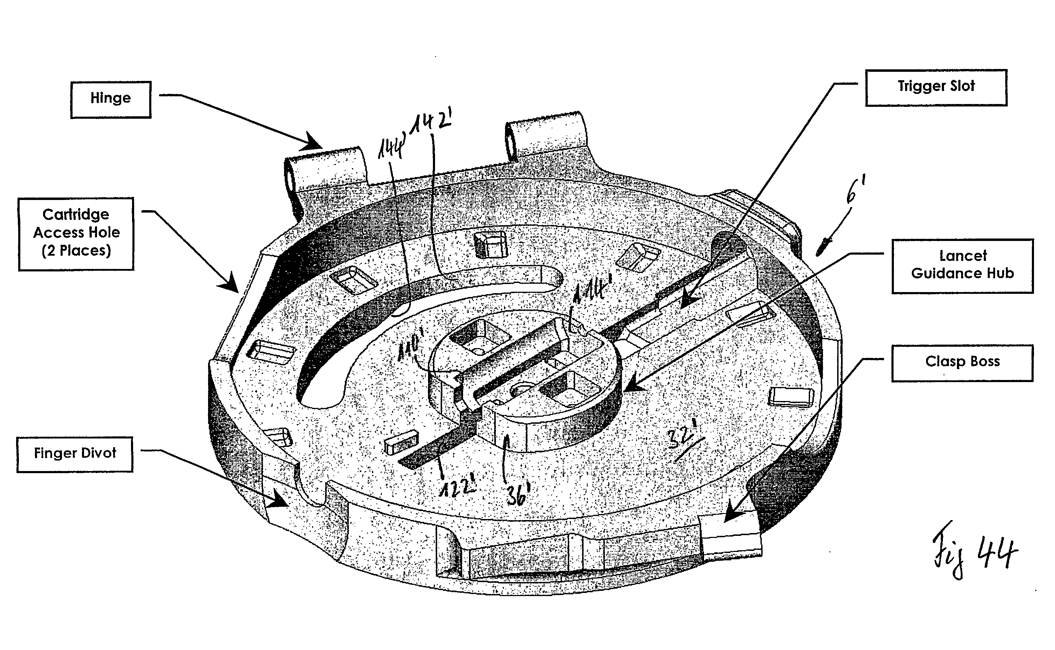

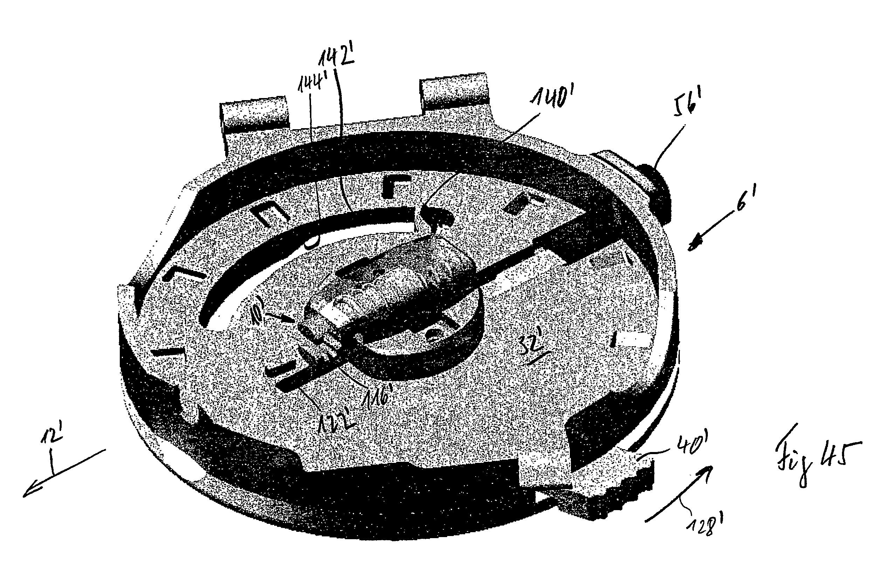

Die Figuren 44 und 45 zeigen den Gehäusekörper 6' mit und ohne die betreffenden Komponenten der Stoßvorrichtung 10'. Man erkennt, dass die domförmige Erhebung 36' in dem Gehäusekörper 6' eine Aufnahme für das Stoßorgan 50', die Stoßfeder 52' und die Rückstellfeder 54' bildet. Durch die Abdeckung 58' sind diese Komponenten verliersicher und in Stechrichtung 12', also in radialer Richtung längsverschieblich in der Aufnahme gehalten. Figur 14 zeigt in stark vergrößerter Darstellung das Stoßorgan 50', welches in Form eines Kolbenmittels oder Stösselmittels ausgebildet ist, das einen abgesetzten Außendurchmesser aufweist und so eine axiale Stufe 108' aufweist, gegen die sich die Rückstellfeder 54' einenends abstützt. Anderenends ist die Rückstellfeder 54' gegen eine Wange 110' der domförmigen Erhebung 36' abgestützt. Am durchmessergrößeren Ende 112' stützt sich die Stoßfeder 52' ab, wobei sie anderenends gegen eine Wange 112' der domförmigen Erhebung 36' abgestützt ist. Das Stoßorgan 50' umfasst des weiteren einen Kopplungsbereich 116', der in Stoßrichtung, aber auch in Umfangsrichtung geöffnet ist. Dieser Kopplungsbereich 116' ist komplementär zu einem Hintergriffsbereich 118' (s. Figur 37) des Haltekörpers 62' ausgebildet und vermag diesen Bereich aufzunehmen, so dass der Haltekörper 62' mit dem Stoßorgan 50' koppelbar ist und eine formschlüssige Mitnahmeverbindung mit dem Stoßorgan 50' ausbildet. Bei der aus Figur 45 ersichtlichen Orientierung des Stoßorgans 50' kann durch Verdrehen des Trägers 14' ein Haltekörper 62' eines Stechelements 8' in diesen Kopplungsbereich 116' hineingedreht werden, so wie dies auch aus Figur 2 ersichtlich ist. Wird das Stoßorgan 50', wenn es in Mitnahmeverbindung mit einem Haltekörper 62' eines Stechelements 8' steht, nach radial innen zurückgezogen, wobei die Stoßfeder 52' gespannt wird, so kann das betreffende Schutzkappenmittel 64', das in radialer Richtung formschlüssig gehalten ist, nicht folgen und der zwischen ihnen befindliche Schwächungsbereich 70' bricht. Sobald das freie spitze Ende 65' des Stechelements 8' aus dem Schutzkappenmittel 64' freikommt, wird dieses - wie vorstehend beschrieben - unter der Wirkung der bügelförmigen federnden Zunge 98' in die Entsorgungsposition 103' verbracht. Das Stechelement 8' zusammen mit dem Haltekörper 62' folgt währenddessen der Spannbewegung des Stoßorgans 50'. Die Stoßvorrichtung 10' befindet sich nun im aktivierten Zustand und kann durch Drücken des Auslösemittels 56' zur Ausführung des Stechvorgangs betätigt werden.

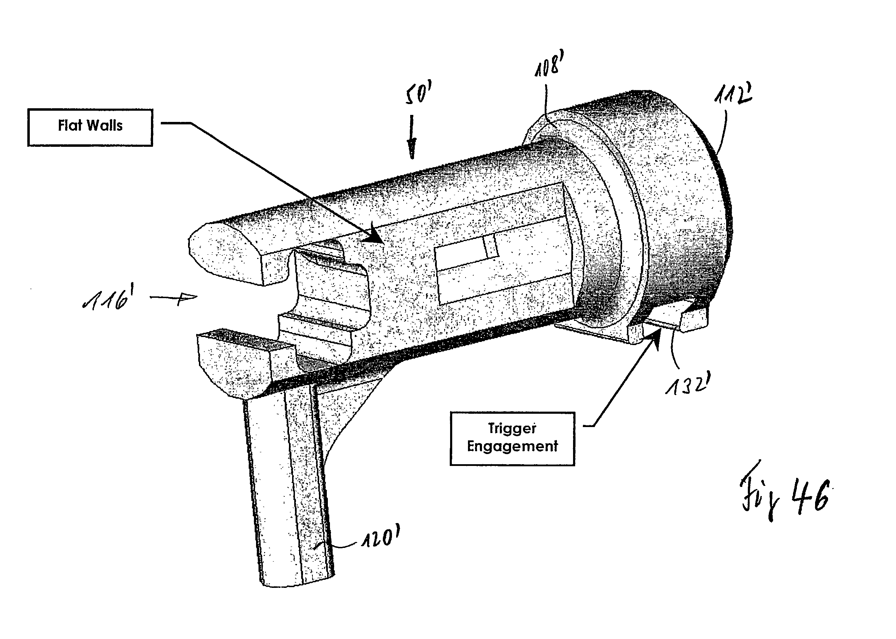

Es wird nun das Spannen der Stoßvorrichtung 10' beschrieben: Wie aus Figur 46 ersichtlich, umfasst das kolben- oder stösselartige Stoßorgan 50' einen quer zur Stechrichtung vorstehenden Spannnocken 120'. Dieser Spannnocken 120' durchgreift eine in radialer Richtung verlaufende lineare Durchbrechung 122' in der Bodenplatte 32' des Gehäusekörpers 6'. Der Spannnocken 120' ragt daher nach unten über die Unterseite der Bodenplatte 32' vor. Er greift dabei in eine Öffnung 124' in dem eingangs erwähnten scheibenförmigen Bauteil 42' mit dem radial vorstehenden Betätigungshebel 40' ein. Diese Öffnung bildet eine Nockenführungskurve 126', derart, dass der Spann-Nocken 120' bei Verdrehung des scheibenförmigen Bauteils 42' entlang dieser Nockenführungskurve 126' nach radial innen verlagert wird. Durch Verschwenken des radial vorstehenden Betätigungshebels 40' in Richtung des Pfeils 128' (Figur 45) wird über die Nockenführungskurve 26' und den Spannnocken 120' das Stoßorgan 50' entgegen der Kraft der Stoßfeder 52' nach radial innen bewegt, so lange bis ein Rastarm 130' des Auslösemittels 56' in eine Rastausnehmung 132' im durchmessergrößeren Abschnitt des Stoßorgans 50' einrastet und das Stoßorgan 50' zunächst im gespannten Zustand hält. Beim Loslassen des Betätigungshebels 40' wird dieser unter der Wirkung der eingangs erwähnten Rückstellfeder 46' (Figur 36) wieder in die Ausgangsposition, wie in Figur 45 dargestellt, zurückbewegt. Wie bereits erwähnt, bilden der Betätigungshebel 40' und das scheibenförmige Bauteil 42' ein Spannmittel 48' für die Stoßvorrichtung 10'.

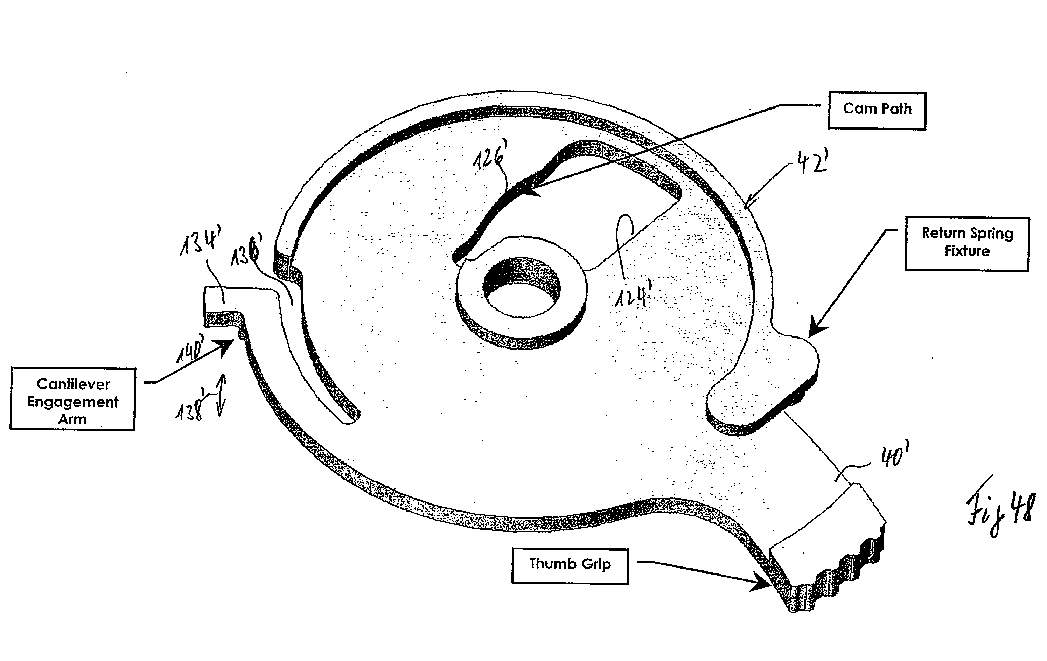

Man erkennt in Figur 48 einen in Umfangsrichtung erstreckten Steg 134' an dem scheibenförmigen Bauteil 42', der aufgrund einer schlitzförmigen Trennlinie 136', die sich ebenfalls im wesentlichen in Umfangsrichtung erstreckt, gegenüber der Ebene des Bauteils 42' geringfügig in axialer Richtung federnd nachgiebig auslenkbar ist (s. Pfeil 138). Am freien Ende des Stegs 134' ist ein in axialer Richtung vorstehender Ansatz 140' ausgebildet, der durch eine in Umfangsrichtung erstreckte schlitzförmige Ausnehmung 142' im Boden 32' des Gehäusekörpers 6' in dessen Inneres eingreift, wie aus Figur 45 ersichtlich ist. Dieser Ansatz 140' bewegt sich bei Verschwenken des Betätigungshebels 40' des Bauteils 42' entlang dieser schlitzförmigen Ausnehmung 142'. Dabei steht der Ansatz 140' in Drehmitnahme mit dem Träger 14', und zwar so lange bis der Steg 134' mit seinem Ansatz 140' an der Unterseite der Bodenplatte 32' des Gehäusekörpers 6' auf ein keilförmiges Rampenmittel 144' aufgleitet, welches aus den Figuren 12 und 13 ersichtlich ist. Bei diesem Aufgleiten wird der Steg 134' in der Darstellung der Figuren 44 und 45 nach unten ausgelenkt und der Ansatz 140' "taucht" in die Bodenplatte 32' ein und kommt dadurch außer Drehmitnahme mit dem Träger 14'. Es findet also nur in dieser ersten Phase der Bewegung des Betätigungshebels 40' ein Transport des Trägers 14' infolge Drehkopplung des Ansatzes 140' mit dem Träger 14' statt. Während dieser Phase wird der Spann-Nocken 120' noch nicht in radialer Richtung nach innen bewegt! Während dieser ersten Phase der Bewegung des Ansatzes 140' bis zu dem Rampenmittel 144' wird durch Drehung des Trägers 14' ein noch ungebrauchtes Stechelement 8' in die Arbeitsposition gebracht. Dabei wird der Haltekörper 62' mit seinem radial innen angeordneten Hintergriffsmittel 118' in den Kopplungsbereich 116' des Stoßorgans 50' gedreht. Die Öffnung 124' in dem scheibenförmigen Bauteil 42' ist nun derart ausgebildet und angeordnet, dass der Spannnocken 120' des Stoßorgans 50' in dem Moment mit der Nockenführungsbahn 126' zusammenwirkt, in welchem der nächstfolgende Haltekörper 62' in den Kopplungsbereich 116' des Stoßorgans 50' eingetreten ist und nicht mit weitertransportiert wird. In dieser Position wird nun durch Entlanggleiten des Spannnockens 120' entlang der Nockenführungskurve 126' das Stoßorgan 50' zusammen mit dem Haltekörper 62' und dem Stechelement 8' nach radial innen gezogen bis der Rastarm 130' mit einem Rasthaken in die Rastausnehmung 132' des Stoßorgans 50' einrastet. Wie bereits vorausgehend beschrieben, wird während dieses Spannvorgangs der Stoßvorrichtung 10' der Schwächungsbereich 70' zwischen Haltekörper 62' und Schutzkappenmittel 64' gebrochen und das Schutzkappenmittel wird in die in Figur 43' dargestellte Entsorgungsposition 103' bewegt, wodurch der Bewegungspfad für das Stechelement 8' freigegeben wird. Wird nun über das Auslösemittel 56' die Stoßvorrichtung 10' betätigt, so schnellt das Stoßorgan 50' zusammen mit dem Haltekörper 12' und dem Stechelement 8' in radialer Richtung nach außen, und das freie Ende 65' des Stechelements 8' schnellt extrem kurzzeitig über die Anlageposition 60' an der Außenseite des Gehäusekörpers 6' vor, um in die Hautoberfläche eines Benutzers extrem kurzzeitig eindringen zu können. Während dieses Stechvorgangs wird die Rückzugsfeder 54' gespannt, welche dann den Haltekörper 62' zusammen mit dem Stechelement 8' rasch wieder in das Innere des Gehäusekörpers 6' zurückbewegt. Auch der abspreizbare Steg 74' unterstützt dann die korrekte Positionierung der gebrauchten Stechelemente 8' am Träger 14' und verhindert beim Austausch einer gebrauchten Trägerkassette gegen eine neue, dass gebrauchte Stechelemente samt Haltekörper 62' aus den Führungsausnehmungen 16' im Träger 14' in radialer Richtung herausrutschen, was eine sichere Entsorgung gebrauchter Stechelemente gewährleistet.

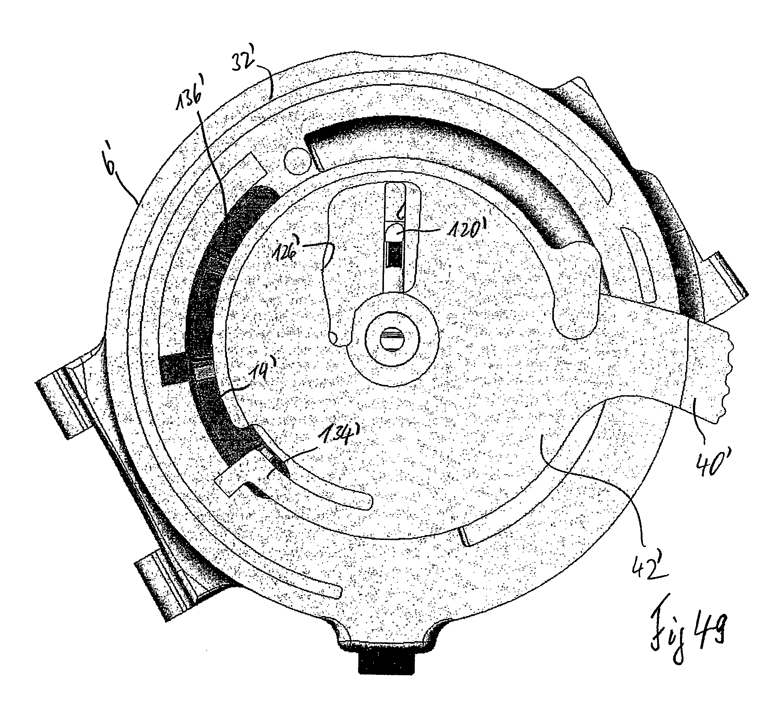

Figur 49 zeigt eine Ansicht auf die Unterseite der Bodenplatte 32' des Gehäusekörpers 6', also ohne den in Figur 36 dargestellten bodenseitigen Deckelteil 44'.

Mit der Erfindung wurde also insgesamt eine Stechvorrichtung mit miniaturisierten Stechelementen entwickelt, die es gestatten, die Stechvorrichtung nach Art und Größe einer Armbanduhr am Handgelenk eines Benutzers mitführbar auszubilden. Die Stechvorrichtung kann zudem in ein Blutanalysegerät integriert sein oder Komponenten eines Blutanalysegeräts der oben beschriebenen Art aufweisen. Die Stechvorrichtung umfasst eine Mehrzahl von insbesondere zwischen 5 und 12 Stechelementen, die als eine Kassette auf dem Träger 14' in den Gehäusekörper 6' eingesetzt werden. Hierfür klappt ein Benutzer das Deckelteil 4' nach oben und setzt eine Trägerkassette in das Innere unter Beachtung gewisser Orientierungsmarken ein. Das Deckelteil 4' wird dann verschlossen und über den Betätigungshebel 40' wird ein erstes Stechelement 8' in die Arbeitsposition gebracht. Dabei wird das Stechelement 8' bzw. sein Gehäusekörper 62' in eine Mitnahmekopplung mit dem Stoßorgan 50' gebracht und im Anschluss daran nach radial innen gezogen. Dabei wird das Schutzkappenmittel 64' am freien Ende 65' des Stechelements 8' abgetrennt und quer zur Stechrichtung 12' in eine Entsorgungsposition 103' auf dem Träger 14' verbracht. Beim Auslösen der so aktivierten Stoßvorrichtung 10' wird das Stoßorgan 50' zusammen mit dem Haltekörper 62' und dem Stechelement 8' in radialer Richtung schlagartig durch Entspannung der Stoßfeder 52' beschleunigt und mittels der Rückzugsfeder 54' wieder in die Ausgangsposition gebracht. Durch erneutes Betätigen des Betätigungshebels 40' wird wiederum über den Ansatz 140' der Träger 14' um eine Position weitergedreht, d. h. das gebrauchte Stechelement wird aus der Arbeitsposition gedreht und ein noch ungebrauchtes Stechelement wird in die Arbeitsposition gebracht usw. Wenn alle Stechelemente aufgebraucht sind, so lässt sich der Träger 14' aufgrund eines Drehanschlags nicht weiterbewegen und der Benutzer wird auf diese Weise darauf hingewiesen, dass eine Kassette mit neuen Stechelementen eingesetzt werden muss.

## Patentansprüche

1. Blutanalysegerät (2) mit einem Gehäusekörper (4), mit einer ein Stechelement (8, 114, 122, 232) aufweisenden Blutentnahmevorrichtung (6), mit einem Testmittel(10) für die Aufnahme einer Minimalmenge von Blut, mit einer eine Auswerteelektronik umfassenden Auswerteeinrichtung (12) und mit einer Anzeigeeinrichtung (14), die ein als ein einziges Gerät handhabbares Komplettsystem bilden, wobei der Gehäusekörper (4) eine der Arbeitsposition (34) des Stechelements (8, 114, 122, 232) zugeordnete Stechposition (22) zum Anlegen einer Hautoberfläche eines Benutzers aufweist und eine an anderer Stelle des Gehäusekörpers ausgebildete Aufgabeposition (30) zum Aufgeben einer aus der zuvor gestochenen Hautoberfläche austretenden Minimalmenge von Blut auf ein Testmittel (10) aufweist, wobei eine Mehrzahl von Testmitteln (10) und Stechelementen (8, 114, 122, 232) in das Gerät einsetzbar ist, die zur Durchführung mehrerer Messungen nacheinander in eine Arbeitsposition (30, 34) bringbar sind, wobei bei Positionierung eines Stechelements (8, 114, 122, 232) in seiner Arbeitsposition (34) das Stechelement in die an der Stechposition (22) angelegte Hautoberfläche eines Benutzers einstechbar ist und aus der Hautoberfläche austretendes Blut durch Anlegen der Hautoberfläche an die Aufgabeposition (30) auf ein Testmittel (10) aufgegeben werden kann, welches sich in einer Arbeitsposition (32) der Testmittel befindet, wobei die Testmittel (10) und die Stechelemente (8, 114, 122, 232) auf einem gegenüber dem Gehäusekörper (4) drehbaren Träger (50) angeordnet und mit diesem in das Gerät einsetzbar sind und dass durch Drehen des Trägers (50) die Testmittel (10) und die Stechelemente (8, 114, 122, 232) in voneinander verschiedene Arbeitspositionen (32, 34) bezüglich des Gehäusekörpers bringbar sind.

2. Blutanalysegerät nach Anspruch 1, **dadurch gekennzeichnet, dass** die Stechelemente und die Testmittel auf demselben manuell handhabbaren Träger angeordnet sind.

3. Blutanalysegerät nach Anspruch 1 oder 2, **dadurch gekennzeichnet, dass** der Träger (50) ein erstes Trägerteil (52) für die Testmittel (10) und ein zweites Trägerteil (56, 170) für die Stechelemente (8, 232) umfasst.

4. Blutanalysegerät nach Anspruch 3, **dadurch gekennzeichnet, dass** die beiden Trägerteile (52, 56) zu einer manuell handhabbaren Einheit montierbar sind.

5. Blutanalysegerät nach Anspruch 3 oder 4, **dadurch gekennzeichnet, dass** die Trägerteile (52, 56) drehfest miteinander koppelbar sind.

6. Blutanalysegerät nach einem oder mehreren der vorstehenden Ansprüche, **dadurch gekennzeichnet, dass** der Träger (50, 170) eine mittige Ausnehmung (80) aufweist, innerhalb der eine Antriebseinrichtung (44, 48) für die Blutentnahmevorrichtung (6) vorgesehen ist.

7. Blutanalysegerät nach einem oder mehreren der vorstehenden Ansprüche, **dadurch gekennzeichnet, dass** der Träger (50, 170) ringartig ausgebildet ist und um das Ringzentrum drehbar ist.

8. Blutanalysegerät nach einem oder mehreren der vorstehenden Ansprüche, **dadurch gekennzeichnet, dass** eine Antriebseinrichtung für den Träger eine Innenverzahnung (82) umfasst.

9. Blutanalysegerät nach einem oder mehreren der vorstehenden Ansprüche, **dadurch gekennzeichnet, dass** die Stechelemente (8, 114, 232) so auf dem Träger (50, 170) angeordnet sind, dass sie in der Arbeitsposition eine Stechbewegung in radialer Richtung in Bezug auf die Drehbarkeit des Trägers ausführen.

10. Blutanalysegerät nach einem oder mehreren der vorstehenden Ansprüche 1 - 8, **dadurch gekennzeichnet, dass** die Stechelemente (122) so auf dem Träger (126) angeordnet sind, dass sie in der Arbeitsposition eine Stechbewegung in axialer Richtung in Bezug auf die Drehbarkeit des Trägers ausführen.

11. Blutanalysegerät nach einem oder mehreren der vorstehenden Ansprüche, **dadurch gekennzeichnet, dass** die Stechelemente (8, 114, 122, 232) auf dem Träger (50 126, 170) vor Ausführung eines Stechvorgangs von einer Sterilitätsbarriere umgeben sind.

12. Blutanalysegerät nach einem oder mehreren der vorstehenden Ansprüche, **dadurch gekennzeichnet, dass** ein jeweiliges Stechelement (114) vor Ausführung eines Stechvorgangs in einem einen Zylinderraum bildenden Hülsenmittel (100) angeordnet und von einem darin bewegbaren Kolbenmittel (106) gehalten ist.

13. Blutanalysegerät nach Anspruch 12, **dadurch gekennzeichnet, dass** das Stechelement (114) ein Einspritzteil des als Kunststoffspritzteil ausgebildeten Kolbenmittels (106) bildet.

14. Blutanalysegerät nach Anspruch 12 oder 13, **dadurch gekennzeichnet, dass** eine Sterilitätsbarriere von dem einseitig geschlossenen Hülsenmittel (100) und dem Kolbenmittel (106) gebildet ist.

15. Blutanalysegerät nach Anspruch 12, 13 oder 14, **dadurch gekennzeichnet, dass** das Hülsenmittel (100) an seinem von dem Kolbenmittel (106) abgewandten Ende von einer Folie (112) abgedeckt ist.

16. Blutanalysegerät nach einem der Ansprüche 12 bis 15, **dadurch gekennzeichnet, dass** das Kolbenmittel (106) ein Dichtungsmittel (118, 120) gegenüber einer Wandung (116) des Zylinderraums aufweist.

17. Blutanalysegerät nach einem der Ansprüche 12 bis 16, **dadurch gekennzeichnet, dass** mehrere Hülsenmittel (100) bandförmig aneinandergefügt und die Bandenden zur Bildung einer Kreisform miteinander verbunden sind.

18. Blutanalysegerät nach einem oder mehreren der vorstehenden Ansprüche, **gekennzeichnet durch** mehrere Vertiefungen (124) in dem Träger (126), in denen ein jeweiliges Stechelement (122) angeordnet ist.

19. Blutanalysegerät nach Anspruch 18, **dadurch gekennzeichnet, dass** eine die Vertiefung (124) begrenzende Wandung (133) deformierbar ausgebildet ist, so dass sie zur Ausführung des Stechvorgangs durch eine Antriebseinrichtung der Blutentnahmevorrichtung auslenkbar ist.

20. Blutanalysegerät nach Anspruch 18 oder 19, **dadurch gekennzeichnet, dass** eine die Vertiefung (124) begrenzende Wandung (133) Schwächungzonen zur Erleichterung der Verformbarkeit aufweist.

21. Blutanalysegerät nach einem Anspruch 18, 19 oder 20, **dadurch gekennzeichnet, dass** die Vertiefung (124) muldenförmig oder halbschalenförmig ausgebildet ist.

22. Blutanalysegerät nach einem der Ansprüche 18 bis 21 ,
**dadurch gekennzeichnet, dass** die Sterilitätsbarriere von einem die Vertiefung (124) überfangenden folienartigen Abdeckmittel (134) gebildet ist.

23. Blutanalysegerät nach einem oder mehreren der vorstehenden Ansprüche, **dadurch gekennzeichnet, dass** die Stechelemente (140) vor Ausführung eines Stechvorgangs an ihrem freien Ende ein Schutzkappenmittel (147) tragen.

24. Blutanalysegerät nach Anspruch 23, **dadurch gekennzeichnet, dass** das Schutzkappenmittel (147) unmittelbar vor Ausführung des Stechvorgangs von dem Stechelement (140) ablösbar ist.

25. Blutanalysegerät nach Anspruch 24, **dadurch gekennzeichnet, dass** das jeweilige Schutzkappenmittel (147) nach dem Ablösen von einem jeweiligen Stechelement (140) aus dem Bewegungspfad des Stechelements entfernbar und in einen Aufnahmeraum (152) verbringbar ist.

26. Blutanalysegerät nach einem oder mehreren der vorstehenden Ansprüche, **dadurch gekennzeichnet, dass** die Testmittel (10) so auf dem Träger (50) angeordnet sind, dass sie in Bezug auf die Drehbarkeit des Trägers axial orientiert sind.

27. Blutanalysegerät nach einem oder mehreren der vorstehenden Ansprüche , **dadurch gekennzeichnet, dass** der Träger (50) einen insbesondere ringscheibenförmigen Trägerteil (52) für die Testmittel (10) aufweist, dessen Ebene senkrecht zur Drehachse (70) des Trägers (50) orientiert ist.

28. Blutzuckeranalysegerät nach einem oder mehreren der vorstehenden Ansprüche, **dadurch gekennzeichnet, dass** die Testmittel (10) in Ausnehmungen (68) des insbesondere ringscheibenförmigen Trägerteils (52) vorgesehen sind.

29. Blutzuckeranalysegerät nach Anspruch einem oder mehreren der vorstehenden Ansprüche, **dadurch gekennzeichnet, dass** die Aufgabeposition (30) durch ein bewegbares Abdeckteil (28) abdeckbar ist, wenn sie nicht benötigt wird.

30. Blutzuckeranalysegerät nach Anspruch 29, **dadurch gekennzeichnet, dass** durch Bewegen des Abdeckteils (28) in Richtung einer Freigabe der Aufgabeposition (30) eine Antriebseinrichtung für das Stechelement aktivierbar ist.

31. Blutzuckeranalysegerät nach einem oder mehreren der vorstehenden Ansprüche, **dadurch gekennzeichnet, dass** die Antriebseinrichtung für das Stechelement durch Spannen eines Federmittels (156) aktivierbar ist.

32. Blutanalysegerät nach einem oder mehreren der vorstehenden Ansprüche, **dadurch gekennzeichnet, dass** ein manuell bewegbares Steuerelement (238) vorgesehen ist, das mit der Antriebsvorrichtung (250) für das Stechelement (8) und mit dem drehbaren Träger (60) so gekoppelt ist, dass bei Bewegen des Steuerelements (238) eine Aktivierung der Antriebsvorrichtung (250) für das Stechelement und eine Drehbewegung des Trägers (60) erfolgt.

33. Blutanalysegerät nach Anspruch 32 , **dadurch gekennzeichnet, dass** das Steuerelement (238) während einer ersten Phase der Bewegung in einer ersten Stellrichtung mit dem Träger (60) in Antriebsverbindung bringbar ist und während einer zweiten Phase der Bewegung entgegen der Stellrichtung außer Antriebsverbindung bringbar ist.

34. Blutanalysegerät nach Anspruch 22 oder 33, **dadurch gekennzeichnet, dass** zur Kopplung des Steuerelements (238) mit dem Träger ein Zahntrieb vorgesehen ist.

35. Blutanalysegerät nach Anspruch 32, 33 oder 34, **dadurch gekennzeichnet**, das die Antriebsvorrichtung (250) für das Stechelement eine Biegefeder (252) umfasst und dass das Steuerelement (238) auf eine Aufnahme (258) für die Biegefeder einwirkt und diese Aufnahme in der Biegeebene der Biegefeder verschwenkt.

36. Blutanalysegerät nach Anspruch 35, **dadurch gekennzeichnet, dass** die Biegefeder über einen Totpunkt hinweg in eine stabile Spannlage spannbar ist.

37. Blutanalysegerät nach einem der Ansprüche 32 bis 36, **dadurch gekennzeichnet, dass** das manuell bewegbare Steuerelement (238) von dem Abdeckteil (28) gebildet ist.

38. Blutanalysegerät nach einem oder mehreren der vorstehenden Ansprüche, **dadurch gekennzeichnet, dass** eine Auslöseeinrichtung (254) für die Antriebsvorrichtung (250) für das Stechelement durch Anlegen einer Hautoberfläche an die Stechposition (22) betätigbar ist.

39. Blutanalysegerät nach Anspruch 38, **dadurch gekennzeichnet, dass** die Auslöseeinrichtung (254) von einem in der Stechposition (22) vorgesehenen Taster (264) gebildet ist.

40. Blutanalysegerät nach Anspruch 38 oder 39, **dadurch gekennzeichnet, dass** die Auslöseeinrichtung (254) in der Stechposition (22) vorgesehen ist und eine Aussparung zum Durchtritt des Stechelements zur Ausführung des Stechvorgangs aufweist.

41. Blutanalysegerät nach einem oder mehreren der vorstehenden Ansprüche, **dadurch gekennzeichnet, dass** ein Retraktionsmechanismus (78) vorgesehen ist, mittels dessen ein jeweiliges Stechelement (8, 232) unmittelbar im Anschluss an den Stechvorgang zurückgezogen werden kann.

42. Blutanalysegerät nach Anspruch 41, **dadurch gekennzeichnet, dass** ein Federmittel (74, 78, 108) zum Zurückziehen eines jeweiligen Stechelements (8, 114, 232) aus der Hautoberfläche des Benutzers vorgesehen ist.

43. Blutanalysegerät nach einem oder mehreren der vorstehenden Ansprüche , **dadurch gekennzeichnet, dass** sich die jeweiligen Stechelemente (8) durch ein jeweiliges Federmittel (74, 78) hindurcherstrecken.

44. Blutanalysegerät nach einem oder mehreren der vorstehenden Ansprüche, **gekennzeichnet durch** eine Sicherheitseinrichtung, die ein Auslösen des Stechvorgangs erst bei bestimmungsgemäßer Handhabung des Geräts erlaubt.

45. Blutanalysegerät nach einem oder mehreren der vorstehenden Ansprüche, **dadurch gekennzeichnet, dass** die Anzahl der als Einheit handhabbaren Testmittel 5 bis 15 beträgt.

46. Blutanalysegerät nach einem der vorstehenden Ansprüche, **dadurch gekennzeichnet, dass** das Gerät eine im wesentlichen kreisscheibenförmige Außenkontur aufweist.

47. Blutanalysegerät nach einem oder mehreren der vorstehenden Ansprüche, **dadurch gekennzeichnet, dass** es eine Zeitanzeigeeinrichtung umfasst.

48. Blutanalysegerät nach einem oder mehreren der vorstehenden Ansprüche, **dadurch gekennzeichnet, dass** der Gehäusekörper (4) mittels eines daran befestigbaren Bandes am Handgelenk eines Benutzers getragen werden kann.

## Claims

1. Blood analyzer (2) having a device body (4) with a blood sampling device (6) which has a pricking element (8, 114, 122, 232), with a testing means (10) for accommodating a minimal quantity of blood, having an analyzer device (12) which comprises an electronic analyzer and having a display device (14), together forming a complete system that can be handled as a single device, whereby the device body (4) has a pricking position (22) which is assigned to the working position (34) of the pricking element (8, 114, 122, 232) for coming in contact with a skin surface of a user and a charging position (30) designed at another location on the body of the device for charging a minimal quantity of blood coming from the previously pricked skin surface onto a testing means (10), whereby a plurality of testing means (10) and pricking elements (8, 114, 122, 232) can be inserted into the device and can be brought one after the other into a working position (30, 34) for performing multiple measurements, whereby when a pricking element (8, 114, 122, 232) is positioned in its working position (34), the pricking element can be inserted into the skin surface of a user which is brought into the pricking position (22) and blood coming from the skin surface can be charged to a testing means (10) by being brought in contact with the skin surface in the charging position (30), said testing means being in a working position (32) of the testing means, **characterized in that** the testing means (10) and the pricking elements (8, 114, 122, 232) are arranged on a carrier (50) which is rotatable with respect to the body (4) of the device and can be inserted together with it into the device, and by rotating the carrier (50) the testing means (10) and the pricking elements (8, 114, 122, 232) can be brought into different working positions (32, 34) with respect to the body of the device.

2. Blood analyzer according to Claim 1, **characterized in that** the pricking elements and the testing means are arranged on the same carrier, which can be handled manually.

3. Blood analyzer according to Claim 1 or 2, **characterized in that** the carrier (50) comprises a first carrier part (52) for the testing means (10) and a second carrier part (56, 170) for the pricking elements (8, 232).

4. Blood analyzer according to Claim 3, **characterized in that** the two carrier parts (52, 56) can be assembled to form a manually operable unit.

5. Blood analyzer according to Claim 3 or 4, **characterized in that** the carrier parts (52, 56) can be linked together in a rotationally fixed manner.

6. Blood analyzer according to one or more of the preceding claims, **characterized in that** the carrier (50, 170) has a central recess (80) within which a drive device (44, 48) for the blood sampling device (6) is provided.

7. Blood analyzer according to one or more of the preceding claims, **characterized in that** the carrier (50,170) is designed in the form of a ring and is rotatable about the center of the ring.

8. Blood analyzer according to one or more of the preceding claims, **characterized in that** a drive device for the carrier includes internal gearing (82).

9. Blood analyzer according to one or more of the preceding claims, **characterized in that** the pricking elements (8, 114, 232) are arranged on the carrier (50, 170) in such a way that when they are in the working position, they execute a pricking movement in the radial direction with respect to the rotatability of the carrier.

10. Blood analyzer according to one or more of the preceding Claims 1 through 8, **characterized in that** the pricking elements (122) are arranged on the carrier (126) in such a way that when they are in the working position, they execute a pricking movement in the axial direction with respect to the rotatability of the carrier.

11. Blood analyzer according to one or more of the preceding claims, **characterized in that** the pricking elements (8, 114, 122, 232) are surrounded by a sterility barrier on the carrier (50, 126, 170) before execution of a pricking operation.

12. Blood analyzer according to one or more of the preceding claims, **characterized in that** before execution of a pricking operation, a particular pricking element (114) is arranged in a sleeve means (100), forming a cylindrical space, and is held by a plunger means (106) which is movable in the sleeve means.

13. Blood analyzer according to Claim 12, **characterized in that** the pricking element (114) forms an injection part of the plunger means (106) designed as a plastic syringe part.

14. Blood analyzer according to Claim 12 or 13, **characterized in that** a sterility barrier is formed by the sleeve means (100) that is closed on all sides and by the plunger means (106).

15. Blood analyzer according to Claim 12, 13 or 14, **characterized in that** the sleeve means (100) is covered by a film (112) on its end facing away from the plunger means (106).

16. Blood analyzer according to one of Claims 12 through 15, **characterized in that** the plunger means (106) has a sealing means (118, 120) with respect to a wall (116) of the cylinder space.

17. Blood analyzer according to one of Claims 12 through 16, **characterized in that** multiple sleeve means (100) are joined together in the form of a strip and the ends of the strips are joined together to form a circular shape.

18. Blood analyzer according to one or more of the preceding claims, **characterized by** multiple recesses (124) in the carrier (126) in each of which is arranged a pricking element (122).

19. Blood analyzer according to Claim 18, **characterised in that** a wall (133) which borders the recess (124) is designed to be deformable so that it can be deflected by a driving device of the blood sampling device to execute the pricking procedure.

20. Blood analyzer according to Claim 18 or 19, **characterized in that** a wall (133) which borders the recess (124) has weakened zones to facilitate the deformability.

21. Blood analyzer according to one of Claims 18, 19 or 20, **characterized in that** the recess (124) is designed like a trough or like a half shell.

22. Blood analyzer according to one of Claims 18 through 21, **characterized in that** the sterility barrier is formed by a film-like covering means (134) which covers the recess (124).

23. Blood analyzer according to one or more of the preceding claims, **characterized in that** the pricking elements (140) carry a safety cap means (147) on their free end before executing a pricking operation.

24. Blood analyzer according to Claim 23, **characterized in that** the safety cap means (147) is releasable from the pricking element (140) immediately before execution of the pricking operation.

25. Blood analyzer according to Claim 24, **characterized in that** the respective safety cap means (147) can be removed from the path of movement of the pricking element and brought into a receptacle space (152) after being released from the respective pricking element (140).

26. Blood analyzer according to one or more of the preceding claims, **characterized in that** the test means (10) are arranged on the carrier (50) in such a way that they are axially oriented with respect to the rotatability of the carrier.

27. Blood analyzer according to one or more of the preceding claims, **characterized in that** the carrier (50) has a carrier part (52) for the test means (10), the carrier part in particular being in the form of a ring disk, the plane of the carrier part being oriented perpendicular to the axis of rotation (70) of the carrier (50).

28. Blood sugar analysis device according to one or more of the preceding claims, **characterized in that** the test means (10) are provided in recesses (68) of the carrier part (52), in particular in the form of a ring disk.

29. Blood sugar analysis device according to one or more of the preceding claims, **characterized in that** the charging position (30) can be covered by a movable cover part (28) when it is not needed.

30. Blood sugar analysis device according to Claim 29, **characterized in that** a drive device for the pricking element can be activated by moving the covering part (28) in the direction of releasing the charging position (30).

31. Blood sugar analysis device according to one or more of the preceding claims, **characterized in that** the drive device for the pricking element can be activated by clamping a spring means (156).

32. Blood analyzer according to one or more of the preceding claims, **characterized in that** a manually movable control element (238) is provided and is connected to the drive device (250) for the pricking element (8) and to the rotatable carrier (60), so that when there is a movement of the control element (238), the drive device (250) for the pricking element is activated and there is a rotational movement of the carrier (60).

33. Blood analyzer according to Claim 32, **characterized in that** during a first phase of the movement in a first actuating direction, the control element (238) can be brought into a drive connection with the carrier (60), and during a second phase of the movement, it can be brought out of the drive connection by moving it in the direction opposite the actuator direction.

34. Blood analyzer according to Claim 22 or 33, **characterized in that** a gear drive is provided for coupling the control element (238) to the carrier.

35. Blood analyzer according to Claim 32, 33 or 34, **characterized in that** the drive mechanism (250) for the pricking element comprises a bending spring (252), and the control element (238) acts on a receptacle (258) for the bending spring and pivots this receptacle into the plane of bending of the bending spring.

36. Blood analyzer according to Claim 35, **characterized in that** the bending spring can be clamped into a stable clamped position across a dead point.

37. Blood analyzer according to one of Claims 32 through 36, **characterized in that** the manually movable control element (238) is formed by the covering part (28).

38. Blood analyzer according to one or more of the preceding claims, **characterized in that** a triggering device (254) for the drive device (250) for the pricking element can be operated by contact of the skin surface with the pricking position (22).

39. Blood analyzer according to Claim 38, **characterized in that** the triggering device (254) is formed by a key (264) provided in the pricking position (22).

40. Blood analyzer according to Claim 38 or 39, **characterized in that** the deployment device (254) is provided in the pricking position (22) and has a recess for the passage of the pricking element for execution of the pricking operation.

41. Blood analyzer according to one or more of the preceding claims, **characterized in that** a retraction mechanism (78) is provided by means of which a particular pricking element (8, 232) can be retracted directly following the pricking operation.

42. Blood analyzer according to Claim 41, **characterized in that** a spring means (74, 78, 108) is provided for retracting a particular pricking element (8, 114, 232) from the skin surface of the user.

43. Blood analyzer according to one or more of the preceding claims, **characterized in that** the particular pricking elements (8) pass through a particular spring means (74, 78).

44. Blood analyzer according to one or more of the preceding claims, **characterized by** a safety device which allows deployment of the pricking operation only when the device is being handled properly.

45. Blood analyzer according to one or more of the preceding claims, **characterized in that** the number of test means that can be handled as one unit amounts to 5 to 15.

46. Blood analyzer according to one or more of the preceding claims, **characterized in that** the device has an outside contour that is essentially in the form of a circular disk.

47. Blood analyzer according to one or more of the preceding claims, **characterized in that** it comprises a time display device.

48. Blood analyzer according to one or more of the preceding claims, **characterized in that** the housing body (4) can be worn on the wrist of a user by means of a strip that can be attached to it.

## Revendications

1. Analyseur de sang (2) avec un corps de boîtier (4), avec un dispositif de prélèvement de sang (6) présentant un élément de piqûre (8, 114, 122, 232), avec un moyen de test (10) destiné à recevoir une quantité minimale de sang, avec un dispositif d'évaluation (12) comportant une électronique d'évaluation et avec un dispositif de visualisation (14), lesquels constituent un système complet maniable comme un seul appareil, le corps de boîtier (4) présentant une position de piqûre (22), associée à la position de travail (34) de l'élément de piqûre (8, 114, 122, 232), pour appliquer une surface de peau d'un utilisateur et une position d'alimentation (30), réalisée à un autre endroit du corps de boîtier, pour l'alimentation d'une quantité minimale de sang sortant de la surface de peau piquée auparavant vers un moyen de test (10), dans l'appareil pouvant être utilisés une pluralité de moyens de test (10) et d'éléments de piqûre (8, 114, 122, 232) qui, pour effectuer plusieurs mesures, peuvent être amenés successivement en une position de travail (30, 34), lors du positionnement d'un élément de piqûre (8, 114, 122, 232) dans sa position de travail (34), l'élément de piqûre pouvant être piqué dans la surface de peau d'un utilisateur appliquée sur la position de piqûre (22) et le sang sortant de la surface de peau par l'application de la surface de peau sur la position d'alimentation (30) pouvant être alimenté vers un moyen de test (10) qui se situe en une position de travail (32) du moyen de test, les moyens de test (10) et les éléments de piqûre (8, 114, 122, 232) étant disposés sur un support (50) rotatif par rapport au corps de boîtier (4) et pouvant être introduits avec ce dernier dans l'appareil et les moyens de test (10) et les élément de piqûre (8, 114, 122, 232) pouvant, en faisant tourner le support (50), être amenés dans des positions de travail (32, 34) différentes l'une de l'autre par rapport au corps de boîtier.

2. Analyseur de sang selon la revendication 1, **caractérisé par le fait que** les éléments de piqûre et les moyens de test sont disposés sur le même support maniable manuellement.

3. Analyseur de sang selon la revendication 1 ou 2, **caractérisé par le fait que** le support (50) comporte un premier élément de support (52) pour les moyens de test (10) et un deuxième élément de support (56, 170) pour les éléments de piqûre (8, 232).

4. Analyseur de sang selon la revendication 3, **caractérisé par le fait que** les deux éléments de support (52, 56) peuvent être montés de manière à former un ensemble maniable manuellement.

5. Analyseur de sang selon la revendication 3 ou 4, **caractérisé par le fait que** les éléments de support (52, 56) peuvent être couplés l'un à l'autre de manière immobile en rotation.

6. Analyseur de sang selon l'une ou plusieurs des revendications précédentes, **caractérisé par le fait que** le support (50, 170) présente un évidement central (80) à l'intérieur duquel est prévu un dispositif d'entraînement (44, 48) du dispositif de prélèvement de sang (6).

7. Analyseur de sang selon l'une ou plusieurs des revendications précédentes, **caractérisé par le fait que** le support (50, 170) est réalisé en forme de bague et est rotatif autour du centre de la bague.

8. Analyseur de sang selon l'une ou plusieurs des revendications précédentes, **caractérisé par le fait qu'**un dispositif d'entraînement du support comporte une denture intérieure (82).

9. Analyseur de sang selon l'une ou plusieurs des revendications précédentes, **caractérisé par le fait que** les éléments de piqûre (8, 114, 232) sont disposés sur le support (50, 170) de sorte qu'ils effectuent, en position de travail, un mouvement de piqûre en direction radiale par rapport à l'aptitude à la rotation du support.

10. Analyseur de sang selon l'une ou plusieurs des revendications précédentes 1 à 8, **caractérisé par le fait que** les éléments de piqûre (122) sont disposés sur le support (126) de sorte qu'ils effectuent, en position de travail, un mouvement de piqûre en direction axiale par rapport à l'aptitude à la rotation du support.

11. Analyseur de sang selon l'une ou plusieurs des revendications précédentes, **caractérisé par le fait que** les élément de piqûre (8, 114, 122, 232) sur le support (50, 126, 170) sont, avant d'effectuer une opération de piqûre, entourés d'une barrière de stérilité.

12. Analyseur de sang selon l'une ou plusieurs des revendications précédentes, **caractérisé par** fait qu'un élément de piqûre (114) respectif est, avant d'effectuer une opération de piqûre, disposé dans un moyen de douille (100) formant un espace cylindrique et maintenu par un moyen de piston (106) mobile dans ce dernier.

13. Analyseur de sang selon la revendication 12, **caractérisé par le fait que** l'élément de piqûre (114) constitue un élément d'injection du moyen de piston (106) réalisé sous forme d'élément d'injection en matière plastique.

14. Analyseur de sang selon la revendication 12 ou 13, **caractérisé par le fait qu'**une barrière de stérilité est constituée par le moyen de douille fermé d'un côté (100) et le moyen de piston (106).

15. Analyseur de sang selon la revendication 12, 13 ou 14, **caractérisé par le fait que** le moyen de douille (100) est, à son extrémité éloignée du moyen de piston (106), recouvert par un film (112).

16. Analyseur de sang selon l'une des revendications 12 à 15, **caractérisé par le fait que** le moyen de piston (106) présente un moyen d'étanchéité (118, 120) par rapport à une paroi (116) de l'espace cylindrique.

17. Analyseur de sang selon l'une des revendications 12 à 16, **caractérisé par le fait que** plusieurs moyens de douille (100) sont assemblés l'un à l'autre en forme de bande et que les extrémités de bande sont assemblées l'une à l'autre pour créer une forme circulaire.

18. Analyseur de sang selon l'une ou plusieurs des revendications précédentes, **caractérisé par** plusieurs creux (124) dans le support (126) dans lequel est disposé un élément de piqûre (122) respectif.

19. Analyseur de sang selon la revendication 18, **caractérisé par le fait qu'**une paroi (133) délimitant le creux (124) est réalisée déformable, de sorte qu'elle puisse être pivotée pour la réalisation de l'opération de piqûre par un dispositif d'entraînement du dispositif de prélèvement de sang.

20. Analyseur de sang selon la revendication 18 ou 19, **caractérisé par le fait qu'**une paroi (133) délimitant le creux (124) présente des zones d'affaiblissement destinées à faciliter la déformabilité.

21. Analyseur de sang selon l'une des revendications 18, 19 ou 20, **caractérisé par le fait que** le creux (124) est réalisé en forme de cuvette ou de demi-coquille.

22. Analyseur de sang selon l'une des revendications 18 à 21, **caractérisé par le fait que** la barrière de stérilité est constituée par un moyen de recouvrement (134) en forme de film entourant par le haut le creux (124).

23. Analyseur de sang selon l'une ou plusieurs des revendications précédentes, **caractérisé par le fait que** les éléments de piqûre (140) portent, avant la réalisation d'une opération de piqûre, un moyen de capuchon protecteur (147) à leur extrémité libre.

24. Analyseur de sang selon la revendication 23, **caractérisé par le fait que** le moyen de capuchon protecteur (147) peut être enlevé de l'élément de piqûre immédiatement avant de réaliser l'opération de piqûre (140).

25. Analyseur de sang selon la revendication 24, **caractérisé par le fait que** le moyen de capuchon protecteur (147) respectif peut, après le détachement d'un élément de piqûre (140) respectif, être éloigné d'un trajet de mouvement de l'élément de piqûre et être déplacé vers un espace de réception (152).

26. Analyseur de sang selon l'une ou plusieurs des revendications précédentes, **caractérisé par le fait que** les moyens de test (10) sont disposés sur le support (50) de sorte qu'ils soient orientés axialement par rapport à l'aptitude à la rotation du support.

27. Analyseur de sang selon l'une ou plusieurs des revendications précédentes, **caractérisé par le fait que** le support (50) présente un élément de support (52) en particulier en forme de disque annulaire pour les moyens de test (10) dont le plan est orienté perpendiculairement à l'axe de la rotation (70) du support (50).

28. Analyseur de glucose sanguin selon l'une ou plusieurs des revendications précédentes, **caractérisé par le fait que** les moyens de test (10) sont prévus dans des évidements (68) de l'élément de support (52) en particulier en forme de disque annulaire.

29. Analyseur de glucose sanguin selon l'une ou plusieurs des revendications précédentes, **caractérisé par le fait que** la position d'alimentation (30) peut être recouverte par un élément de recouvrement mobile (28) lorsqu'elle n'est pas requise.

30. Analyseur de glucose sanguin selon la revendication 29, **caractérisé par le fait que** par le déplacement de l'élément de recouvrement (28) en direction d'un dégagement de la position de travail (30) peut être activé un dispositif d'entraînement de l'élément de piqûre.

31. Analyseur de glucose sanguin selon l'une ou plusieurs des revendications précédentes, **caractérisé par le fait que** le dispositif d'entraînement de l'élément de piqûre peut être activé par tension d'un moyen de ressort (156).

32. Analyseur de sang selon l'une ou plusieurs des revendications précédentes, **caractérisé par le fait qu'**il est prévu un élément de commande (238) déplaçable manuellement qui est couplé au dispositif d'entraînement (250) de l'élément de piqûre (8) et au support rotatif (60) de sorte que lors du déplacement de l'élément de commande (238) aient lieu une activation du dispositif d'entraînement (250) de l'élément de piqûre et un mouvement de rotation du support (60).

33. Analyseur de sang selon la revendication 32, **caractérisé par le fait que** l'élément de commande (238) peut être amené, pendant une première phase du déplacement dans une première direction de réglage, en connexion d'entraînement avec le support (60) et peut être amené, pendant une deuxième phase de réglage dans une direction contraire à la direction de réglage, hors connexion d'entraînement.

34. Analyseur de sang selon la revendication 22 ou 33, **caractérisé par le fait qu'**il est prévu un pignon denté pour le couplage de l'élément de commande (238) avec le support.

35. Analyseur de sang selon la revendication 32, 33 ou 34, **caractérisé par le fait que** le dispositif d'entraînement (250) de l'élément de piqûre comporte un ressort de flexion (252) et que l'élément de commande (238) agit sur un dispositif de réception (258) du ressort de flexion et que ce dispositif de réception pivote dans le plan de flexion du ressort de flexion.

36. Analyseur du sang selon la revendication 35, **caractérisé par le fait que** le ressort de flexion peut être tendu, au-delà d'un point mort, en une position de tension stable.

37. Analyseur de sang selon l'une des revendications 32 à 36, **caractérisé par le fait que** l'élément de commande déplaçable manuellement (238) est formé par l'élément de recouvrement (28).

38. Analyseur de sang selon l'une ou plusieurs des revendications précédentes, **caractérisé par le fait qu'**un dispositif de déclenchement (254) du dispositif d'entraînement (250) de l'élément de piqûre peut être actionné par l'application d'une surface de peau sur la position de piqûre (22).

39. Analyseur de sang selon la revendication 38, **caractérisé par le fait que** le dispositif de déclenchement (254) est formé par un palpeur (264) prévu à la position de piqûre (22).

40. Analyseur de sang selon la revendication 38 ou 39, **caractérisé par le fait que** le dispositif de déclenchement (254) est prévu à la position de piqûre (22) et présente un évidement destiné au passage de l'élément de piqûre destiné à réaliser l'opération de piqûre.

41. Analyseur de sang selon l'une ou plusieurs des revendications précédentes, **caractérisé par le fait qu'**il est prévu un mécanisme de retrait (78) au moyen duquel peut être retiré d'un élément de piqûre (8, 232) respectif immédiatement après l'opération de piqûre.

42. Analyseur de sang selon la revendication 41, **caractérisé par le fait qu'**il est prévu un moyen de ressort (74, 78, 108) destiné à retirer un élément de piqûre (8, 114, 232) respectif de la surface de peau de l'utilisateur.

43. Analyseur de sang selon l'une ou plusieurs des revendications précédentes, **caractérisé par le fait que** les éléments de piqûre (8) respectifs s'étendent à travers un moyen de ressort (74, 78) respectif.

44. Analyseur de sang selon l'une ou plusieurs des revendications précédentes, **caractérisé par** un dispositif de sécurité qui ne permet un déclenchement de l'opération de piqûre qu'en cas de manipulation correcte de l'appareil.

45. Analyseur de sang selon l'une ou plusieurs des revendications précédentes, **caractérisé par le fait que** le nombre de moyens de test maniables comme un ensemble est de 5 à 15.

46. Analyseur de sang selon l'une des revendications précédentes, **caractérisé par le fait que** l'appareil présente un contour extérieur sensiblement en forme de disque circulaire.

47. Analyseur de sang selon l'une ou plusieurs des revendications précédentes, **caractérisé par le fait qu'**il comporte un dispositif d'affichage de temps.

48. Analyseur de sang selon l'une ou plusieurs des revendications précédentes, **caractérisé par le fait que** le corps de boîtier (4) peut être porté au poignet d'un utilisateur au moyen d'une bande pouvant y être fixée.
